(19) Europäisches Patentamt / European Patent Office / Office européen des brevets

(11) EP 2 791 275 B1

(12) # EUROPÄISCHE PATENTSCHRIFT

(45) Veröffentlichungstag und Bekanntmachung des Hinweises auf die Patenterteilung:
**31.05.2017 Patentblatt 2017/22**

(21) Anmeldenummer: **12805427.7**

(22) Anmeldetag: **05.12.2012**

(51) Int Cl.:
***C09K 11/06*** (2006.01) ***H05B 33/18*** (2006.01)
***A61N 5/06*** (2006.01) ***H01J 61/44*** (2006.01)
***G02F 1/00*** (2006.01) ***H01L 31/055*** (2014.01)
***C09B 69/10*** (2006.01) ***G02F 1/35*** (2006.01)
***H01L 51/44*** (2006.01) ***H01L 51/50*** (2006.01)

(86) Internationale Anmeldenummer:
**PCT/EP2012/005017**

(87) Internationale Veröffentlichungsnummer:
**WO 2013/087162 (20.06.2013 Gazette 2013/25)**

(54) **ORGANISCHE SENSIBILISATOREN FÜR DIE AUFWÄRTSKONVERSION (UP-CONVERSION)**
ORGANIC SENSITIZERS FOR UP-CONVERSION
SENSIBILISATEURS ORGANIQUES POUR CONVERSION ASCENDANTE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.12.2011 DE 102011121022**

(43) Veröffentlichungstag der Anmeldung:
**22.10.2014 Patentblatt 2014/43**

(73) Patentinhaber: **Merck Patent GmbH**
**64293 Darmstadt (DE)**

(72) Erfinder:
- **BUCHHOLZ, Herwig**
  **60599 Frankfurt am Main (DE)**
- **PAN, Junyou**
  **60320 Frankfurt am Main (DE)**
- **HEUN, Susanne**
  **65812 Bad Soden (DE)**

(56) Entgegenhaltungen:
**EP-A1- 0 032 373** **EP-A1- 2 067 839**
**WO-A1-2006/008068**

- **KEIVANIDIS PANAGIOTIS E ET AL: "Up-Conversion Photoluminescence in Polyfluorene Doped with Metal(II)-Octaethyl Porphyrins", ADVANCED MATERIALS, WILEY VCH VERLAG, DE, Bd. 15, Nr. 24, 17. Dezember 2003 (2003-12-17), Seiten 2095-2098, XP002352203, ISSN: 0935-9648, DOI: 10.1002/ADMA.200305717**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 791 275 B1

## Beschreibung

Gegestand der vorliegenden Erfindung ist die Bereistellung von neuen Zusammensetzungen enthaltend Sensibilisatoren für Up-Conversion mittels TTA (Triplett-Triplett Annihilation), sowie elektronische Vorrichtungen enthaltend diese Zusammensetzungen.

Unter Up-Conversion (UpC) versteht man ganz allgemein die Erzeugung von Exzitonen hoher Energie aus Exzitonen niedriger Energie, wobei die Exzitonen niedriger Energie entweder durch elektrische, elektromagnetische oder optische Anregung erzeugt werden und die Energie der Exzitonen hoher Energie zumindest teilweise in Form von Photonen wieder abgegeben wird. UpC wurde bereits an einer Reihe organischer Materialien beobachtet (T. Kojei et al., Chem. Phys. Lett. 1998, 298, 1; G. S. He et al., Appl. Phys. Lett. 1996, 68, 3549; R. Schroeder et al., J. Chem. Phys. 2002, 116, 3449; J. M. Lupton, Appl. Phys. Lett. 2002, 80, 186; C. Bauer et al., Adv. Mater. 2002, 14, 673).

Um UpC zu erreichen, werden unterschiedliche Verfahren eingesetzt, beispielsweise die simultane Absorption von zwei oder mehr Photonen niedriger Energie unter Verwendung köhärenter Lichtquellen (Laser). Allerdings benötigt man hierzu sehr hohe Lichtintensitäten im Bereich von $MW/cm^2$ bis zu $GW/cm^2$, da die beiden Photonen niedriger Energie praktisch zeitgleich absorbiert werden müssen. Hierbei handelt es sich um einen nicht-linear optischen Effekt, der im allgemeinen mit relativ geringer Konversionseffizienz verläuft. Ein anderes Verfahren sieht die sequentielle Multiphotonen Absorption vor. Auch hier werden sehr hohe Intensitäten benötigt, da mit den später eintreffenden Photonen bereits angeregte Zustände weiter angeregt werden müssen. Da die Nutzung dieser Mechanismen mit einem großen Aufwand verbunden ist und trotzdem im allgemeinen nur zu geringen Energiedichten gedoppelter Strahlung führt, stellen sie ein Hindernis für die praktische Anwendung dar. Diese wäre aber sehr wünschenswert, da oft nur der energiereichere Teil des Spektrums (blau) für Anwendungen wirksam ist, die Stabilität hochenergetischer blauer Zustände jedoch deutlich geringer ist als diejenige niedrigenergetischer roter.

UpC wurde, beispielsweise, in einem System aus einem methylsubstituierten Leiterpolymer (MeLPPP) dotiert mit Platin-octaethylporphyrin (PtOEP) als Sensibilisator (S. A. Bagnich, H. Bässler, Chem. Phys. Lett. 2003, 381, 464) und an Polyfluoren, dotiert mit Metall(II)-octaethylporphyrin, beschrieben (P. E. Keivanidis et al., Adv. Mater. 2003, 15, 2095). Durch die Anwesenheit des Metallkomplexes konnten die Pumpintensitäten des Lasers im Vergleich zur Up-Conversion an einfachen Polyfluoren-Systemen, die keine Metallkomplexe enthielten, um fünf Größenordnungen reduziert werden. Jedoch ist die Effizienz der Up-Conversion gering, das Verhältnis der integrierten Photolumineszenz des Polyfluorens bei Anregung des Metallkomplexes im Vergleich zur direkten Anregung des Polyfluorens wurde für Palladium-porphyrin zu 1 : 5000 ermittelt. Durch die Verwendung von Platin-Porphyrin konnte die Effizienz um den Faktor 18 verbessert werden, so dass sich ungefähr ein Verhältnis von 1 : 300 ergibt. Allerdings bleibt in diesen Systemen die Emission des Metallkomplexes deutlich wahrnehmbar und ist somit ein störender Verlustkanal.

Gelänge es, einen effizenten Prozess zur UpC zu nutzen, hätte das für zahlreiche Anwendungen weitreichende Konsequenzen. So liegt eine mögliche Anwendung im Bereich organischer Solarzellen (O-SC). Bisher tragen hier meistens nur sichtbare (typischerweise blaue und grüne) sowie die UV-Anteile des einfallenden Lichtes zur Erzeugung freier Ladungsträger bei, da in diesem Prozeß die Bindungsenergie von Loch und Elektron überwunden werden muß. Mit Hilfe von UpC kann aber auch rotes oder sogar infrarotes Licht zur Deviceeffizienz beitragen, wenn daraus energiereicheres blaues oder grünes Licht erzeugt werden kann, das dann wiederum absorbiert werden oder via Förster-Transfer hochenergetische Exzitonen erzeugen kann. Gegebenenfalls können sogar direkt freie Ladungsträger erzeugt werden.

Eine weitere Anwendung liegt im Bereich organischer Leuchtdioden, beispielsweise zur Erzeugung blauen oder weißen Lichts durch gleichzeitige Emission von blau und rot/gelb. Bestehende Anwendungen, die von einer effizienteren UpC profitieren würden, sind z.B. im Bereich organischer blauer Laser, die mit kommerziell erhältlichen grünen oder rotenLasern gepumpt werden können. So können die Streuung und die lineare Absorption reduziert und die Photostabilität des Materials erhöht werden. Eine weitere Anwendungsmöglichkeit ist beispielsweise bei Vernetzungsreaktionen, wobei durch Verwendung von grünem Licht UV-Licht erzeugt werden kann, dessen Einwirkung auf einen Sensibilisator die Vernetzungsreaktion initiieren kann. Nochmals eine weitere Anwendungsmöglichkeit sind Schalter, bei denen nur eine bestimmte Wellenlänge, bei der ein relativ schmalbandiger Sensibilisator absorbiert, die Emission von blauem Licht triggert. Weiterhin möglich sind Systeme zur optischen Datenspeicherung, biologische bzw. medizinische Anwendungen, etc..

Ein weiteres vielversprechendes Verfahren, um UpC zu erreichen ist die Triplett-Triplett-Annihilation (TTA; Abbildung 1; Cheng et al., Phys. Chem. Chem. Phys., 12, 66 (2010); Baluschev et al. Appl. Phys. Lett. 90, 181103 (2007); J. E. Auckett et al. J. Phys: Conference Series 185 (2009) 012002). Ein Sensibilisator (I) wird mittels der Energie $E_{in}$ aus dem Grundzustand $S_0$ in einen angeregten Singulett Zustand ($S_1$) angeregt. Es kommt zum Intersystem Crossing (ISC), d.h. unter Spinumkehr zum Übergang in den ersten angeregten Triplett-Zustand $T_1$. Danach kommt es zur Übertragung der Erergie aus $T_1$ des Sensibilisators auf das $T_1$ Niveau des Akzeptors (II) (TTET - Triplett-Triplett Energietransfer), wobei als Konkurrenzprozess die Phosphoreszenz $h\nu_I$ aus $T_1$ des Sensibilisators möglich ist. Schließlich führt ein bimolekularer Stoß unter zwei Akzeptoren, die sich beide im angeregten $T_1$ Zustand befinden, dazu, dass der eine Akzeptor in den angeregten $S_n$ Zustand und der andere Akzeptor in den elektronischen Grundzustand $S_0$ überführt wird

($T_1$+$T_1$ → $S_n$+$S_0$). Nach Relaxation (IC-Internal Conversion) von $S_n$ auf $S_1$ erfolgt die Emission $h\nu_{out}$ des Akzeptors aus dem $S_1$ Zustand. Ein entscheidender Vorteil von TTA-Up-Conversion (TTA-UpC) liegt darin, dass sie unabhängig von der Entstehungsgeschichte und, sofern sich die Moleküle in ausreichender Nähe zueinander befinden, von der Besetzungsdichte der Zustände ist.

Auch zur TTA-UpC werden als Sensibilisatoren meistens organische Metallkomplexe eingesetzt, denn die Anwesenheit eines Schweratoms erhöht aufgrund der Spin-Bahn-Kopplung die Intersystem Crossing Rate (ISC) erheblich. Aufgrund dieser starken Spin-Bahn-Kopplung ist allerdings auch die strahlende Übergangswahrscheinlichkeit von $T_1$ zu $S_0$ (Phosphoreszenz) erhöht, was zu einer Effizienzminderung der TTA-UpC und einer zusätzlichen, nicht upkonvertierten Emission führt. Bis heute sind nur wenige organische Sensibilisatoren für TTA-UpC bekannt, die keine Schweratome enthalten. In einer kürzlich publizierten Arbeit von J. Zhao et al. (RSC Advances, 1, 937 (2011)) wurden organische Sensibilisatoren für die Anwendung in der Photolumineszenz publiziert, die statt des typischen Metallions eine Jodsubstitution nutzen. Es handelt sich um auf BODIPY basierte Moleküle, die einerseits die bekannt hohe Absorption dieser Klasse von Fluoreszenzfarbstoffen nutzen, die Fluoreszenzquantenausbeute aber aufgrund der erhöhten ISC-Rate zurückdrängen. Die Substitution mit Jod ist jedoch für die Anwendung in organischen Elektrolumineszenzvorrichtungen unerwünscht. Weiterhin offenbaren die Autoren wenige weitere organische Sensibilisatoren (2,3-Butandion, Acridon und Diphenylketon), die sich für UpC im Rahmen der Photolumineszenz eignen, für Elektrolumineszenzanwendungen jedoch wegen ihrer niedrigen Siedepunkte bzw. elektronischen Instabilität ungeeignet sind.
WO 2006(008068 A1 beschreibt ein System, das Up-Conversion ermöglicht und das ein Polymer, welches ein kondensiertes aromatisches Ringsystem als Emittereinheit enthält, sowie einen schweratomhaltigen Sensibilisator umfasst.

EP 2067839 A1 offenbart ein Zweikomponentensystem, das mittels TTA Up-Conversion ermöglicht. Hierfür werden meso-Tetraphenyltetrabenzoporphyrin Palladium (PdTPTBP) als Sensibilisator und Perylen als Emitter eingesetzt, Als alternative Sensibilisatoren werden in EP 2067839 A1 Coumarinderivate beschrieben.
cWegen der relativ geringen UpC -Effizienz der bisherigen Systeme haben Wegen der relativ geringen UpC -Effizienz der bisherigen Systeme haben UpC-Prozesse, unabhängig vom zugrundeliegenden physikalisches Prinzip, bisher in der Optoelektronik keine Rolle gespielt. Zur Nutzung roten Lichtes in organischen Solarzellen müsste dort in signifikantem Anteil blaues Licht entstehen, um so zusätzlich freie Ladungsträger zu generieren. In OLEDs entstehen aufgrund der Spin-Statistik Triplett-zustände in großer Zahl, die jedoch im allgemeinen nicht-leuchtend sind und also einen Verlustkanal darstellen (Ausnahme sind die schon genannten Bauteile mit organometallischen Schweratomkomplexen in der Emissionsschicht). Mit Hilfe von effizienter UpC könnten diese nichtemittierenden Zustände zu emittierenden höherenergetischen Singulettzuständen umgewandelt werden und so doch zur Deviceeffizienz beitragen.

Daherh wäre es für die Anwendung wünschenswert, eine deutliche weitere Steigerung der TTA-UpC zu erreichen und die Effizienz der existierenden Systeme und Sensibilisatoren weiter zu steigern. Dies war die der Erfindung zugrunde liegende Aufgabe.

Es wurde nun überraschend gefunden, dass bestimmte organische Sensibilisatoren ohne Schweratome für TTA-UpC besonders gut geeignet sind, sehr gute Effizienzen zeigen und keine störende Restemission aufweisen. Diese eigenen sich sowohl als Sensibilisatoren für die Photolumineszenz als auch für die Elektrolumineszenz.

Gegenstand der vorliegenden Erfindung ist daher eine Zusammensetzung für Up-Conversion, vorzugsweise für Up-Conversion in elektrolumineszierenden Vorrichtungen, enthaltend wenigstens einen Sensibilisator, der ein Polymer, Oligomer, Dendrimer oder kleines Molekül, das dadurch definiert ist, dass es ein Molekulargewicht kleiner als 4000 g/mol aufweist, darstellt und wenigstens einen fluoreszierenden organischen Emitter, dadurch charakterisiert, dass der Sensibilisator Struktureinheiten enthält, die aus den folgenden Verbindungen mit den allgemeinen Formel (1) ausgewählt sind, mit der Maßgabe, dass der Emitter kein organischer Metallkomplex ist

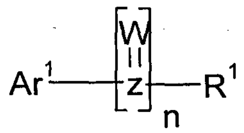

Formel (1)

wobei für die verwendeten Symbole gilt:

n ist entweder 1, 2 oder 3, bevorzugt 1 oder 2 und ganz bevorzugt 1;

W ist gleich oder verschieden bei jedem Auftreten gleich 0, S oder Se, bevorzugt O oder S, ganz bevorzugt O;

$Ar^1$ ein aromatischer oder heteroaromatischer Ring oder ein aromatisches oder heteroaromatisches Ringsystem,

wobei die Ringe mit einem oder meheren Resten $R^1$ substituiert sein können, mit der Maßgabe, dass $Ar^1$ wenigstens 9 Ringatome enthält;

Z ist gleich oder verscheiden bei jedem Auftreten C, S oder S(=O), bevorzugt C;

$R^1$ ist gleich oder verschieden bei jedem Auftreten H, D, F, Cl, Br, I, $N(R^2)_2$, CN, $Si(R^2)_3$, $B(OR^2)_2$, $C(=O)R^2$, $P(=O)(R^2)_2$, $S(=O)R^2$, $S(=O)_2R^2$, $OSO_2R^2$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten $R^2$ substituiert sein kann, wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy-, Arylalkoxy- oder Heteroaryloxygruppe mit 5 bis 60 aromatischen Ringatomen, die durch einen oder mehrere Reste $R^2$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^2$ substituiert sein kann, oder eine Kombination aus zwei oder mehr dieser Gruppen oder eine vernetzbare Gruppe Q;

$R^2$ ist gleich oder verschieden bei jedem Auftreten H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $R^3C{=}CR^3$, C=C, $Si(R^3)_2$, $Ge(R^3)_2$, $Sn(R^3)_2$, C=O, C=S, C=Se, $C{=}NR^3$, $P(=O)(R^3)$, SO, $SO_2$, $NR^3$, O, S oder $CONR^3$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen Ringatomen, das jeweils durch einen oder mehrere Reste $R^3$ substituiert sein kann, oder eine Aryloxy-, Arylalkoxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination aus zwei oder mehr dieser Gruppen; dabei können zwei oder mehrere benachbarte Reste $R^2$ miteinander ein mono- oder polycyclisches, aliphatisches oder aromatisches Ringsystem bilden;

$R^3$ ist gleich oder verschieden bei jedem Auftreten H, D, F oder ein aliphatischer, aromatischer und/oder heteroaromatischer Kohlenwasserstoffrest mit 1 bis 20 C-Atomen, in dem auch ein oder mehrere H-Atome durch F ersetzt sein können; dabei können zwei oder mehrere Substituenten $R^3$ auch miteinander ein mono- oder polycyclisches, aliphatisches oder aromatisches Ringsystem bilden.

"Vernetzbare Gruppe" in Sinne der vorliegenden Erfindung bedeutet eine funktionelle Gruppe, die in der Lage ist, irreversibel zu reagieren. Dadurch wird ein vernetztes Material gebildet, das unlöslich ist. Die Vernetzung kann gewöhnlich durch Wärme oder durch UV-, Mikrowellen-, Röntgen- oder Elektronenstrahlung unterstützt werden. Beispiele für vernetzbare Gruppen Q sind Einheiten, die eine Doppelbindung, eine Dreifachbindung, eine Vorstufe, die zu einer in situ Bildung einer Doppel- bzw. Dreifachbindung in der Lage ist, oder einen heterocyclischen additionspolymerisierbaren Rest enthalten. Bevorzugte Reste Q umfassen Vinyl, Alkenyl, vorzugsweise Ethenyl und Propenyl, $C_{4-20}$-Cycloalkenyl, Azid, Oxiran, Oxetan, Di(hydrocarbyl)amino, Cyanatester, Hydroxy, Glycidylether, $C_{1-10}$-Alkylacrylat, $C_{1-10}$-Alkylmethacrylat, Alkenyloxy, vorzugsweise Ethenyloxy, Perfluoralkenyloxy, vorzugsweise Perfluorethenyloxy, Alkinyl, vorzugsweise Ethinyl, Maleimid, Tri($C_{1-4}$)-alkylsiloxy und Tri($C_{1-4}$)-alkylsilyl. Besonders bevorzugt ist Vinyl und Alkenyl.

Ein kleines Molekül im Sinne der vorliegenden Erfindung ist ein Molekül, das kein Polymer, Oligomer oder Dendrimer oder eine Mischung (Blend) hieraus ist. Insbesondere unterscheiden sich kleine Moleküle von Polymeren, Oligomeren oder Dendrimeren dadurch, dass sie keine Wiederholungseinheiten aufweisen. Das Molekulargewicht kleiner Moleküle ist typischerweise im Bereich von Polymeren und Oligomeren mit wenigen Wiederholungseinheiten und darunter. Das Molekulargewicht kleiner Moleküle ist bevorzugt kleiner als 4000 g/mol, ganz bevorzugt kleiner als 3000 g/mol und ganz besonders kleiner als 2000 g/mol.

Polymere haben 10 bis 10000, bevorzugt 20 bis 5000 und ganz bevorzugt 50 bis 2000 Wiederholungseinheiten. Oligomere haben 2 bis 9 Wiederholungseinheiten. Der Verzweigungsindex von Polymeren und Oligomeren liegt zwischen 0 (lineares Polymer ohne Verzweigung) und 1 (vollständig verzweigtes Polymer). Der Begriff Dendrimer wird hierin so verstanden wie durch M. Fischer et al. in Angew. Chem., Int. Ed. 1999, 38, 885 beschrieben.

Das Molekulargewicht (Mw) von Polymeren liegt bevorzugt im Bereich zwischen etwa 10000 und etwa 2000000

g/mol, ganz bevorzugt zwischen etwa 100000 und etwa 1500000 g/mol, und ganz besonder bevorzugt zwischen etwa 200000 und etwa 1000000 g/mol. Die Bestimmung von Mw erfolgt nach Methoden, die dem Fachmann wohl bekannt sind, mittels Gelpermeationschromatographie (GPC) mit Polystyrol als innerem Standard, zum Beispiel.

Unter einer Mischung (Blend) wird eine Mischung verstanden, die wenigstens eine polymere, dendritische oder oligomere Komponente enthält.

Unter der Triplett-Energie einer Verbindung wird die Energiedifferenz zwischen dem niedrigsten Triplettzustand $T_1$ und dem Singulett-Grundzustand So verstanden.

Die Energiedifferenz zwischen $T_1$ und So, hiernach einfach Triplett Niveau $T_1$ genannt, kann sowohl mittels Spektroskopie als auch mittels quantenchemischer Simulation (Time-Dependent DFT) berechnet werden. Für organische Verbindungen, die kein Metall enthalten, kann $T_1$ durch zeitaufgelöste Spektroskopie bei tiefen Temperaturen wie folgt gemessen werden: 100 nm eines zum Beispiel durch Spincoating hergestellten Films oder einer amorphen aufgedampften Schicht auf Quarzglas werden durch einen getripelten YAG-Laser (@ 355 nm) oder einen $N_2$-Laser (@ 337 nm) bei Helium-Temperatur (< 10 K) angeregt. Die verzögerte Photolumineszenz wird durch sogenannte "gegatete" Detektion nach einer bestimmten Zeit (z.B. 1 $\mu$s) aufgezeichnet. Die Wellenlänge der Emission im Zeitfenster der verzögerten Lumineszenz entspricht dann dem Übergang $T_1$ nach So und damit, umgerechnet in Energiewerte, dem $T_1$ Niveau des untersuchten Systems. Die Simulationsmethode für $T_1$ wird in den Beispielen näher bschrieben. Die Korrelation zwischen Messung und Simulation ist bekanntermaßen sehr gut.

-Unter einem Sensibilisator im Sinne dieser Erfindung wird eine Verbindung verstanden, die Licht im Bereich der eingestrahlten Wellenlänge absorbiert und danach durch Intersystem-Crossing (ISC) in einen Triplett-Zustand übergeht, oder die unter elektrischer Anregung durch die Rekombination von Elektronen und Löchern Triplett-Zustände erzeugt und diesen gegebenenfalls nachfolgend auf das Akzeptormolekül durch Triplett-Triplett-Energietransfer (TTET) übertragen kann. Dabei liegt das Triplett-Niveau des Sensibilisators oberhalb des Triplett-Niveaus des Akzeptormoleküls.

Unter Phosphoreszenz im Sinne der vorliegenden Anmeldung wird Lumineszenz aus einem angeregten Zustand mit höherer Spinmultiplizität verstanden, also aus einem Zustand mit einer Spinquantenzahl S größer oder gleich 1. Bevorzugt ist unter Phosphoreszenz im Sinne der vorliegenden Erfindung Lumineszenz zu verstehen, bei der Strahlung aus einem angeregten Triplett (S=1, 2S+1=3) und/oder aus einem angeregten Quintett (S=2, 2S+1=5) Zustand emittiert wird, ganz bevorzugt aus einem angeregten Triplett-Zustand.

Unter Fluoreszenz im Sinne der vorliegenden Erfindung wird Lumineszenz aus einem angeregten Singulett-Zustand verstanden, vorzugsweise aus dem ersten angeregten Singulett-Zustand $S_1$.

Eine Arylgruppe im Sinne dieser Erfindung enthält 6 bis 40 C-Atome; eine Heteroarylgruppe im Sinne dieser Erfindung enthält 1 bis 39 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 60 C-Atome im Ringsystem. Ein heteroaromatisches Ringsystem im Sinne dieser Erfindung enthält 1 bis 59 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verknüpft sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe unterbrochen sind. Weiterhin sollen Systeme, in denen mehrere Aryl- und/oder Heteroarylgruppen durch eine Einfachbindung miteinander verknüpft sind, wie z. B. Biphenyl, Terphenyl oder Bipyridin, als aromatisches bzw. heteroaromatisches Ringsystem verstanden werden.

Im Rahmen der vorliegenden Erfindung werden unter einem aliphatischen Kohlenwasserstoffrest bzw. einer Alkylgruppe bzw. einer Alkenyl- oder Alkinylgruppe, die typischerweise 1 bis 40 oder auch 1 bis 20 C-Atome enthalten kann, und in der auch einzelne H-Atome oder $CH_2$-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy,

Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte $CH_2$-Gruppen durch $R^1C=CR^1$, C=C, $Si(R^1)_2$, $Ge(R^1)_2$, $Sn(R^1)_2$, C=O, C=S, C=Se, $C=NR^1$, $P(=O)(R^1)$, SO, $SO_2$, $NR^1$, O, S oder $CONR^1$ ersetzt sein können; weiterhin können auch ein oder mehrere H-Atome durch D, F, Cl, Br, I, CN oder $NO_2$, bevorzugt F, Cl oder CN, weiter bevorzugt F oder CN, besonders bevorzugt CN ersetzt sein.

Unter einem aromatischen oder heteroaromatischen Ringsystem mit 5 - 60 aromatischen Ringatomen, welches noch jeweils mit den oben genannten Resten $R^1$ oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenonthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Die Tatsache, dass der Sensibilisator Struktureinheiten enthält, die aus den folgenden Verbindungen mit der allgemeinen Formel (1) ausgewählt werden, bedeutet, dass der Sensibilisator entweder genau den Verbindungen der Formel (1) entspricht oder die Strukturen der Formel (1) als Substruktur enthält. So kann es sich hierbei auch um Polymere, Oligomere oder Dendrimere handeln, in die Strukturen gemäß der Formel (1) eingebaut wurden.

Die Substituenten $Ar^1$ und $R^1$ in der Verbindung der Formel (1) können auch durch kovalente Bindungen miteinander verbunden sein, um beispielsweise ein cyclisches oder polycyclisches Ringsystem zu bilden.

Es ist bevorzugt im Sinne der vorliegenden Erfindung, dass der Sensibilisator ein kleines Molekül mit der Struktur der allgemeinen Formel (1) ist.

In einer bevorzugten Ausführungsform der vorliegende Erfindung ist der Sensibilisator gemäß Formel (1) ausgewählt aus den Verbindungen der Formeln (3) bis (7), ganz bevorzugt aus den Verbindungen der Formeln (3), (4) und (5), ganz besonders bevorzugt aus den Verbindungen der Formeln (3) und (5) und insbesondere bevorzugt aus den Verbindungen der Formel (3).

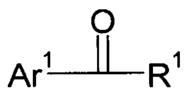

Formel (3)

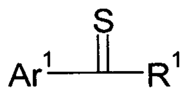

Formel (4)

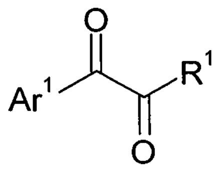

Formel (5)

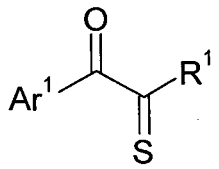

Formel (6)

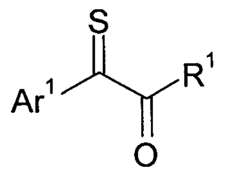

Formel (7)

wobei die Reste $Ar^1$ und $R^1$ wie oben angegeben definiert sind.

Bevorzugte Gruppen für $Ar^1$ sind ausgewählt aus der Gruppe der aromatischen oder heteroaromatischen Ringe oder Ringsysteme, bspw. aus der Gruppe der Fluorene, Spriobifluorene, Phenanthrene, Indenofluorene, Carbazolen, Indenocarbazolen, Indolocarbazole, Dihydrophenanthrene, Naphtalinen, Antharcene, Pyrene, Triazine und Benzanthracene, Triarylamine, Dibenzofuran, Azaborole, Diazasilole, Diazaphosphole, Azacarbazole, Benzidine, Tetraaryl-para-phenylendiamine, Triarylphosphine, Phenothiazine, Phenoxazine, Dihydrophenazine, Thianthrene, Dibenzo-paradioxine, Phenoxathiine, Azulene, Perylenylene, Biphenylylene, Terphenylylene, Tolanylene, Stilbenylene, Bisstyrylarylene, Benzothiadiazole, Chinoxaline, Phenothiazine, Phenoxazine, Dihydrophenazine, Bis(thiophenyl)-arylene, Oligo(thiophenylen)e, Phenazine, Rubrene, Pentacene, Perylene sowie von Derivaten hiervon.

Beispiele hierfür sind 4,5-Dihydropyrene, 4,5,9,10-Tetrahydropyrene und Fluorene wie in US 5,962,631, WO 2006/052457 A2 und in WO 2006/118345A1 offenbart, 9,9'-Spiro¬bifluorene wie in WO 2003/020790 A1 offenbart, 9,10-Phenanthrene wie in WO 2005/104264 A1 offenbart, 9,10-Dihydrophenanthrene wie in WO 2005/014689 A2 offenbart, 5,7-Dihydro¬dibenzo¬oxepine und cis- und trans-Indenofluorene wie in WO 2004041901 A1 und WO 2004113412 A2 offenbart, Binaphthylene wie in WO 2006/063852 A1 offenbart und weitere Einheiten wie in WO 2005/056633A1, EP 1344788A1, WO 2007/043495A1, WO 2005/033174 A1, WO 2003/099901A1 und DE 102006003710 offenbart.

Bevorzugt ist $Ar^1$ in den Formeln (1) bis (7) ausgewählt aus den Gruppen der folgenden Formeln (8) bis (14)

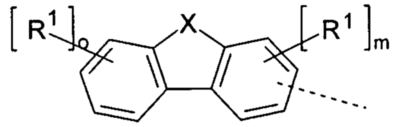

Formel (8)

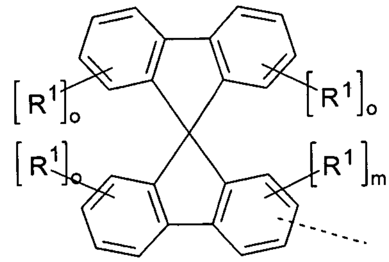

Formel (9)

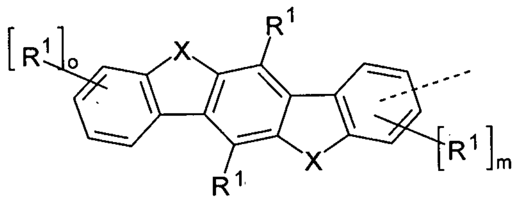

Formel (10)

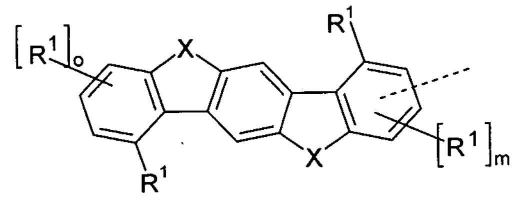

Formel (11)

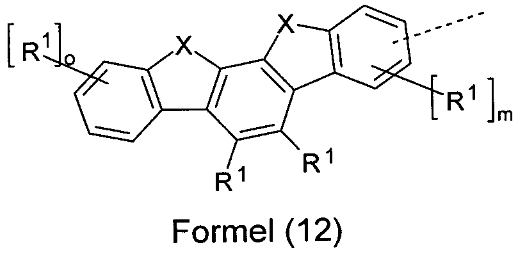

Formel (12)

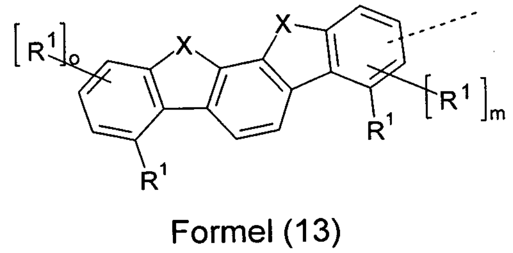

Formel (13)

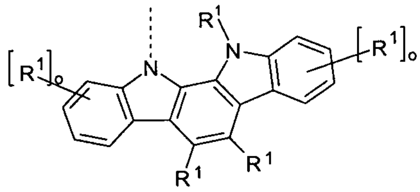

Formel (14)

wobei $R^1$ dieselbe Bedeutung hat, wie oben beschrieben, die gestrichelte Bindung die Verknüpfungsposition darstellt, und weiterhin gilt:

X ist gleich oder verschieden bei jedem Auftreten eine bivalente Brücke, ausgewählt aus $B(R^1)$, $C(R^1)_2$, $Si(R^1)_2$, C=O, $C=NR^1$, $C=C(R^1)_2$, O, S, S=O, $SO_2$, $N(R^1)$, $P(R^1)$ und $P(=O)R^1$;

□m ist bei jedem Auftreten gleich oder verschieden 0, 1, 2 oder 3;

m ist bei jedem Auftreten gleich oder verschieden 0, 1, 2 oder 3;

◦ ist bei jedem Auftreten gleich oder verschieden 0, 1, 2, 3 oder 4.

Besonders bevorzugte Gruppen $Ar^1$ sind gewählt aus den Gruppen der folgenden Formeln (15) bis (23),

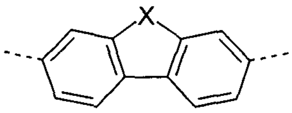

Formel (15)

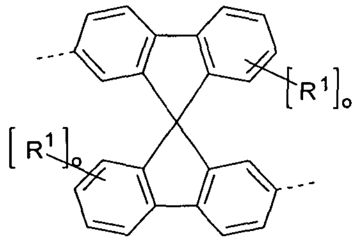

Formel (16)

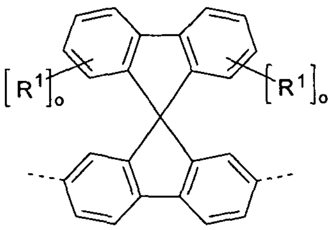

Formel (17)

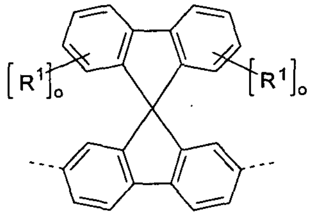

Formel (18)

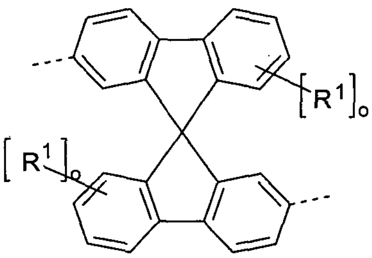

Formel (19)

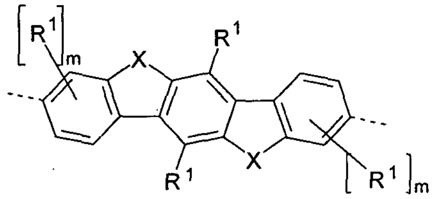

Formel (20)

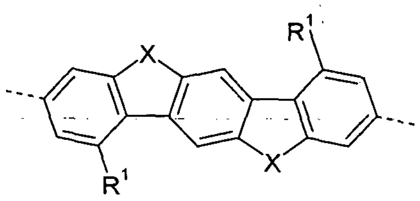

Formel (21)

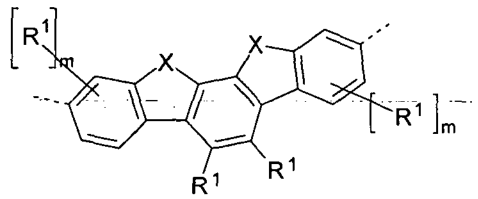

Formel (22)

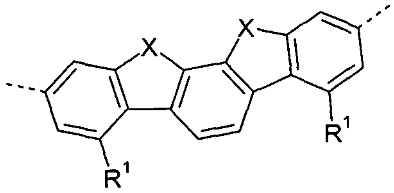

Formel (23)

wobei die verwendeten Symbole und Indizes dieselbe Bedeutung haben, wie oben beschrieben. Dabei ist X bevorzugt gleich oder verschieden gewählt aus $C(R^1)_2$, $N(R^1)$, O und S, besonders bevorzugt $C(R^1)_2$.

In einer weiterhin bevorzugten Ausführungsform der vorliegenden Erfindung ist $R^1$ in den Verbindungen der Formeln (8) bis (23) gleich $Ar^1$. Weiterhin bevorzugt is X gleich oder verschieden gewählt aus $C(R^2)_2$, $N(R^2)$, O und S, besonders bevorzugt $C(R^2)_2$, wobei $R^2$ wie in Formel (1) und (2) definiert ist.

$R^1$ in den verbindungen der Formeln (1) bis (23) ist bevorzugt gleich oder verschieden bei jedem Auftreten $N(R^2)_2$, CN, $Si(R^2)_3$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten $R^2$ substituiert sein kann, wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy-, Arylalkoxy- oder Heteroaryloxygruppe mit 5 bis 60 aromatischen Ringatomen, die durch einen oder mehrere Reste $R^2$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^2$ substituiert sein kann, oder eine Kombination aus zwei oder mehr dieser Gruppen oder eine vernetzbare Gruppe Q;

Ganz bevorzugt ist $R^1$ in den Formeln (3) bis (7) gleich oder verschieden bei jedem Auftreten ausgewählt aus einer der folgenden Formeln (34) bis (222), wobei die Verbindungen mit den angegebenen Formeln (34) bis (222) mit einem oder meherern, identischen oder verschiedenen Resten $R^2$ substituiert sein können, wobei $R^2$ oben defineirt wurde.

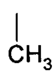

Formel (34)

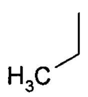

Formel (35)

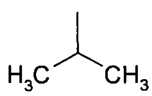

Formel (36)

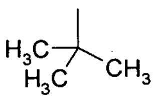

Formel (37)

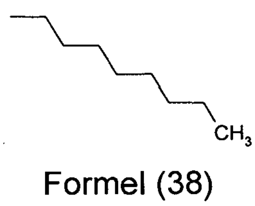

Formel (38)

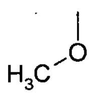

Formel (39)

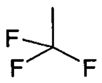

Formel (40)

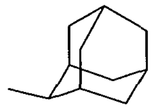

Formel (41)

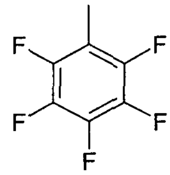

Formel (42)

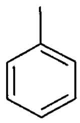

Formel (43)

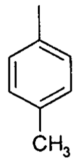

Formel (44)

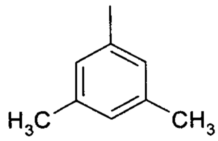

Formel (45)

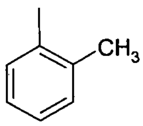

Formel (46)

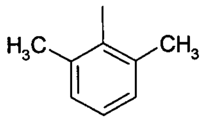

Formel (47)

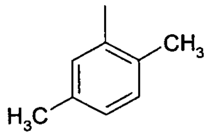

Formel (48)

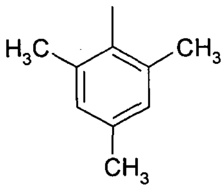

Formel (49)

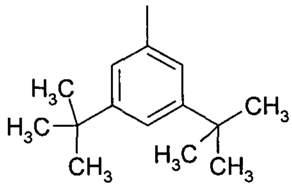

Formel (50)

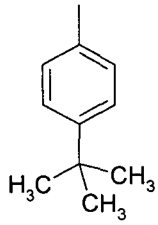

Formel (51)

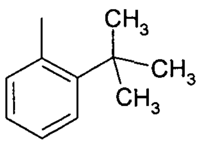

Formel (52)

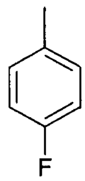

Formel 53)

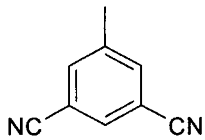

Formel (54)

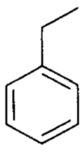

Formel (55)

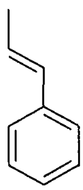

Formel (56)

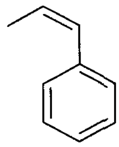

Formel (57)

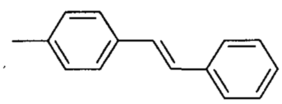
Formel (58)

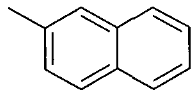
Formel (59)

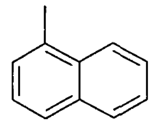
Formel (60)

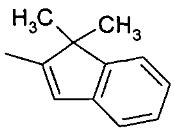
Formel (61)

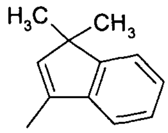
Formel (62)

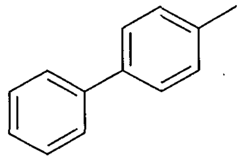
Formel (66)

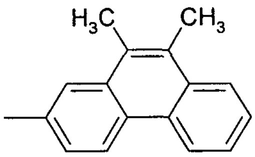
Formula (67)

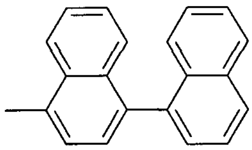
Formula (68)

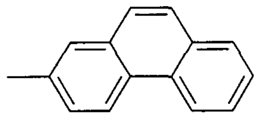
Formel (69)

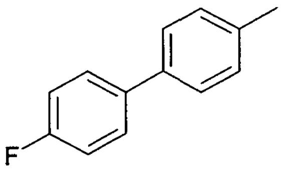
Formel (70)

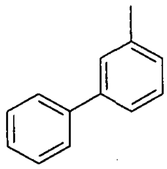
Formel (71)

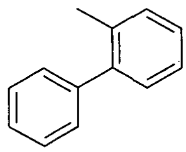
Formel (72)

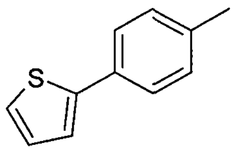
Formel (73)

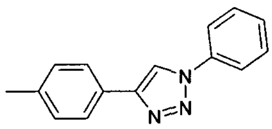
Formel (74)

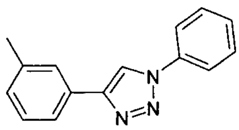
Formel (75)

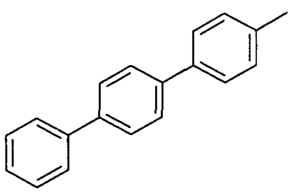
Formel (76)

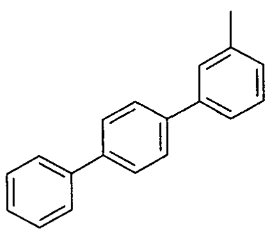
Formel (77)

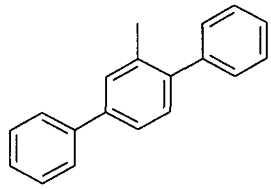
Formel (78)

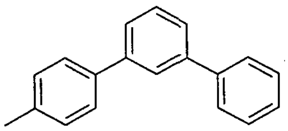
Formel (79)

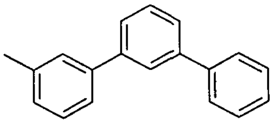
Formel (80)

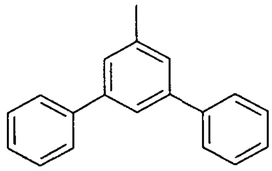
Formel (81)

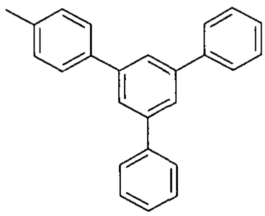
Formel (82)

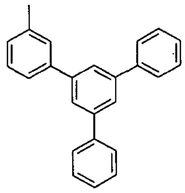
Formel (83)

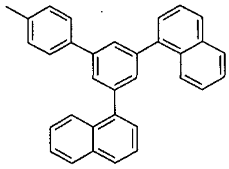
Formel (84)

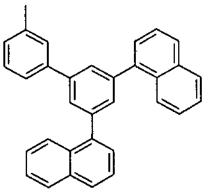
Formel (85)

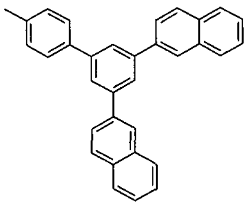
Formel (86)

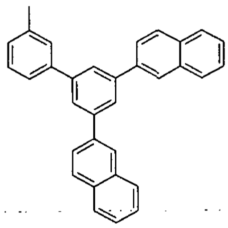
Formel (87)

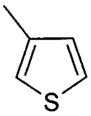
Formel (91)

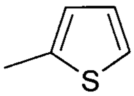
Formel (92)

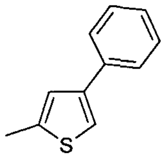
Formel (93)

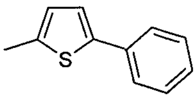
Formel (94)

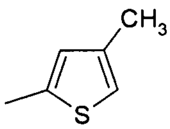
Formel (95)

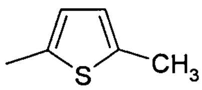
Formel (96)

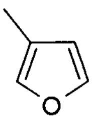
Formel (97)

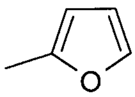
Formel (98)

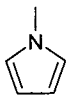
Formel (99)

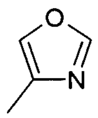
Formel (100)

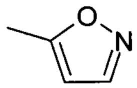
Formel (101)

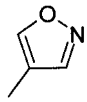
Formel (102)

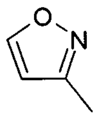
Formel (103)

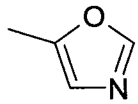
Formel (104)

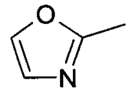
Formel (105)

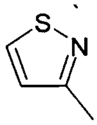
Formel (106)

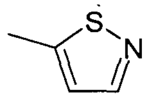
Formel (107)

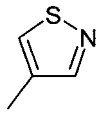
Formel (108)

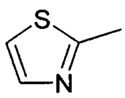
Formel (109)

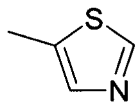
Formel (110)

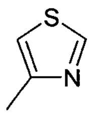
Formel (111)

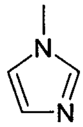
Formel (112)

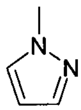
Formel (113)

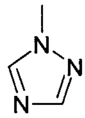
Formel (114)

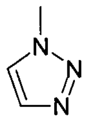
Formel (115)

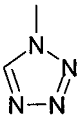
Formel (116)

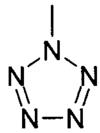
Formel (117)

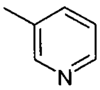
Formel (118)

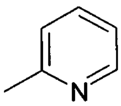
Formula (119)

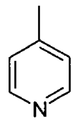
Formel (120)

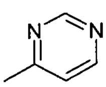
Formel (121)

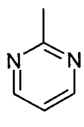
Formula (122)

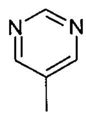
Formel (123)

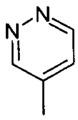
Formel (124)

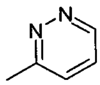
Formel (125)

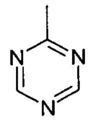
Formel (126)

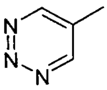
Formel (127)

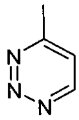
Formel (128)

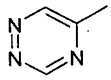
Formel (129)

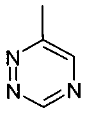
Formel (130)

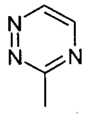
Formel (131)

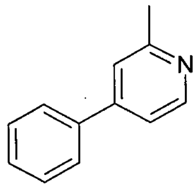
Formel (132)

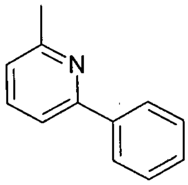
Formel (133)

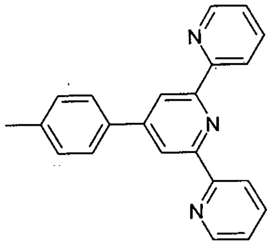
Formel (134)

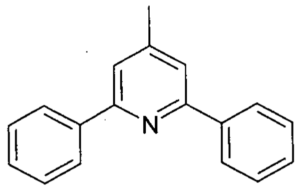
Formel (135)

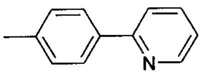
Formel (136)

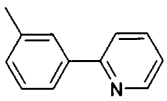
Formel (137)

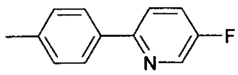
Formel (138)

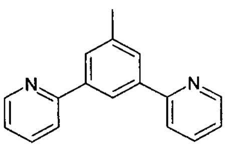
Formel (139)

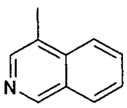
Formel (140)

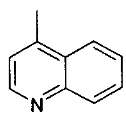
Formel (141)

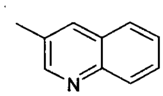

Formel (142)

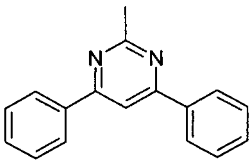

Formel (143)

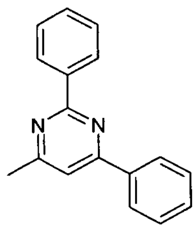

Formel (144)

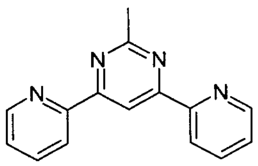

Formel (145)

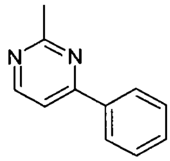

Formel (146)

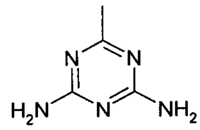

Formel (147)

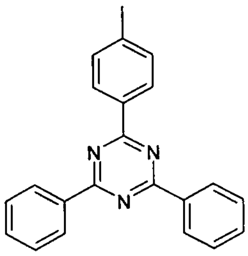

Formel (148)

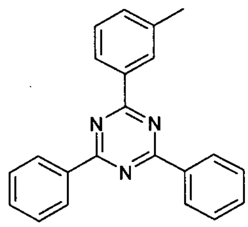

Formel (149)

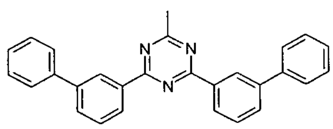

Formel (150)

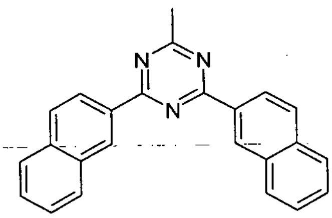

Formel (151)

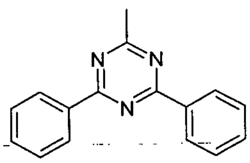

Formel (152)

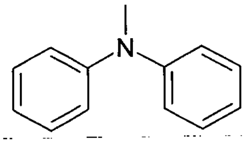

Formel (153)

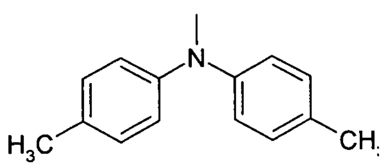

Formel (154)

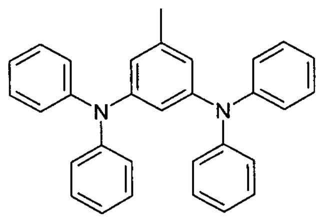

Formel (155)

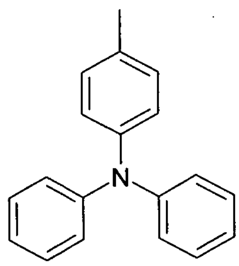

Formel (156)

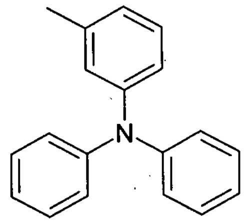

Formel 157)

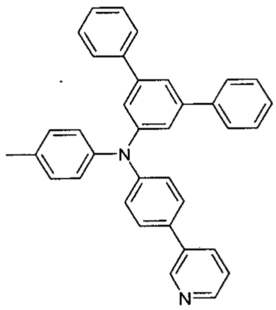

Formel (160)

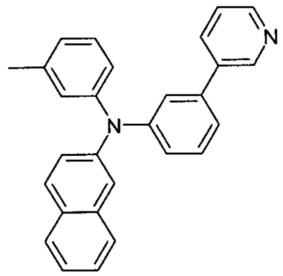

Formel (161)

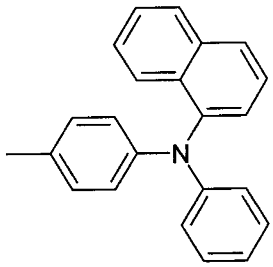

Formel (162)

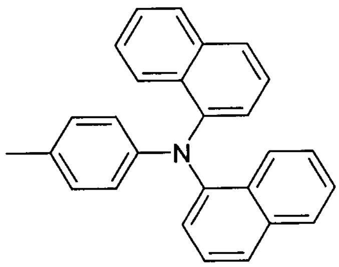

Formel (163)

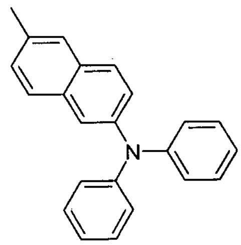

Formel (164)

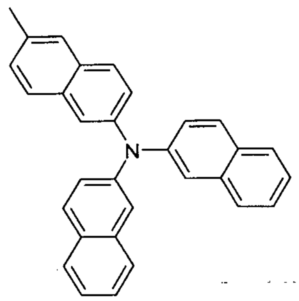

Formel (165)

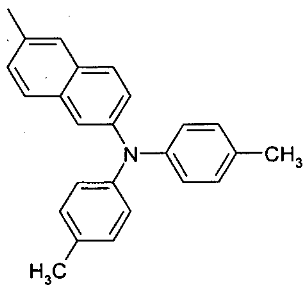

Formel (166)

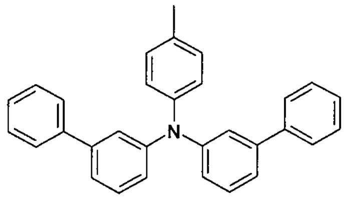

Formel (167)

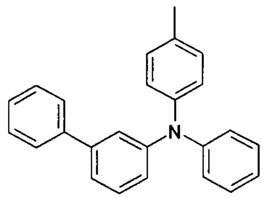

Formel (168)

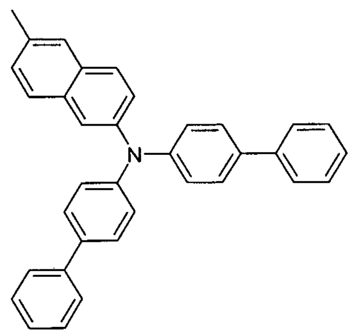
Formel (169)

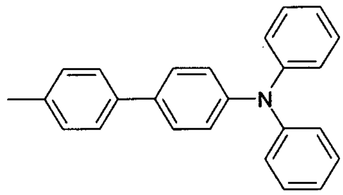
Formel (170)

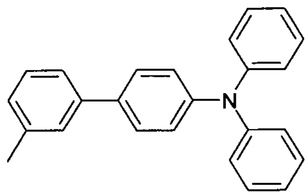
Formel (171)

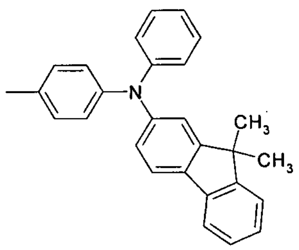
Formel (172)

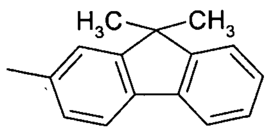
Formel (173)

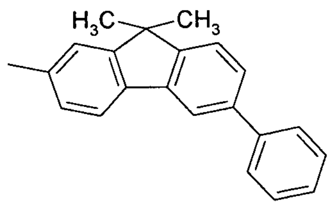
Formel (174)

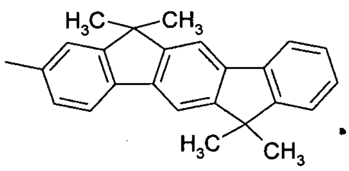
Formel (175)

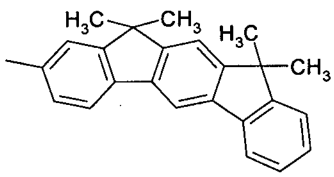
Formel (176)

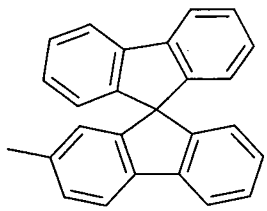
Formel (177)

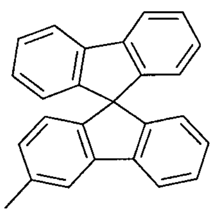
Formel (178)

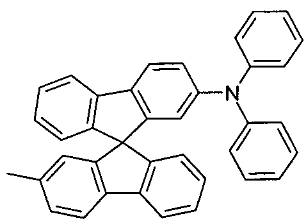
Formel (179)

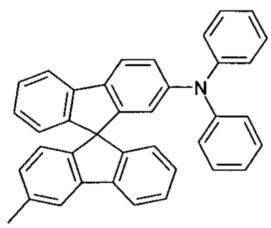
Formel (180)

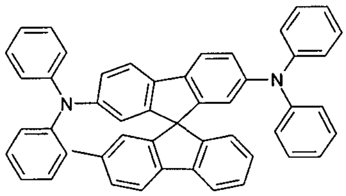

Formel (181)

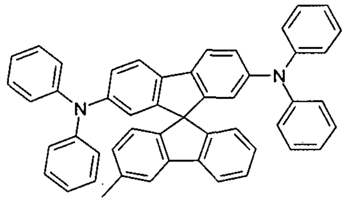

Formel (182)

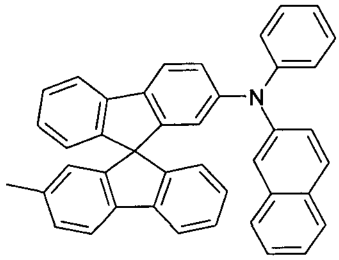

Formel (183)

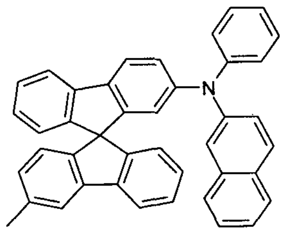

Formel (184)

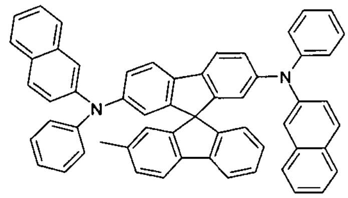

Formel (185)

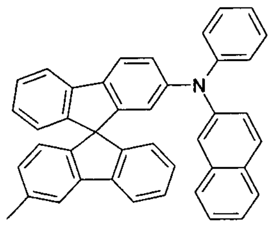

Formel (186)

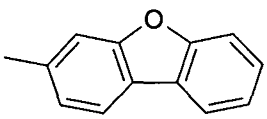

Formel (187)

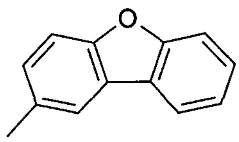

Formel (188)

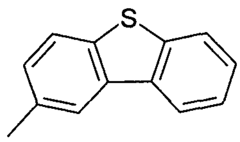

Formel (189)

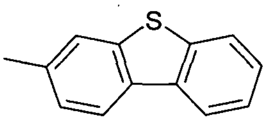

Formel (190)

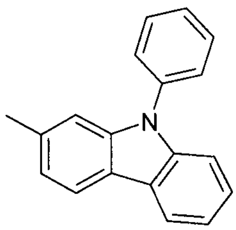

Formel (191)

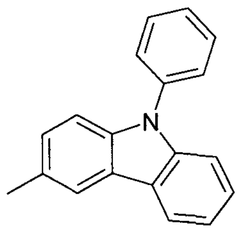

Formel (192)

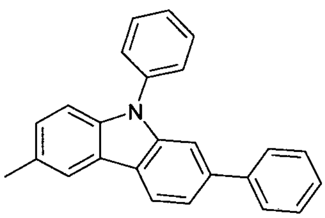

Formel (193)

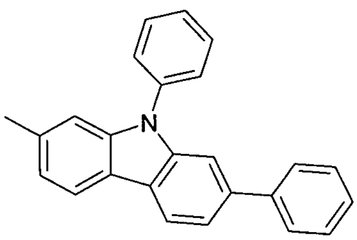

Formel (194)

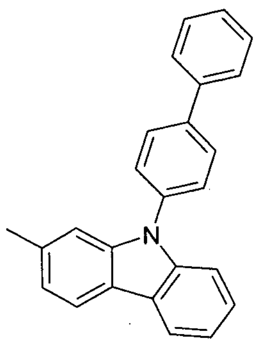

Formel (195)

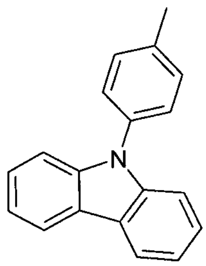

Formel (196)

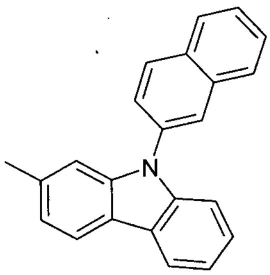

Formel (197)

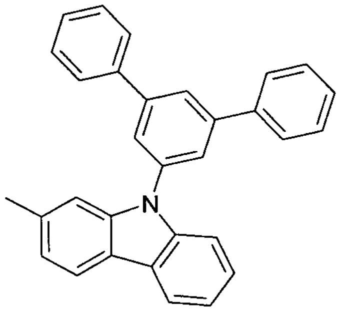

Formel (198)

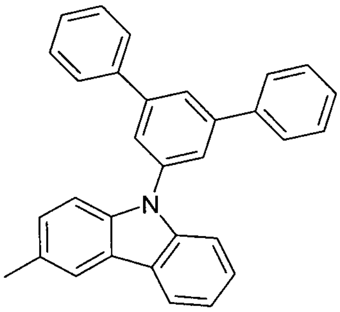

Formel (199)

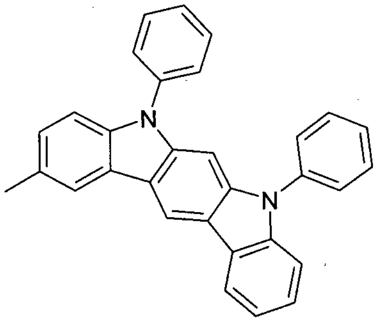

Formel (200)

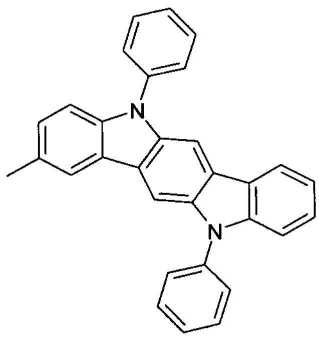

Formel (201)

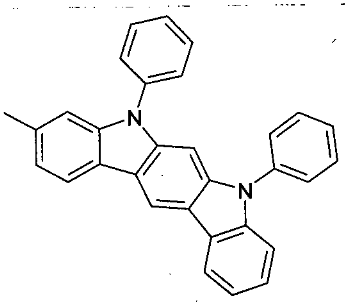

Formel (202)

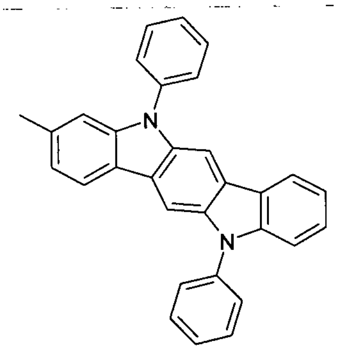

Formel (203)

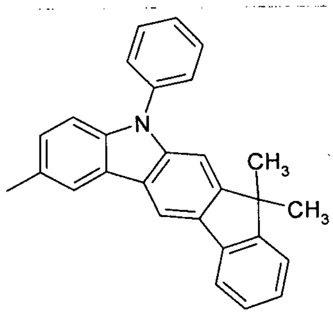

Formel (204)

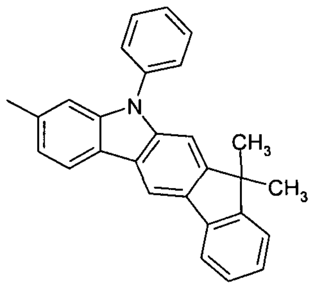

Formel (205)

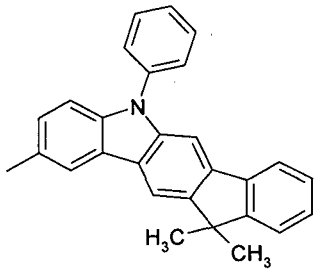

Formel (206)

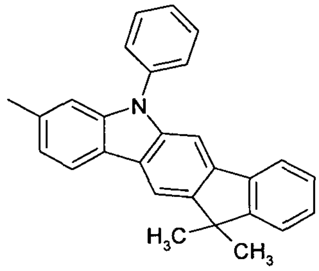

Formel (207)

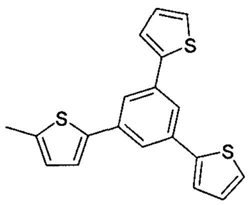

Formel (208)

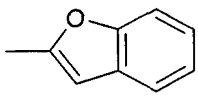

Formel (209)

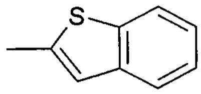

Formel (210)

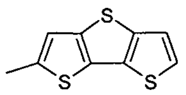

Formel (211)

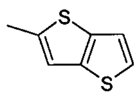

Formel (212)

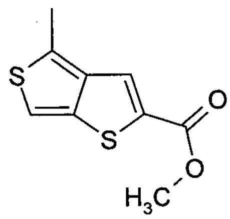

Formel (213)

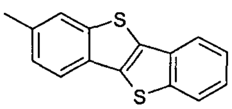

Formel (214)

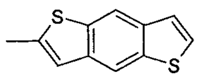

Formel (215)

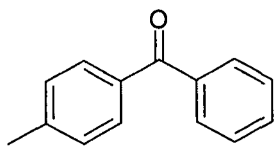

Formel (216)

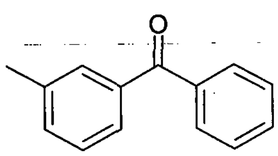

Formel (217)

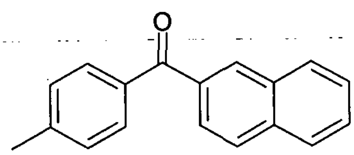

Formel (218)

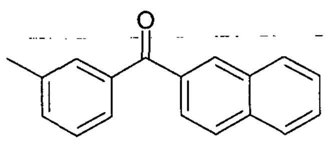

Formel (219)

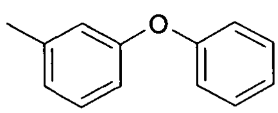

Formel (220)

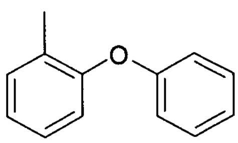

Formel (221)

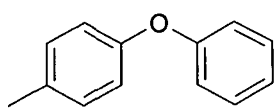

Formel (222)

Es ist weiterhin bevorzugt im Sinne der vorliegenden Erfindung, dass die Sensibilisatoren der allgemeinen Formel (1) symmetrisch aufgebaut sind, d.h. dass $R^1$ gleich $Ar^1$ ist, mit der Maßgabe, dass nun beide $Ar^1$ identisch sind und jeweils wenigstens 9 Ringatome enthalten.

Im Folgenden sind, ohne limitierend zu sein, einige bevorzugte Verbindungen offenbart, die als Sensibilisatoren in Zusammensetzung zur TTA-UpC im Sinne der vorliegenden Erfindung verwendet werden können.

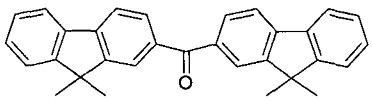

Formel (223)

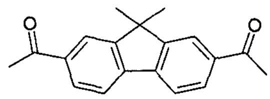

Formel (224)

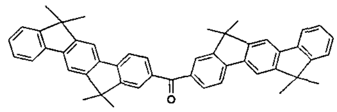

Formel (225)

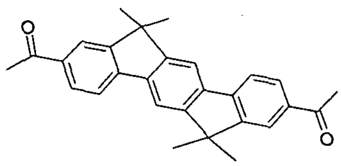

Formel (226)

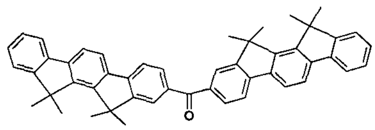

Formel (227)

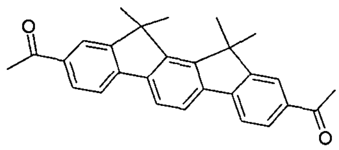

Formel (228)

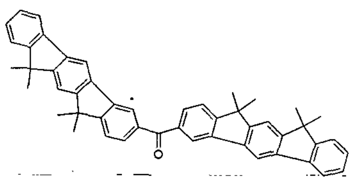

Formel (229)

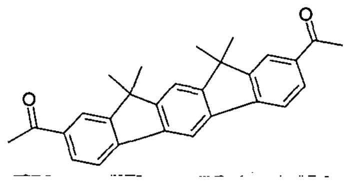

Formel (230)

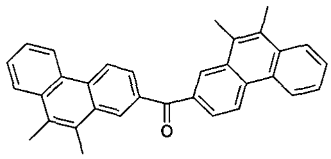

Formel (231)

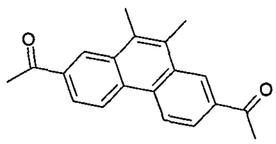

Formel (232)

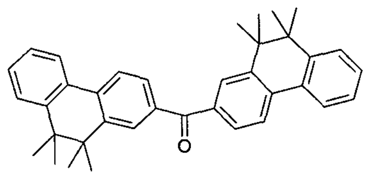

Formel (233)

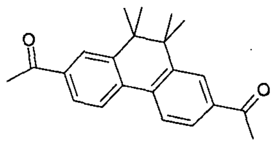

Formel (234)

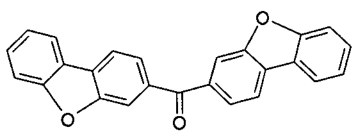

Formel (235)

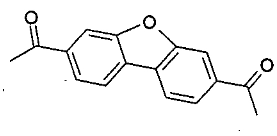

Formel 236)

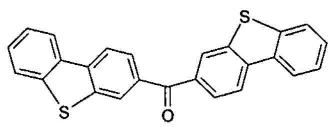

Formel (237)

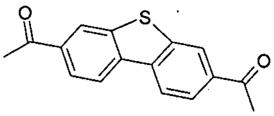

Formel (238)

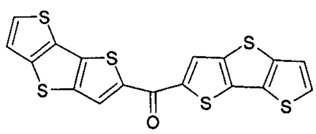

Formel (239)

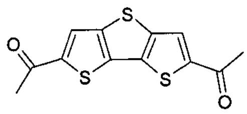

Formel (240)

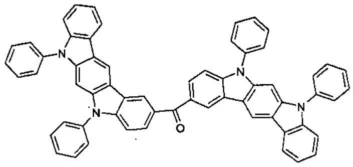

Formel (241)

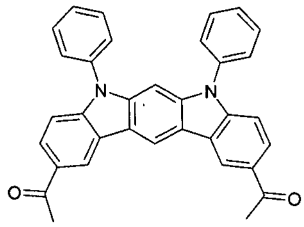

Formel (242)

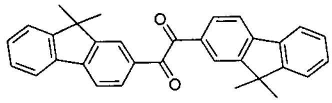

Formel (243)

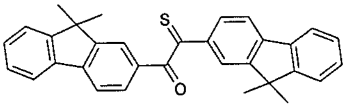

Formel (244)

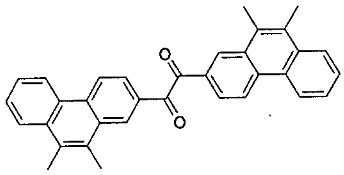

Formel (245)

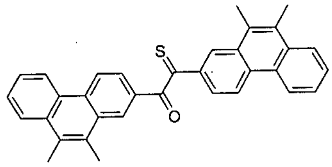

Formel (246)

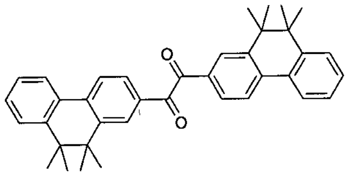

Formel (247)

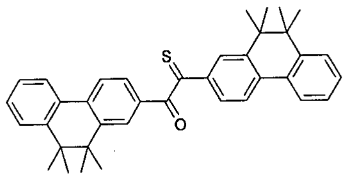

Formel (248)

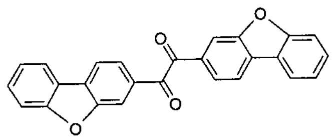

Formel (249)

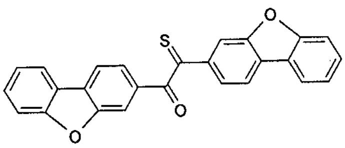

Formel (250)

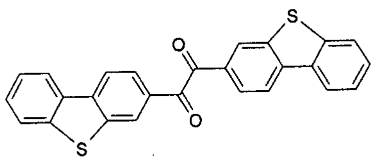

Formel (251)

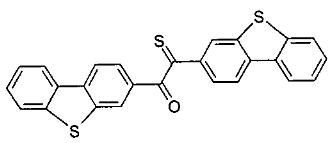

Formel (252)

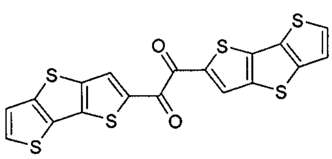

Formel (253)

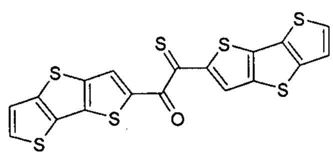

Formel (254)

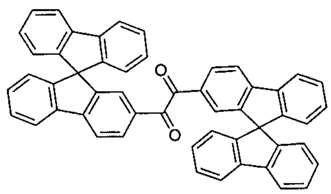

Formel (255)

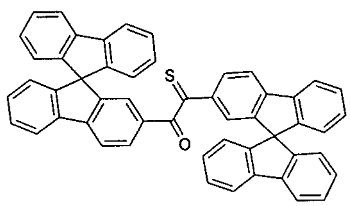
Formel (256)

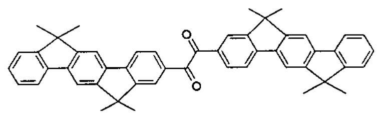
Formel (257)

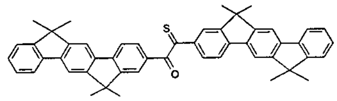
Formel (258)

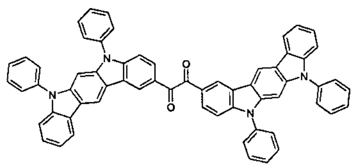
Formel (259)

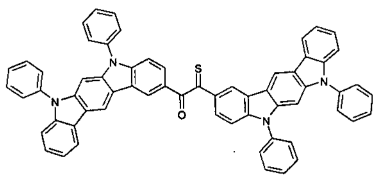
Formel (260)

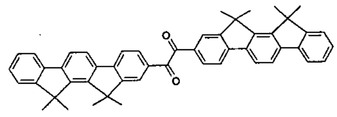
Formel (261)

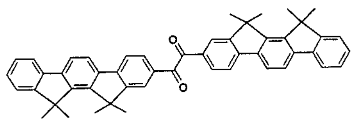
Formel (262)

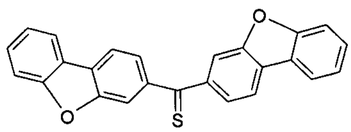
Formel (263)

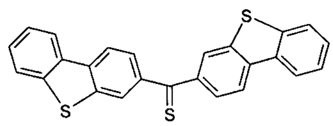
Formel (264)

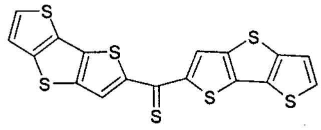
Formel (265)

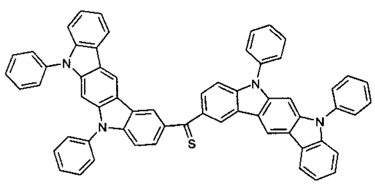
Formel (266)

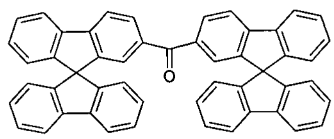
Formel (267)

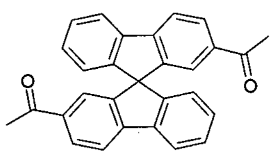
Formel (268)

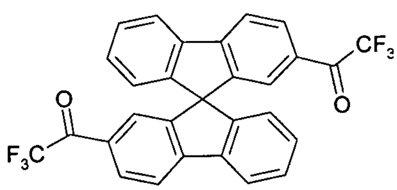
Formel (269)

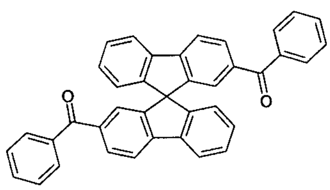
Formel (270)

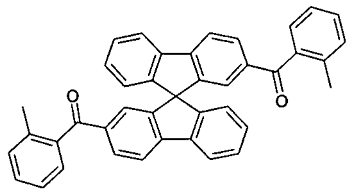

Formel (271)

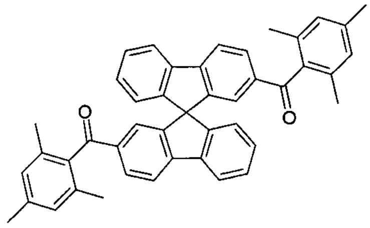

Formel (272)

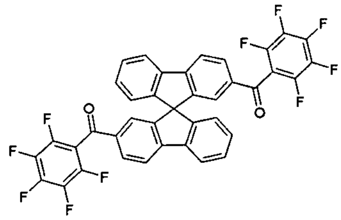

Formel (273)

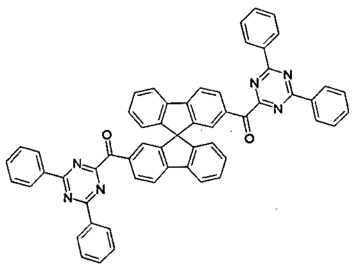

Formel (274

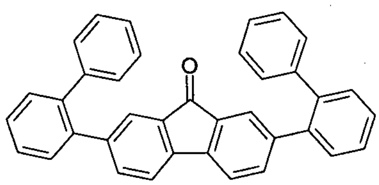

Formel (275)

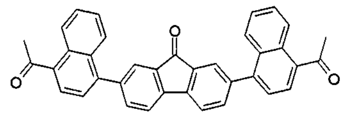

Formel (276)

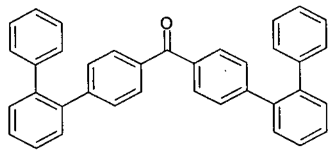

Formel (277)

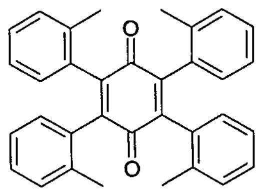

Formel (278)

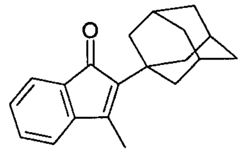

Formel (279)

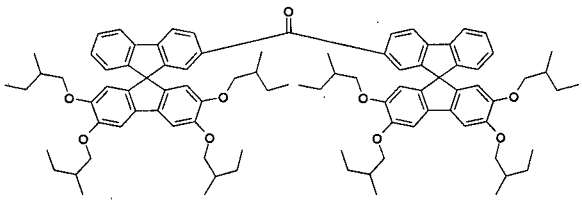

Formel (280)

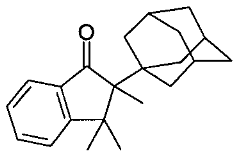

Formel (281)

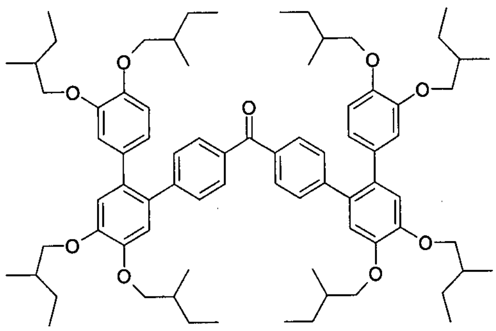

Formel (282)

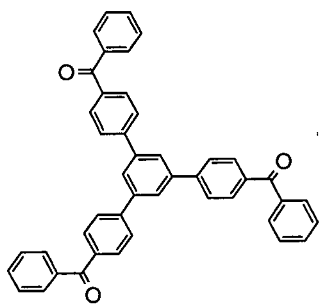

Formel (283)

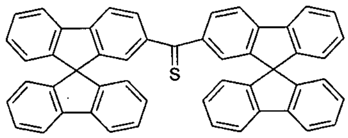

Formel (284)

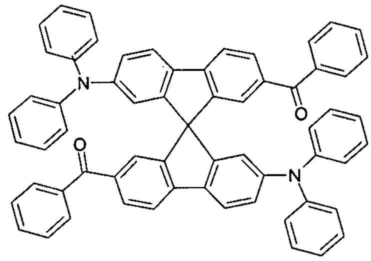

Formel (285)

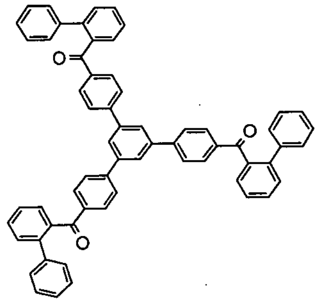

Formel (286)

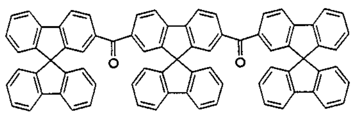

Formel (287)

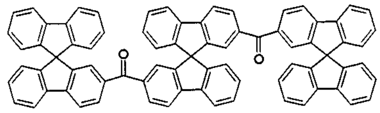

Formel (288)

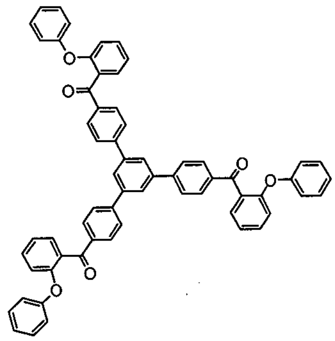

Formel (289)

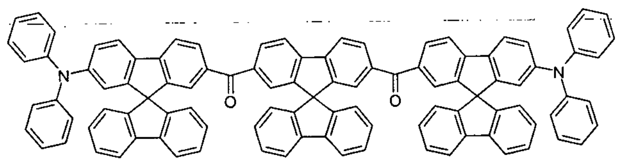

Formel (290)

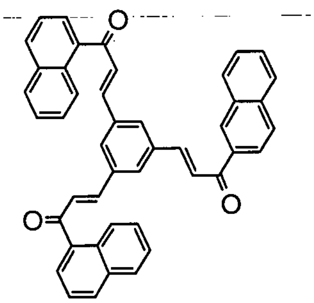

Formel (291)

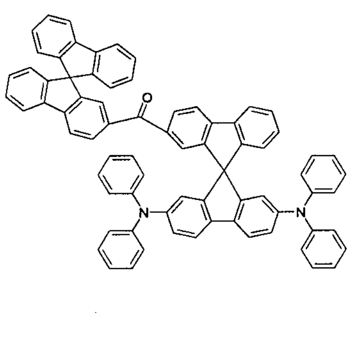

Formel (292)

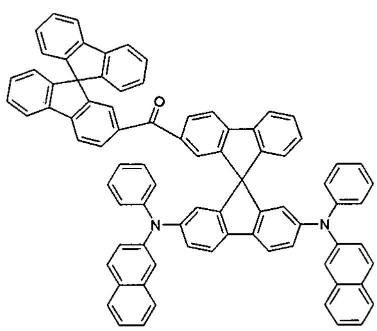

Formel (293)

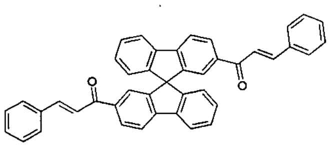

Formel (294)

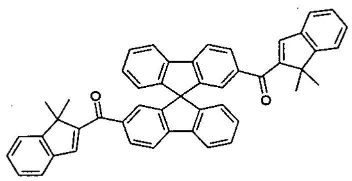 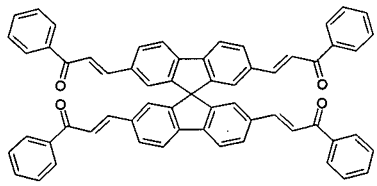 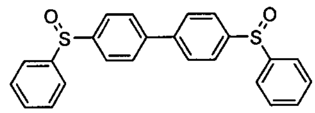

Formel (295) Formel (296) Formel (297)

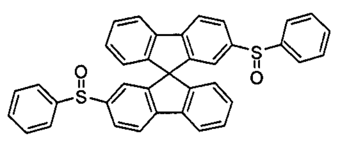 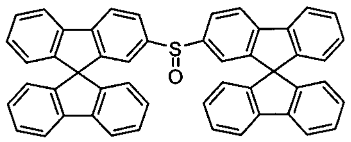 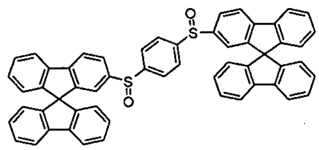

Formel (298) Formel (299) Formel (300)

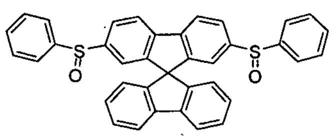 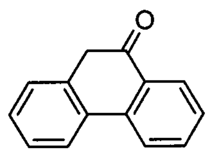 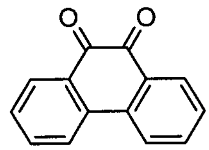

Formel (301) Formel (302) Formel (303)

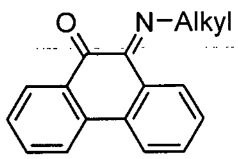 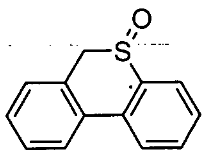

Formel (304) Formel (305)

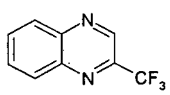 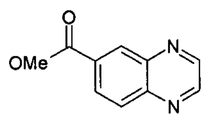

Die erfinndungsgemäßen Zusammensetzungen enthalten neben dem wenigstens einen Sensibilisator wenigstens einen fluoreszenten Emitter, der kein Metallkomplex ist.

Bevorzugt ist eine erfindungsgemäße Zusammensetzung enthaltend 3, ganz bevorzugt 2 und ganz besonders bevorzugt einen Sensibilisator.

Weiterhin bevorzugt sind erfindungsgemäße Zusammensetzungen enthaltend 3, ganz bevorzugt 2 und ganz besonders bevorzugt einen fluoreszierenden Emitter.

Ganz bevorzugt sind erfindungsgemäße Zusammensetzungen enthaltend 2 Sensibilisatoren und 3, bevorzugt 2 und ganz bevorzugt einen fluoreszierenden Emitter.

Ganz besonders bevorzugt sind erfindungsgemäße Zusammensetzungen enthaltend einen Sensibilisator und zwei fluoreszierende Emitter.

Insbesondere bevorzugt sind erfindungsgemäße Zusammensetzungen enthaltend einen Sensibilisator und einen fluoreszierenden Emitter.

Der Gewichtsanteil des Sensibilisators an der erfindungsgemäßen Zusammensetzung beträgt 1.0 Gew.-% bis 97 Gew.-%, bevorzugt 5 Gew.-% bis 95 Gew.-%, ganz bevorzugt 10 Gew.-% bis 93 Gew.-%, und ganz besonders bevorzugt 20 Gew.-% bis 93 Gew.-%.

Fluoreszierende Emitter, die in den erfindungsgemäßen Zusammensetzungen und Vorrichtungen zur TTA-UpC eingesetzt werden können, sind wie folgt beschrieben.

In einer bevorzugten Ausführungsform ist der Emitter ein blauer oder UV Emitter.

Im Kontext der vorliegenden Erfindung haben die Begriffe Singulett Emitter, Singulett Dotanden, fluoreszierende Emitter und fluoreszierende Dotanden dieselbe Bedeutung.

Geeignete Dotanden sind ausgewählt aus der Klasse der Monostyrylamine, der Distyrylamine, der Tristyrylamine, der Tetrastyrylamine, der Styrylphosphine, der Styrylether und der Arylamine. Unter einem Monostyrylamin wird eine Verbindung verstanden, die eine substituierte oder unsubstituierte Styrylgruppe und mindestens ein, bevorzugt aromatisches, Amin enthält. Unter einem Distyrylamin wird eine Verbindung verstanden, die zwei substituierte oder unsubstituierte Styrylgruppen und mindestens ein, bevorzugt aromatisches, Amin enthält. Unter einem Tristyrylamin wird eine Verbindung verstanden, die drei substituierte oder unsubstituierte Styrylgruppen und mindestens ein, bevorzugt aromatisches, Amin enthält. Unter einem Tetrastyrylamin wird eine Verbindung verstanden, die vier substituierte oder unsubstituierte Styrylgruppen und mindestens ein, bevorzugt aromatisches, Amin enthält. Die Styrylgruppen sind besonders bevorzugt Stilbene, die auch noch weiter substituiert sein können. Entprechende Phosphine und Ether sind in Analogie zu den Aminen definiert. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 2- oder in 9-Position. Unter einem aromatischen Anthracendiamin wird eine Verbindung verstanden, in der zwei Diarylaminogruppen direkt an eine Anthracengruppe gebunden sind, vorzugsweise in 2,6- oder in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte Dotanden sind gewählt aus Indenofluorenaminen bzw. -diaminen, beispielsweise gemäß WO 2006/122630, Benzoindenofluorenaminen bzw. -diaminen, beispielsweise gemäß WO 2008/006449, und Dibenzoindenofluorenaminen bzw. -diaminen, beispielsweise gemäß WO 2007/140847. Beispiele für Dotanden aus der Klasse der Styrylamine sind substituierte oder unsubstituierte Tristilbenamine oder die Dotanden, die in den Patentanmeldungen WO 2006/000388, WO 2006/058737, WO 2006/000389, WO 2007/065549 und WO 2007/115610 beschrieben sind. Weiterhin bevorzugt sind überbrückte aromatische Kohlenwasserstoffe, wie z. B. die in WO 2010/012328 offenbarten Verbindungen.

Bevorzugte fluoreszierende Dotanden sind die Verbindungen der folgenden Formeln (338) und (339)

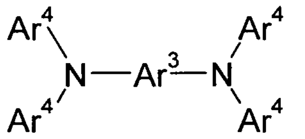

Formel (338)

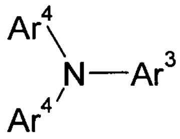

Formel (339)

wobei für die verwendeten Symbole gilt:

- $Ar^3$ ist eine kondensierte Aryl- bzw. Heteroarylgruppe bzw. ein kondensiertes aromatisches oder heteroaromatisches Ringsystem mit 10 bis 40 aromatischen Ringatomen, welches durch einen oder mehrere Reste $R^2$ substituiert sein kann;

- $Ar^4$ ist bei jedem Auftreten gleich oder verschieden ein aromatisches oder heteroaromatisches Ringsystem mit 5 bis 30 aromatischen Ringatomen, welches durch einen oder mehrere Reste $R^4$ substituiert sein kann; dabei können auch zwei Reste $Ar^4$, welche an dasselbe Stickstoffatom binden, durch eine Einfachbindung oder eine Brücke, ausgewählt aus $B(R^4)$, $C(R^4)_2$, $Si(R^4)_2$, C=O, $C=NR^4$, $C=C(R^4)_2$, O, S, S=O, $SO_2$, $N(R^4)$, $P(R^4)$ und $P(=O)R^4$, miteinander verknüpft sein;

- $R^4$ ist bei jedem Auftreten gleich oder verschieden H, D, F, Cl, Br, I, CHO, $N(R^5)_2$, $C(=O)R^5$, $P(=O)(R^5)_2$, $S(=O)R^5$, $S(=O)_2R^5$, $CR^5=C(R^5)_2$, CN, $NO_2$, $Si(R^5)_3$, $B(OR^5)_2$, $B(R^5)_2$, $B(N(R^5)_2)_2$, $OSO_2R^5$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder eine Alkenyl- oder Alkinylgruppe mit 2 bis 40 C-Atomen, wobei die Alkyl-, Alkoxy-, Thioalkoxy-, Alkenyl- oder Alkinylgruppe jeweils mit einem oder mehreren Resten $R^5$ substituiert sein

kann, wobei eine oder mehrere nicht benachbarte $CH_2$-Gruppen durch $R^5C=CR^5$, C=C , $Si(R^5)_2$, C=O, C=S, $C=NR^5$, $P(=O)(R^5)$, SO, $SO_2$, $NR^5$, O, S oder $CONR^5$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 30 aromatischen Ringatomen, das jeweils durch einen oder mehrere Reste $R^5$ substituiert sein kann, oder eine Aryloxy- oder Heteroaryloxygruppe mit 5 bis 30 aromatischen Ringatomen, die durch einen oder mehrere Reste $R^5$ substituiert sein kann; dabei können zwei oder mehrere benachbarte Substituenten $R^5$ auch miteinander ein mono- oder polycyclisches, aliphatisches oder aromatisches Ringsystem bilden;

$R^5$ ist bei jedem Auftreten gleich oder verschieden H, D oder ein aliphatischer, aromatischer und/oder heteroaromatischer Kohlenwasserstoffrest mit 1 bis 20 C-Atomen, in dem auch H-Atome durch D, CN oder F ersetzt sein können; dabei können zwei oder mehrere benachbarte Substituenten $R^5$ auch miteinander ein mono- oder polycyclisches, aliphatisches oder aromatisches Ringsystem bilden.

In einer bevorzugten Ausführungsform der Erfindung ist $Ar^3$ eine kondensierte Arylgruppe bzw. ein kondensiertes aromatisches Ringsystem. Bevorzugte kondensierte Arylgruppen bzw. aromatische Ringsysteme $Ar^3$ sind ausgewählt aus der Gruppe bestehend aus Anthracen, Pyren, Fluoranthen, Naphthacen, Chrysen, Benzanthracen, Benzofluoren, Triphenylen, Perylen, cis- oder trans-Monobenzoindenofluoren und cis- oder trans-Dibenzoindenofluoren, die jeweils durch einen oder mehrere Reste $R^4$ substituiert sein können.

In einer bevorzugten Ausführungsform der Erfindung ist $Ar^4$ ein aromatisches Ringsystem. Bevorzugte aromatische Ringsysteme $Ar^4$ sind gleich oder verschieden bei jedem Auftreten ausgewählt aus der Gruppe bestehend aus Phenyl, 1- oder 2-Naphthyl, ortho-, meta- oder para-Biphenyl, 2-Fluorenyl oder 2-Spirobifluorenyl, die jeweils durch einen oder mehrere Reste $R^4$ substituiert sein können.

Bevorzugte Reste $R^4$ sind gleich oder verschieden bei jedem Auftreten ausgewählt aus der Gruppe bestehend aus H, D, F, CN, geradkettigen Alkylgruppen mit 1 bis 10 C-Atomen oder verzweigten Alkylgruppen mit 3 bis 10 C-Atomen.

Weitere bevorzugte fluoreszierende Dotanden sind die Verbindungen der folgenden Formel (340),

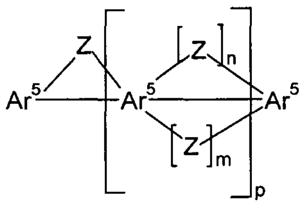

Formel (340)

wobei $R^4$ die oben genannte Bedeutung aufweist und für die weiteren verwendeten Symbole und Indizes gilt:

$Ar^5$ ist bei jedem Auftreten gleich oder verschieden eine Aryl- oder Heteroarylgruppe mit 5 bis 30 aromatischen Ringatomen, die mit einem oder mehreren Resten $R^2$ substituiert sein kann, mit der Maßgabe, dass mindestens eine Gruppe $Ar^5$ für eine kondensierte Aryl- oder Heteroarylgruppe mit 10 bis 30 aromatischen Ringatomen steht;

Z ist bei jedem Auftreten gleich oder verschieden ausgewählt aus der Gruppe bestehend aus $BR^4$, $C(R^4)_2$, $Si(R^4)_2$, C=O, $C=NR^4$, $C=C(R^4)_2$, O, S, S=O, $SO_2$, $NR^4$, PR4 und $P(=O)R^4$;

m, n ist 0 oder 1, mit der Maßgabe, dass m + n = 1 ist;

p ist 1, 2 oder 3;

dabei bilden jeweils zwei Gruppen $Ar^5$ und Z zusammen einen Fünfring oder einen Sechsring, bevorzugt jeweils einen Fünfring.

In einer bevorzugten Ausführungsform der Erfindung beträgt die Summe aller $\pi$-Elektronen der Gruppen $Ar^5$ mindestens 28, wenn p = 1 ist, und beträgt mindestens 34, wenn p = 2 ist, und beträgt mindestens 40, wenn p = 3 ist.

In einer bevorzugten Ausführungsform der Erfindung steht mindestens eine Gruppe $Ar^5$ für eine kondensierte Arylgruppe mit 10 bis 18 C-Atomen, insbesondere ausgewählt aus der Gruppe bestehend aus Naphthalin, Phenanthren, Anthracen, Pyren, Fluoranthen, Naphthacen, Chrysen, Benzanthracen, Benzphenanthren und Triphenylen und die anderen beiden Gruppen $Ar^5$ stehen gleich oder verschieden bei jedem Auftreten für eine Arylgruppe mit 6 mit 18 C-Atomen, bevorzugt gleich oder verschieden bei jedem Auftreten für Phenyl oder Naphthyl.

In einer weiteren bevorzugten Ausführungsform der Erfindung ist Z gleich oder verschieden bei jedem Auftreten ausgewählt aus der Gruppe bestehend aus $C(R^4)_2$, C=O, $NR^4$, O und S, besonders bevorzugt gleich oder verschieden bei jedem Auftreten $C(R^4)_2$ oder $NR^4$, ganz besonders bevorzugt $C(R^4)_2$.

Geeignete fluoreszierende Dotanden sind weiterhin die im Folgenden abgebildeten Strukturen, sowie die in JP 06/001973, WO 2004/047499, WO 2006/098080, WO 2007/065678, US 2005/0260442 und WO 2004/092111 offenbarten Strukturen.

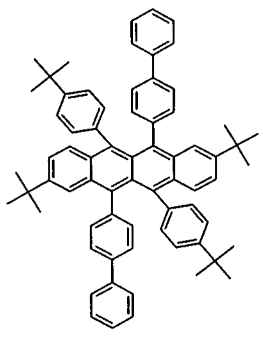

Formel (341)

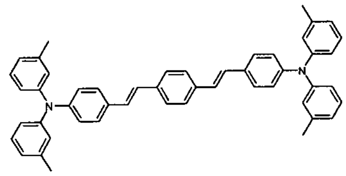

Formel (342)

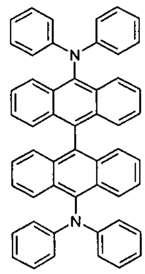

Formel (343)

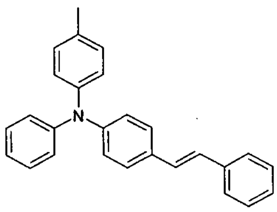

Formel (344)

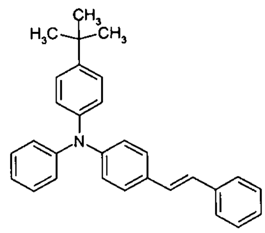

Formel (345)

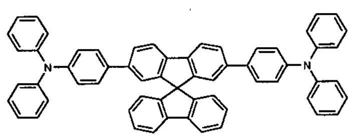

Formel (346)

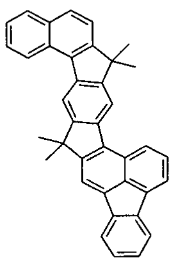

Formel (347)

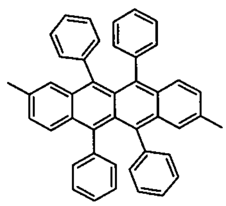

Formel (348)

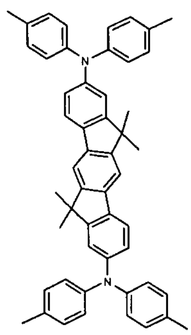

Formel (349)

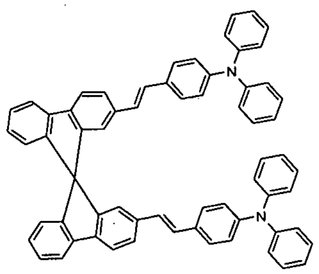

Formel (350)

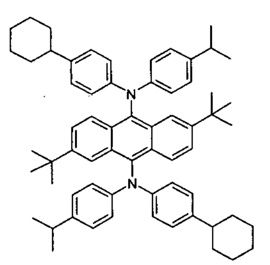

Formel (351)

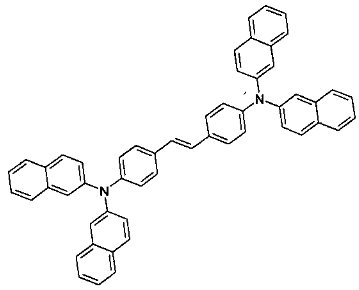

Formel (352)

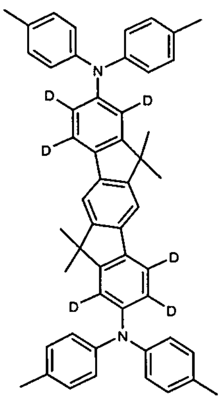

Formel (353)

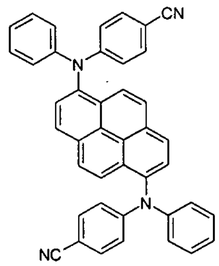

Formel (354)

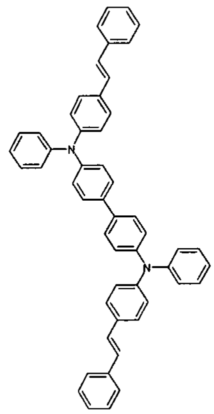

Formel (355)

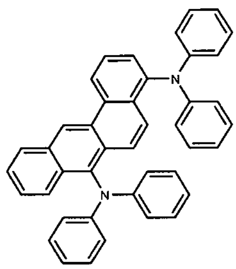

Formel (356)

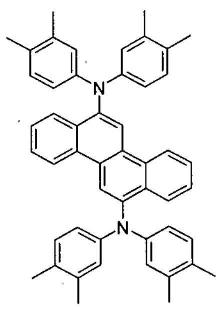

Formel (357)

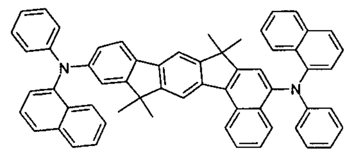

Formel (358)

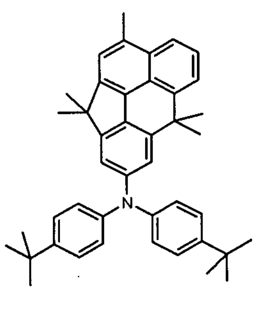

Formel (359)

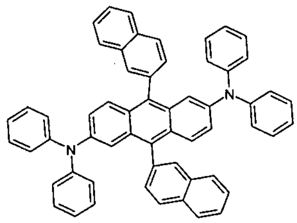

Formel (360)

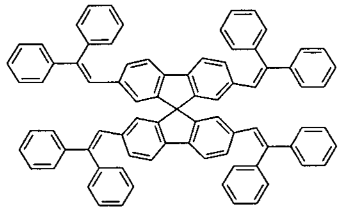

Formel (361)

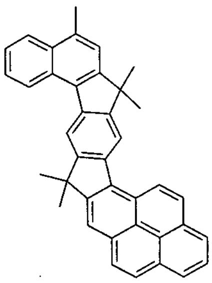

Formel (362)

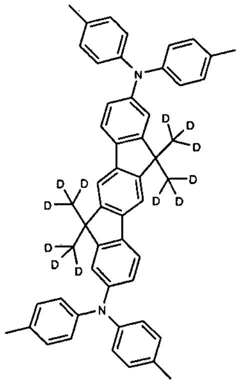

Formel (363)

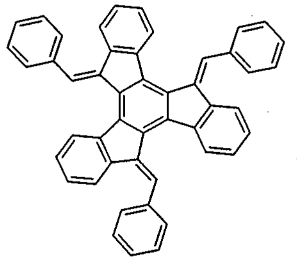

Formel (364)

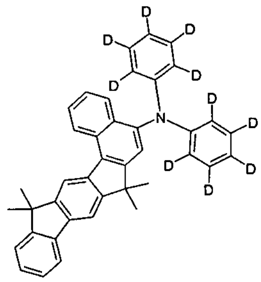

Formel (365)

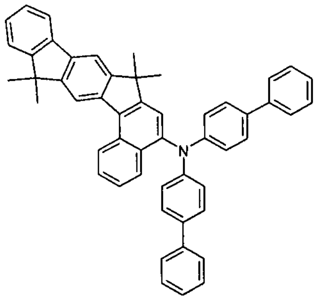

Formel (366)

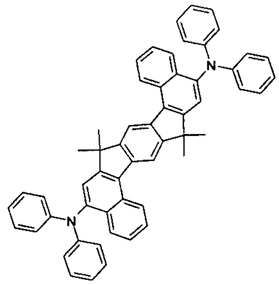

Formel (367)

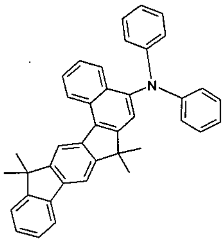

Formel (368)

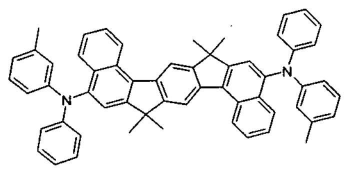

Formel (369)

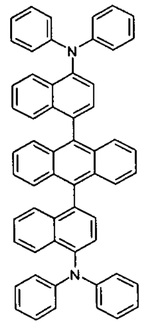

Formel (370)

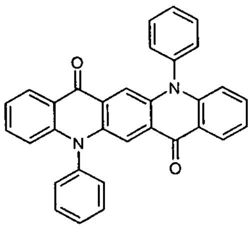

Formel (371)

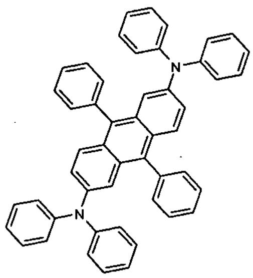

Formel (372)

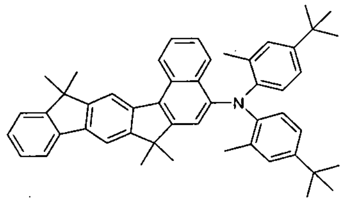

Formel (373)

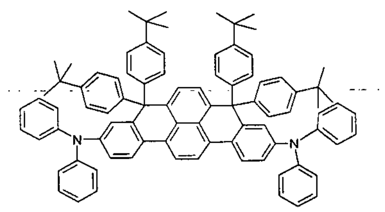

Formel (374)

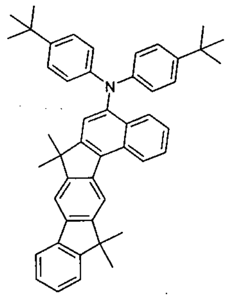

Formel (375)

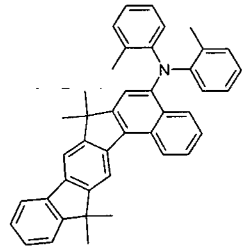

Formel (376)

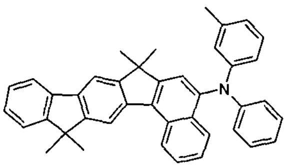

Formel (377)

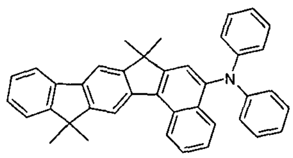

Formel (378)

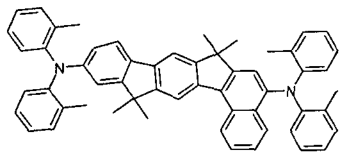

Formel (379)

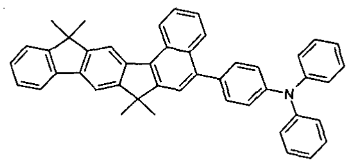

Formel (380)

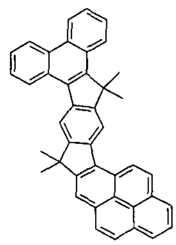

Formel (381)

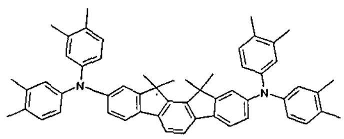

Formel (382)

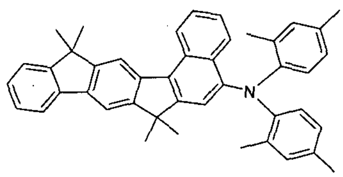

Formel (383)

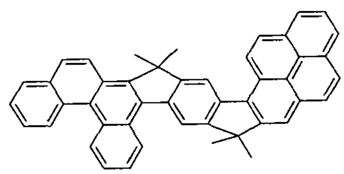

Formel (384)

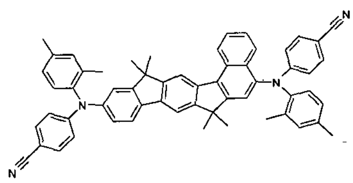

Formel (385)

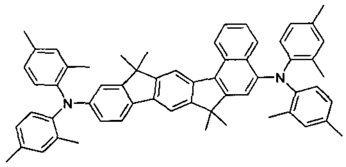

Formel (386)

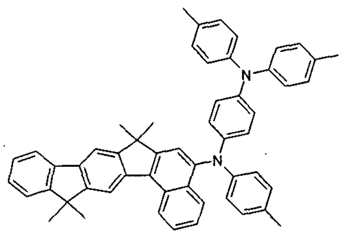

Formel (387)

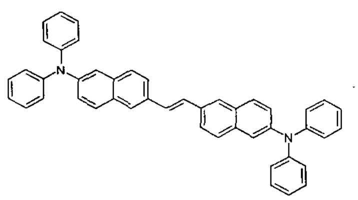

Formel (388)

Die erfindungsgemäßen Zusammensetzungen sind dadurch charakterisiert, dass das Triplett Niveau des Sen-

sibilisators $T_1$(S) größer ist als das Triplett Niveau des Emitters $T_1$(E).

In einer Ausführungsform sind die erfindungsgemäßen Zusammensetzungen dadurch gekennzeichnet, dass das Singulett Niveau des Emitters $S_1$(E) höher ist als das Singulett Niveau des Sensibilisators $S_1$(S) (Abbildung 1).

In einer weiteren bevorzugten Ausführungsform sind die erfindungsgemäßen Zusammensetzungen dadurch gekennzeichnet, dass das Singulett Niveau des Emitters $S_1$(E) niederiger ist als das Singulett Niveau des Sensibilisators $S_1$(S) (Abbildung 2).

Die ISC Rate des Sensibilisators soll dabei höher sein als die Emissionsrate des Sensibilisators aus $S_1$(S). Die ISC Rate einer organischen Verbindung kann mittels "Zeeman phosphorescence microwave double resonance (PMDR) Spectroscopy bestimmt werden, wie Zinsli et.al. in Chem. Phys. Lett. Vol 34, 403(1975) beschrieben haben-Dort wurde auch die ISC Rate von Chinoxalin besimmt.Die sehr hohe ISC Rate von Naphthyridin, Phthalazin und Chinoxalin wurde bereits von Boldridge et al. (J. Phys. Chem. 86, 1976, 1982) und von Komorowski et al. (J. Photochem. 30, 141, 1985) belegt.

Die erfindungsgemäßen Zusammensetzungen sind dadurch charakterisiert, dass die Quatanausbeute der Phosphoreszenz des Sensibilisators bei 20°C oder höheren Temperaturen sehr gering ist, bevorzugt nicht mehr als 2%, ganz bevorzugt nicht mehr als 1%, ganz besonder bevorzugt nicht mehr als 0.2%. Insbesondere bevorzugt zeigt der Sensibilisator bei 20°C weder Fluoreszenz noch Phosphoreszenz.

Wie oben bereits ausgeführt eignen sich die erfindungsgemäßen Zusammensetzungen für UpC. Daher ist ein weiterer Gegenstand der vorliegenden Erfindung die Verwendung der erfindungsgemäßen Zusammensetzung enthaltend wenigstens eine Verbindung der allgemeinen Formel (1) und wenigstens einen fluoreszierenden Emitter für UpC, insbesondere für UpC in elektrolumineszierenden Vorrichtungen.

Die erfindungsgemäßen Zusammensetzungen werden dabei in der Emissionsschicht eingesetzt. Die vorliegendende Erfindung betrifft daher auch eine Emissionsschicht enthaltend die erfindungsgemäßen Zusammensetzungen.

Die vorliegende technische Lehre kann weiter verallgemeinert werden auf alle Up-Conversion Systeme oder Zusammensetzungen, die für den Zweck des Up-Conversion eingesetzt werden können, um elektrolumineszierende Vorrichtungen zu entwickeln, die Licht im blauen Bereich des Spektrums oder UV-Strahlung emittieren.

Beispielhaft ist die Verwendung einer Zusammensetzung für Up-Conversion in elektrolumineszierenden Vorrichtungen zur Erzeugung von Licht oder Strahlung im UV-Bereich.

Bevorzugt handelt es sich bei den Vorrichtungen um organische Elektrolumineszenzvorrichtungen.

Vorliegend soll unter blauem Licht bevorzugt Licht mit einer Wellenlänge im Bereich von 380 und 490 nm verstanden werden.

UV-Strahlung im Sinne der vorliegenden Erfindung ist bevorzugt Strahlung mit einer Wellenlänge im Bereich von 200 und 380 nm. Insbesondere bevorzugt ist die Emission von UV-A Strahlung (315 bis 380 nm) und/oder von UV-B Strahlung (280 bis 315 nm).

Ein weiterer Gegenstand der vorliegenden Erfindung betrifft optische und/oder elektronische Vorrichtungen zur Up-Conversion enthaltend mindestens eine erfindungsgsmäße Zusammensetzung.

(Die Vorrichtungen können dabei ausgewählt werden aus der Gruppe Die Vorrichtungen können dabei ausgewählt werden aus der Gruppe bestehend aus organischen Elektrolumineszenzvorrichtungen, wie zum Beispiel organische lichtemittierende Dioden (OLED), organische lichtemittierende Transistoren, organische lichtemittierende elektrochemische Zellen, organische lichtemittierende elektrochemische Transistoren, oder einem organischen Laser, wobei eine OLED besonders bevorzugt ist.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Ein möglicher Schichtaufbau ist bspw. der folgende: Kathode/ EML/Zwischeschicht/Pufferschicht/Anode, wobei EML die emittierende Schicht repräsentiert. Dabei kann die organische Elektrolumineszenzvorrichtung eine emittierende Schicht enthalten, oder sie kann mehrere emittierende Schichten enthalten.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner $10^{-5}$ mbar, bevorzugt kleiner $10^{-6}$ mbar aufgedampft. Es ist aber auch möglich, dass der Anfangsdruck noch geringer ist, beispielsweise kleiner $10^{-7}$ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden. Diese Verfahren eignen sich insbesondere auch für Oligomere, Dendrimere und Polymere.

Eine weitere Ausführungsform der vorliegenden Erfindung bezieht sich auf Formulierungen enthaltend eine oder mehrere der erfindungsgemäßen Zusammensetzungen sowie ein oder mehrere Lösungsmittel. Die Formulierung eignet sich hervorragend zum Erzeugen von Schichten aus Lösung.

Geeignete und bevorzugte Lösungsmittel sind beispielsweise Toluol, Anisol, Xylole, Methylbenzoat, Dimethylanisole, Trimethylbenzole, Tetralin, Veratrole, Tetrahydrofuran, Cyclohexanon, Chlorbenzol oder Dichlorbenzole sowie Gemische derselben.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäße organische Elektrolumineszenzvorrichtung kann beispielsweise in Displays oder für Beleuchtungszwecke verwendet werden, aber auch für medizinische oder kosmetische Anwendungen.

Die erfindungsgemäßen Zusammensetzungen eignen sich zum Einsatz in lichtemittierenden Vorrichtungen. Somit sind diese Verbindungen sehr vielseitig einsetzbar. Einige der Hauptanwendungsgebiete sind dabei Display- oder Beleuchtungs-Technologien. Weiterhin ist es besonders vorteilhaft, die Zusammensetzungen sowie Vorrichtungen enthaltend diese Verbindungen im Bereich der Phototherapie einzusetzen.

Ein weiterer Gegenstand der vorliegenden Erfindung bezieht sich daher auf die Verwendung der erfindungsgemäßen Zusammensetzungen und Vorrichtungen enthaltend die Zusammensetzungen zur Behandlung, Prophylaxe und Diagnose von Erkrankungen. Noch ein weiterer Gegenstand der vorliegenden Erfindung bezieht sich auf die Verwendung, der erfindungsgemäßen Zusammensetzungen und Vorrichtungen enthaltend die Zusammensetzungen in der Kosmetik.

Ein weiterer Gegenstand der vorliegenden Erfindung bezieht sich auf die erfindungsgemäßen Zusammensetzungen und Vorrichtungen enthaltend die Zusammensetzungen zur Herstellung von Geräten, d.h. von Bestrahlungsgeräten, zur Therapie, Prophylaxe und/oder Diagnose therapeutischer Erkrankungen.

Weiterhin betrifft die vorliegende Erfindung Vorrichtungen enthaltend die erfindungegemäßen Zusammensetzungen zur Verwendung zur Behandlung der Haut mit Phototherapie.

Noch ein Gegenstand der vorliegenden Erfindung betrifft die Verwendung der Vorrichtung enthaltend die erfindungegemäßen Zusammensetzungen in der Kosmetik.

Phototherapie oder Lichttherapie findet in vielen medizinischen und/oder kosmetischen Bereichen Anwendung. Die erfindungsgemäßen Zusammensetzungen und Vorrichtungen enthaltend die Zusammensetzungen können daher zur Therapie und/oder Prophylaxe und/oder Diagnose von allen Erkrankungen und/oder in kosmetischen Anwendungen eingesetzt werden, für die der Fachmann die Anwendung von Phototherapie in Betracht zieht. Der Begriff Phototherapie beinhaltet dabei neben der einfachen Bestrahlung auch die photodynamischen Therapie (PDT) sowie das Desinfizieren, Sterilisieren und Konservieren im Allgemeinen. Behandelt werden können mittels Phototherapie oder Lichttherapie nicht nur Menschen oder Tiere, sondern auch jegliche andere Art lebender oder unbelebter Materie. Hierzu gehören, bspw., Pilze, Bakterien, Mikroben, Viren, Eukaryonten, Prokaryonten, Nahrungsmittel, Getränke, Wasser und Trinkwasser. Auch können Behälter zum Frischhalten von Lebensmitteln oder anderen Gegenständen mit den erfindungsgemäßen Vorrichtungen versehen werden.

Der Begriff Phototherapie beinhaltet auch jede Art der Kombination von Lichttherapie und anderen Therapiearten, wie bspw. die Behandlung mit Wirkstoffen. Viele Lichttherapien haben zum Ziel, äußere Partien eines Objektes zu bestrahlen oder zu behandeln, so wie die Haut von Menschen und Tieren, Wunden, Schleimhäute, Auge, Haare, Nägel, das Nagelbett, Zahnfleisch und die Zunge. Die erfindungsgemäße Behandlung oder Bestrahlung kann daneben auch innerhalb eines Objektes durchgeführt werden, um bspw. innere Organe (Herz, Lunge etc.) oder Blutgefäße oder die Brust zu behandeln.

Die erfindungsgemäßen therapeutischen und/oder kosmetischen Anwendungsgebiete sind bevorzugt ausgewählt aus der Gruppe der Hauterkrankungen und Haut-assoziierten Erkrankungen oder Veränderungen bzw. Umstände wie bspw. Psoriasis, Hautalterung, Hautfaltenbildung, Hautverjüngung, vergrößerte Hautporen, Cellulite, ölige/fettige Haut, Follikulitis, aktinische Keratose, precancerose aktinische Keratose, Haut Läsionen, sonnengeschädigte und sonnengestresste Haut, Krähenfüße, Haut Ulkus, Akne, Akne rosacea, Narben durch Akne, Akne Bakterien, Photomodulierung fettiger/öliger Talgdrüsen sowie deren umgebende Gewebe, Ikterus, Neugeborenenikterus, Vitiligo, Hautkrebs, Hauttumore, Crigler Naijar, Dermatitis, atopische Dermatitis, diabetische Hautgeschwüre sowie Desensibilisierung der Haut.

Besonders bevorzugt im Sinne der Erfindung sind die Behandlung und/oder Prophylaxe von Psoriasis, Akne, Cellulite, Hautfaltenbildung, Hautalterung, Ikterus und Vitiligo.

Weitere erfindungsgemäße Anwendungsgebiete für die Zusammensetzungen und/oder Vorrichtungen enthaltend die erfindungsgemäßen Zusammensetzungen sind ausgewählt aus der Gruppe der Entzündungserkrankungen, rheumatoide Arthritis, Schmerztherapie, Behandlung von Wunden, neurologische Erkrankungen und Umstände, Ödeme, Paget's Erkrankung, primäre und metastasierende Tumoren, Bindegewebserkrankungen bzw. -Veränderungen, Veränderungen des Kollagens, Fibroblasten und von Fibroblasten stammende Zellspiegel in Geweben von Säugetieren, Bestrahlung der Retina, neovasculare und hypertrophe Erkrankungen, allergische Reaktionen, Bestrahlung der Atemwege, Schwitzen, okulare neovaskulare Erkrankungen, virale Infektionen besonders Infektionen durch Herpes Simplex oder HPV (Humane Papillomviren) zur Behandlung von Warzen und Genitalwarzen.

Besonders bevorzugt im Sinne der Erfindung sind die Behandlung und/oder Prophylaxe von rheumatoider Arthritis, viraler Infektionen, und Schmerzen.

Weitere erfindungsgemäße Anwendungsgebiete für die Zusammensetzungen und/oder Vorrichtungen enthaltend die erfindungsgemäßen Zusammensetzungen sind ausgewählt aus der Winterdepression, Schlafkrankheit, Bestrahlung zur Verbesserung der Stimmung, Linderung von Schmerzen besonders Muskelschmerzen durch bspw. Verspannungen oder Gelenkschmerzen, Beseitigung der Steifheit von Gelenken und das Aufhellen der Zähne (Bleaching).

Weitere erfindungsgemäße Anwendungsgebiete für die Zusammensetzungen und/oder Vorrichtungen enthaltend die erfindungsgemäßen Zusammensetzungen sind ausgewählt aus der Gruppe der Desinfektionen. Mit den erfindungsgemäßen Zusammen-setzungen und/oder Vorrichtungen enthaltend die erfindungsgemäßen Zusammensetzungen können jegliche Art von Objekten (unbelebte Materie) oder Subjekten (lebende Materie wie bspw. Mensch und Tier) zum Zweck der Desinfektion, Sterilisation oder Konservierung behandelt werden. Hierzu zählt, zum Beispiel, die Desinfektion von Wunden, die Reduktion von Bakterien, das Desinfizieren chirurgischer Instrumente oder anderer Gegenstände, das Desinfizieren oder Konservieren von Nahrungs- und Lebensmitteln, von Flüssigkeiten, insbesondere Wasser, Trinkwasser und andere Getränke, das Desinfizieren von Schleimhäuten und Zahnfleisch und Zähnen. Unter Desinfektion wird hierbei die Reduktion lebender mikrobiologischer Verursacher unerwünschter Effekte, wie Bakterien und Keime, verstanden.

Zu dem Zweck der oben genannten Phototherapie emittieren Vorrichtungen enthaltend die erfindungsgemäßen Verbindungen bevorzugt Licht der Wellenlänge zwischen 280 and 1000 nm, besonders bevorzugt zwischen 290 and 800 nm und insbesondere bevorzugt zwischen 380 and 600 nm.

Besonders vorteilhaft sind die Zusammen-setzungen und/oder Vorrichtungen enthaltend die erfindungsgemäßen Zusammensetzungen aufgrund der Tatsache, dass mittels UpC auch eine UV-Emission möglich ist. Dies ist für bestimmte Anwendungsgebiete wichtig und mittels Vorrichtungen aus dem Stand der technik noch nicht möglich. So wird, bspw., Psoriasis durch Bestrahlung mit Strahlung der Wellenlänge um 311 nm behandelt.

In einer besonders bevorzugten Ausführungsform der vorliegenden Erfindung werden die erfindungsgemäßen Zusammensetzungen in einer organischen lichtemittierenden Diode (OLED) oder einer organischen lichtemittierenden elektrochemischen Zelle (OLEC) zum Zwecke der Phototherapie eingesetzt. Sowohl die OLED als auch die OLEC können dabei einen planaren oder Fiber- bzw. Faser-artigen Aufbau mit beliebigem Querschnitt (z.B. rund, oval, polygonal, quadratisch) mit einem ein- oder mehrschichtigen Aufbau aufweisen. Diese OLECs und/oder OLEDs können in andere Vorrichtungen eingebaut werden, die weitere mechanische, adhäsive und/oder elektronische Bausteine (z.B. Batterie und/oder Steuerungseinheit zur Einstellung der Bestrahlungszeiten, -intensitäten und -wellenlängen) enthalten. Diese Vorrichtungen enthaltend die erfindungsgemäßen OLECs und/order OLEDs sind vorzugsweise ausgewählt aus der Gruppe enthaltend Pflaster, Pads, Tapes, Bandagen, Manschetten, Decken, Hauben, Schlafsäcken, Textilien und Stents.

Die Verwendung der genannten Vorrichtungen zum genannten therapeutischen und/oder kosmetischen Zweck ist besonders vorteilhaft gegenüber dem Stand der Technik, da mit Hilfe der erfindungsgemäßen Vorrichtungen unter Verwendung der OLEDs und/oder OLECs homogene Bestrahlungen geringer Bestrahlungsintensitäten an nahezu jedem Ort und zu jeder Tageszeit möglich sind. Die Bestrahlungen können stationär, ambulant und/oder selbst, d.h., ohne Einleitung durch medizinisches oder kosmetisches Fachpersonal durchgeführt werden. So können, bspw., Pflaster unter der Kleidung getragen werden, so dass eine Bestrahlung auch während der Arbeitszeit, in der Freizeit oder während des Schlafes möglich ist. Auf aufwendige stationäre/ambulante Behandlungen mit kann in vielen Fällen verzichtet bzw. deren Häufigkeit reduziert werden. Die erfindungsgemäßen Vorrichtungen können zum Wiedergebrauch gedacht sein oder Wegwerfartikel darstellen, die nach ein-, zwei oder dreimaligem Gebrauch entsorgt werden können.

Weitere Vorteile gegenüber dem Stand der Technik sind bspw. eine geringere Wärmeentwicklung und emotionale Aspekte. So werden Neugeborene, die aufgrund einer Gelbsucht (Ikterus) therapiert werden müssen, typischerweise mit verbundenen Augen in einem Brutkasten, ohne körperlichen Kontakt zur den Eltern bestrahlt, was eine emotionale Stresssituation für Eltern und Neugeborene darstellt. Mit Hilfe einer erfindungsgemäßen Decke enthaltend die erfindungsgemäßen OLEDs und/oder OLECs kann der emotionale Stress signifikant vermindert werden. Zudem ist eine bessere Temperierung des Kindes durch eine verringerte Wärmeproduktion der erfindungsgemäßen Vorrichtungen gegenüber herkömmlicher Bestrahlungsgeräte möglich.

Die erfindungsgemäßen Zusammensetzungen und/oder Vorrichtungen enthaltend die erfindungsgemäßen Zusammensetzungen, insbesondere organische Elektrolumineszenzvorrichtungen, zeichnen sich durch folgende überraschende Vorteile gegenüber dem Stand der Technik aus:

1. Die organischen Sensibilisatoren, die Zusammensetzungen und Vorrichtungen enthaltend diese sind wesentlich stabiler, vor allem gegenüber Luftsauerstoff und anderen Umwelteinflüssen, als Metallkomplexe aus dem Stand der Technik.

2. Die organischen Sensibilisatoren, Zusammensetzungen und Formulierungen enthaltend diese sind sehr einfach herzustellen und eignen sich insbesondere auch für die Massenproduktion.

3. Die erfindungsgemäßen Vorrichtungen liefern höhere Effizienzen als alle bisherigen elektolumineszierenden Vorrichtungen zur UpC.

4. Die erfindungsgemäßen Vorrichtungen ermöglichen die effiziente Nutzung von Triplett-Exzitonen, ohne, dass Verbindungen mit einem Schwermetallatom verwendet werden. Ohne die Verwendung von Schwermetallatomen können Triplettexzitonen normalerweise nicht genutzt werden (Abbilungen 3 und 4).

5. Die erfindungsgemäßen Vorrichtungen ermöglichen die Realisierung von UV-Emissionen unter Verwendung gängiger elektrolumineszierender Vorrichtungen.

6. Die Betriebsspannung der erfindungsgemäßen Vorrichtungen ist sehr niedrig.

7. Die erfindungsgemäßen Vorrichtungen zeigen keine Restemission im langwelligen Bereich, was in Zusammensetzungen gemäß dem Stand der Technik häufig zu sehen ist.

8. Viele erfindungsgemäße Zusammensetzungen lassen sich aus Lösung prozessieren. So lassen sich auf einfache Weise Schichten bilden und kostengünstig herstellen.

Die genannten Vorteile gehen nicht mit einer Verschlechterung der weiteren elektronischen Eigenschaften einher.

Auch ohne weitere Ausführungen wird davon ausgegangen, dass ein Fachmann die obige Beschreibung in weitestem Umfang nutzen kann.

Die Erfindung wird durch die nachfolgenden Beispiele und Abbildung näher erläutert, ohne sie dadurch einschränken zu wollen.

Kurzbeschreibung der Abbildungen

Abbildung 1: Vereinfachtes Jablonski Diagramm zur Veranschaulichung der Up-Conversion mittels TTA (Triplett-Triplett-Annihilation) unter optischen Anregung. Der Sensibilisator (I) wird mittels der Energie Ein (in Form von Photon) aus dem Grundzustand So in den angeregten Singulett Zustand $S_1$ angeregt. Es kommt zum Intersystem Crossing (ISC), d.h. unter Spinumkehr zum Übergang in den ersten angeregten Triplett-Zustand $T_1$. Danach kommt es zur Übertragung der Erergie aus $T_1$ des Sensibilisators auf das $T_1$ Niveau des Akzeptors (II) (TTET - Triplett-Triplett Energietransfer), wobei als Konkurrenzprozess die Phosphoreszenz $h\nu_l$ aus $T_1$ des Sensibilisators möglich ist. Wegen des rein organischen Charakters der Sensibilisatoren dieser Erfindung ist dieser Konkurrenzprozess im Vergleich zu schwermetallhaltigen gemäß dem Stand der Technik hier stark unterdrückt. Schließlich führt ein bimolekularer Stoß unter zwei Akzeptoren, die sich beide im angeregten Ti Zustand befinden, dazu, dass der eine Akzeptor in den angeregten $S_n$ Zustand und der andere Akzeptor in den elektronischen Grundzustand So überführt wird. Nach Relaxation (IC-Internal Conversion) von $S_n$ auf $S_1$ erfolgt die Emission $h\nu_{out}$ des Akzeptors aus dem $S_1$ Zustand

Abbildung 2: Eine Ausführungsform unter optischen Anregung, wobei das erste angeregte Singulett Niveau des Emitters $S_1$(E) niedriger ist als das des Sensibilisators $S_1$(S).

Abbildung 3: Eine Ausführungsform unter elektrischen Anregung, wobei das erste angeregte Singulett Niveau des Emitters $S_1$(E) höher ist als das des Sensibilisators $S_1$(S). Die Energie Ein repräsentiert die Energie des Elektron-Loch-Paars, wobei die Elektronen von der Kathode und die Löcher von der Anode injeziert wurden. Das Elektron-Loch-Paar rekombiniert auf dem Sensibilisator. Es bilden sich die angeregten Zustände $S_1$ und $T_1$. Der weitere Ablauf entspricht dem in Abbildung 1.

Abbildung 4: Eine Ausführungsform unter elektrischen Anregung, wobei das erste angeregte Singulett Niveau des Emitters $S_1$(E) niedriger ist als das des Sensibilisators $S_1$(S).

## Beispiele

### Beispiel 1

**Materialien und Synthese**

Das Polymer H1, das die Monomere (M1-M4) in den unten stehenden mol-Prozenten enthält, wird durch SUZUKI-Kupplung gemäß WO 2003/048225 hergestellt. H1 wird als erfindungsgemäßer Sensibilisator verwendet.

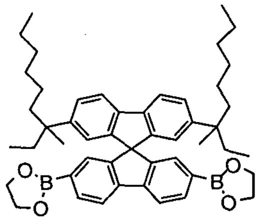

M1
50mol%

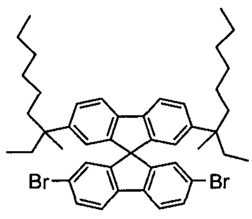

M2
27mol%

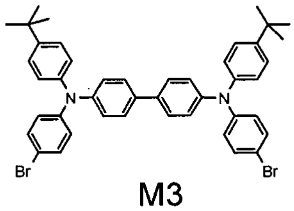

M3
8mol%

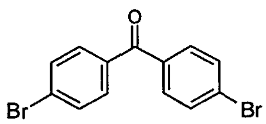

M4
15mol%

H2 wird nach WO 2004/093207 hergestellt.

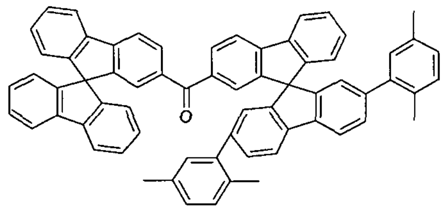

H1 und H2 werden als Sensibilisatoren verwendet. Ihre PL-Spektren (Photolumineszenz) zeigen für eine Anregung bei 325 nm im Falle von H1 ein schwaches Signal und für H2 nur Rauschen. Dies ist ein Beleg für eine hohe Intersystem-Crossing-Rate beider Sensibilitsatoren. Emitter1 wird nach WO 2008/006449 und Emitter2 nach DE 102008035413 hergestellt.

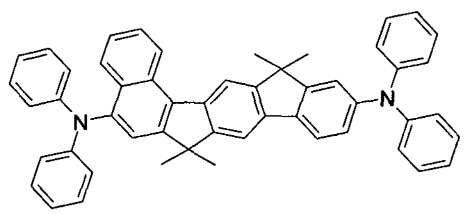

Emitter1

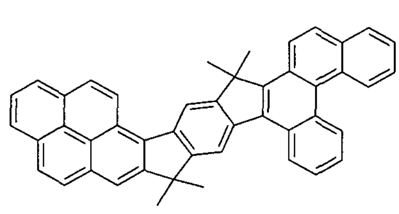

Emitter2

Als Referenzmaterialien werden organische Sensibilisatoren, die bekannt sind als Sensibilisatoren für UpC in der Photolumineszenz, eingesetzt (Nach J. Phys. Chem. A, Vol. 113, 2009 5913 und RSC Advances, 2011, 1, 937):

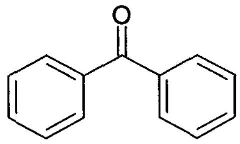
R1

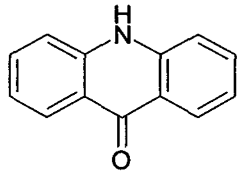
R2

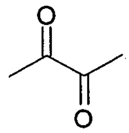
R3

R3 ist für elektrolumineszierende Vorrichtungen aufgrund seines fluiden Charakters ungeeignet.

**Beispiel 2**

**Quantenchemische Simulationen der Materialien**

Die HOMO- (highest occupied molecular orbital) und LUMO- (lowest unoccupied molecular orbital) Lagen sowie das Triplett/Singlett Niveau der organischen organischen Verbindungen werden über quantenchemische Rechnungen bestimmt. Hierzu wird das Programmpaket "Gaussian03W" (Gaussian Inc.) verwendet. Zur Berechnung organischer Substanzen ohne Metalle wird zuerst eine Geometrieoptimierung mit Hilfe einer semi-empirischen Methode "Ground State/Semi-empirical/ Default Spin/AM1" (Charge 0/Spin Singlet) durchgeführt. Im Anschluss erfolgt auf Grundlage der optimierten Geometrie eine Energierechnung. Hierbei wird die Methode "TD-SCF/DFT/ Default Spin/B3PW91" (time dependent - self consistent field/ density functional theory) mit dem Basissatz "6-31 G(d)" verwendet (Charge 0/Spin Singlet). Die wichtigsten Ergebnisse sind HOMO/LUMO-Niveaus und Energien für die Triplett-und Singulettangeregten Zustände. Die ersten angeregtenTriplett und Singulett Zustände, T1 und S1, sind hierbei am wichtigsten. Aus der Energierechnung erhält man das HOMO HEh bzw. LUMO LEh in Hartree-Einheiten. Daraus werden die HOMO- und LUMO-Werte in Elektronenvolt (eV) wie folgt bestimmt, wobei sich diese Beziehungen aus der Kalibrierung anhand von Cyclovoltammetriemessungen (CV) ergeben:

$$\mathrm{HOMO(eV)} = ((\mathrm{HEh}*27.212)-0.9899)/1.1206$$

$$\mathrm{LUMO(eV)} = ((\mathrm{LEh}*27.212)-2.0041)/1.385$$

Diese Werte sind im Sinne dieser Anmeldung als energetische Lage des HOMO-Niveaus bzw. des LUMO-Niveaus der Materialien anzusehen. Als Beispiel erhält man für die Verbindung H2 (Tabelle 1) aus der Rechnung ein HOMO von -0,20435 Hartrees und ein LUMO von -0,06350 Hartrees, was einem kalibrierten HOMO von -5,85 eV, einem kalibrierten LUMO von -2,69 eV.

**Tabelle 1**

| Material | Homo Corr. [eV] | Lumo Corr. [eV] | Triplett T1 [eV] | Singulett S1 [eV] |
|---|---|---|---|---|
| H2 | -5,85 | -2,69 | 2,70 | 3,35 |
| Emitter1 | -5,12 | -2,60 | 1,98 | 2,76 |
| Emitter2 | -5,37 | -2,86 | 1,81 | 2,81 |

Für Polymere, insbesondere konjugierte Polymere, werden die Berechnungen auf Trimere beschränkt, d.h. für ein Polymer enthaltend die Monomere M1 und M2 werden die Trimere M2-M1-M2 und / oder M1-M2-M1 berechnet, wobei polymerisierbare Gruppen entfernt werden. Weiterhin werden lange Alkylketten auf eine kurzer Kette reduziert. Exemplarisch soll dies anhand des Polymers H1 in der folgenden Darstellung verdeutlicht werden. Die gute Übereinstimmung zwischen CV-Messungen und Simulationen von Polymeren ist in WO 2008/011953 A1 offenbart.

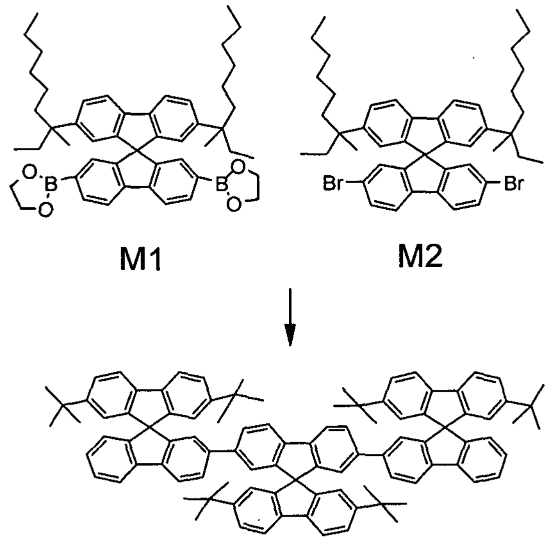

M1 M2

M1-M2-M1 or M2-M1-M2

Diese Werte sind im Sinne dieser Anmeldung als energetische Lage des HOMO-Niveaus bzw. des LUMO-Niveaus der Materialien anzusehen. Als Beispiel erhält man für das Polymer P1 (M1-M2-M1 in der Tabelle 2) mittels Simulation ein HOMO von -0,19301 Hartrees und ein LUMO von - 0,05377 Hartrees, was einem kalibrierten HOMO von -5,57 eV, einem kalibrierten LUMO von -2,50 eV entspricht.

**Tabelle 2** Energieniveau von Polymer H1

| | Homo Corr. [eV] | Lumo Corr. [eV] | Singulett S1 [eV] | Triplett T1 [eV] |
|---|---|---|---|---|
| M1-M2-M1 | -5,57 | -2,50 | 3,04 | 2,48 |
| M1-M3-M1 | -5,03 | -2,32 | 3,02 | 2,47 |
| M1-M4-M1 | -5,86 | -2,82 | 3,26 | 2,60 |

Anhand der Ergebnsse aus Tabelle 1 und 2 kann man erkennen, dass H1 und H2 ein T1- und S1-Niveau besitzen, das höher ist als das der Emitter1 und Emitter2.

**Beispiel 3**

**Lösungen und Zusammensetzungen enthaltend Sensibilisatoren sowie die Emitter 1 oder 2**

Lösungen, wie sie in Tabelle 3 zusammengefasst sind, werden wie folgt hergestellt: Zunächst werden die Sensibilisatoren und die Emitter in 10 ml Chlorbenzol in der angegebenen Konzentration gelöst und so lange gerührt, bis die Lösung klar ist. Die Lösung wird unter Verwendung eines Filters Millipore Millex LS, Hydrophobic PTFE 5.0 μm filtriert.

**Tabelle 3**

| | Zusammensetzung | Verhältnis (bezogen auf Gewicht) | Konzentration |
|---|---|---|---|
| Lösung 1 | H1 + Emitter1 | 93% : 7% | 12 mg/ml |
| Lösung 2 | H1 + Emitter2 | 93% : 7% | 12 mg/ml |
| Lösung 3 | H2 + Emitter1 | 93% : 7% | 24 mg/ml |
| Lösung 4 | H2 + Emitter2 | 93% : 7% | 24 mg/ml |
| Lösung 5 | R1 + Emitter1 | 93% : 7% | 24 mg/ml |
| Lösung 6 | R2 + Emitter1 | 93% : 7% | 24 mg/ml |

Die Lösungen werden verwendet, um die emittierende Schicht von OLEDs zu beschichten. Die entsprechende Feststoffzusammensetzung kann erhalten werden, indem das Lösungsmittel der Lösungen verdampft wird. Diese kann für die Herstellung weiterer Formulierungen verwendet werden.

**Beispiel 4**

**Herstellung der OLEDs**

OLED1 bis OLED6 mit der typischen Schichtenfolge, ITO/PEDOT/Interlayer/EML/Kathode (ITO - Indium-Zinnoxid Anode; EML - Emissionsschicht), werden unter Verwendung der entsprechenden Lösungen aus Tabelle 3 wie folgt hergestellt, d.h., OLED1 wird hergestellt mittels Lösung 1, OLED2 mittels Lösung 2 etc..

1. Auftragen von 80 nm PEDOT (Baytron P Al 4083) auf ein ITObeschichtetes Glassubstrat durch Spin-Coating. NAscfiließendes Ausheizen für 10 Minuten bei 120°C.
2. Auftragen von 20 nm eines Interlayers durch Spin-Coating einer Toluollösung von HIL-012 (Merck KGaA) (Konzentration 0.5 Gew.%) in einer Glovebox.
3. Ausheizen des Interlayers bei 180°C für 1 h in einer Glovebox.
4. Auftragen von 80 nm der emittierenden Schicht durch Spin-Coating einer der Lösung aus Tabelle 3.
5. Ausheizen der Vorrichtung bei 180°C für 10 min.
6. Aufdampfen einer Ba/Al-Kathode (3 nm + 150 nm).
7. Verkapselung der Vorrichtung.

Dabei werden bei der Herstellung der Vorrichtungen nur Techniken eingesetzt, die dem Fachmann gut bekannt sind.
OLED5 und OLED6 dienen als Vergleichbeispiele.

**Beispiel 5**

**Charakterisierung der OLEDs**

Die so erhaltenen OLEDs werden nach Standardmethoden charakterisiert, die dem Fachmann auf dem Gebiet gut bekannt sind. Dabei werden die folgenden Eigenschaften gemessen: UIL-Charakteristik, Elektrolumineszenzspektrum, Farbkoordinaten, Effizienz und Betriebsspannung. Die Ergebnisse sind in Tabelle 4 zusammengefasst, wobei OLED5 und OLED6 als Vergleich gemäß dem Stand der Technik dienen. In Tabelle 4 steht U(100) für die Spannung bei 100 $cd/m^2$, und U(1000) für die Spannung bei 1000 $cd/m^2$. Die Daten für die beiden OLEDs 5 und 6 können nicht ermittelt werden, da sie keine Elektrolumineszenz gezeigt haben.

**Tabelle 4**

| | Max. Eff. [cd/A] | U(1000) [V] | U(100) [V] | CIEx @ 100 $cd/m^2$ | CIEy @ 100 $cd/m^2$ | Max. EQE % |
|---|---|---|---|---|---|---|
| OLED1 | 1,3 | 5,7 | 4,3 | 0,19 | 0,31 | 0,63 |
| OLED2 | 1,2 | 5,8 | 4,3 | 0,16 | 0,22 | 0,71 |
| OLED3 | 1,2 | 9,3 | 6,3 | 0,19 | 0,34 | 0,52 |
| OLED4 | 1,2 | 8,4 | 5,8 | 0,16 | 0,26 | 0,67 |
| OLED5 | - | - | - | - | - | - |
| OLED6 | - | - | - | - | - | - |

Alle Sensibilisatoren H1-H2 enthalten Benzophenone oder Derivative.
Wie Tabelle 4 zeigt, können mit den erfindungsgemäßen Sensibilisatoren und Zusammensetzungen überraschend gute OLEDs hergestellt werden (OLED1, 2, 3, und 4). Dabei ist zu berücksichtigen, dass es sich um noch nicht optimierte Vorrichtungen zur Elektrolumineszenz handelt. Der Fachmann kann diese ohne erfinderisches Zutun unter Anwendung ihm gut bekannter Techniken mittels Routineexperimenten weiter verbessern.
Weiterhin wird die absolute PL Effizienz (Photolumineszenz) der emittierenden Schicht von OLED1 und OLED2 gemessen. Die Effizienzen beider sind kleiner als 0.5%, was noch niedriger als die entsprechende EQE ist. Der Sensibilisator H2 zeigt kein PL-Signal in der Schicht. In diesem Zusammenhang lässt sich der Mechanismus von den erfindungsgemäßen Vorrichtungen am besten mit der vorgeschlagenen TTA-UpC erklären. Die Vergleichbeispiele OLED5 und 6 haben nicht funktioniert.

## Patentansprüche

1. Zusammensetzung für Up-Conversion, enthaltend wenigstens einen Sensibilisator, der ein Polymer, Oligomer, Dendrimer oder kleines Molekül, das dadurch definiert ist, dass es ein Molekulargewicht kleiner als 4000 g/mol aufweist, ist und wenigstens einen fluoreszierenden organischen Emitter, der kein organischer Metallkomplex ist, **dadurch gekennzeichnet, dass** der Sensibilisator eine oder mehrere Struktureinheiten enthält, die aus den folgenden Verbindungen mit der allgemeinen Formel (1) ausgewählt sind und das Triplett-Niveau $T_1(S)$ des Sensibilisators höher liegt, als das Triplett-Niveau des Emitters $T_1(E)$,

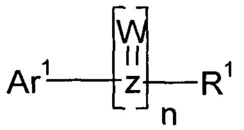

Formel (1),

wobei für die verwendeten Symbole gilt:

n ist entweder 1, 2 oder 3, bevorzugt 1 oder 2 und ganz bevorzugt 1;
W ist gleich oder verschieden bei jedem Auftreten gleich O, S oder Se, bevorzugt O oder S, ganz bevorzugt O;
$Ar^1$ ist ein aromatischer oder heteroaromatischer Ring oder ein aromatisches oder heteroaromatisches Ringsystem, wobei die Ringe mit einem oder meherern Resten $R^1$ substituiert sein können, mit der Maßgabe, dass $Ar^1$ wenigstens 9 Ringatome enthält;
Z ist gleich oder verschieden bei jedem Auftreten C oder S, bevorzugt C;
$R^1$ ist gleich oder verschieden bei jedem Auftreten H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten $R^2$ substituiert sein kann, wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy-, Arylalkoxy- oder Heteroaryloxygruppe mit 5 bis 60 aromatischen Ringatomen, die durch einen oder mehrere Reste $R^2$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^2$ substituiert sein kann, oder eine Kombination aus zwei oder mehr dieser Gruppen oder eine vernetzbare Gruppe Q;
$R^2$ ist gleich oder verschieden bei jedem Auftreten H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $R^3C=CR^3$, C=C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S oder $CONR^3$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen Ringatomen, das jeweils durch einen oder mehrere Reste $R^3$ substituiert sein kann, oder eine Aryloxy-, Arylalkoxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination aus zwei oder mehr dieser Gruppen; dabei können zwei oder mehrere benachbarte Reste $R^2$ miteinander ein mono- oder polycyclisches, aliphatisches oder aromatisches Ringsystem bilden;
$R^3$ ist gleich oder verschieden bei jedem Auftreten H, D, F oder ein aliphatischer, aromatischer und/oder heteroaromatischer Kohlenwasserstoffrest mit 1 bis 20 C-Atomen, in dem auch ein oder mehrere H-Atome durch

F ersetzt sein können; dabei können zwei oder mehrere Substituenten $R^3$ auch miteinander ein mono- oder polycyclisches, aliphatisches oder aromatisches Ringsystem bilden.

2. Zusammensetzung gemäß Anspruch 1, **dadurch gekennzeichnet, dass** die Struktureinheiten der Formel (1) ausgewählt sind aus den der Formeln (3) bis (7),

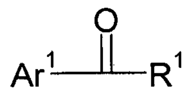

Formel (3)

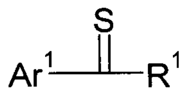

Formel (4)

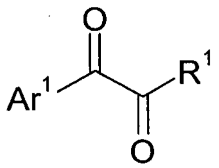

Formel (5)

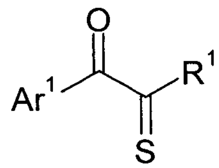

Formel (6)

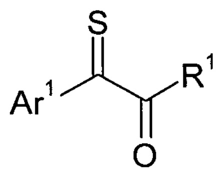

Formel (7),

wobei für die verwendeten Symbole die Definitionen des Anspruchs 1 gelten.

3. Zusammensetzung gemäß Anspruch 1 oder 2, **dadurch gekennzeichnet, dass** $Ar^1$ ausgewählt ist aus den Gruppen der Formeln (8) bis (14),

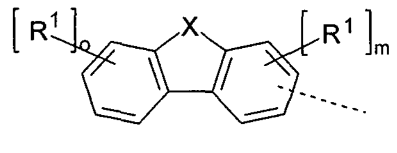

Formel (8)

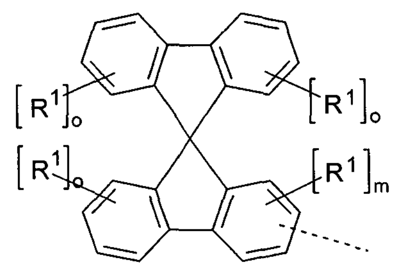

Formel (9)

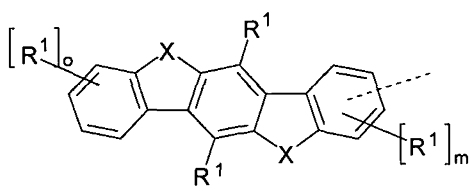

Formel (10)

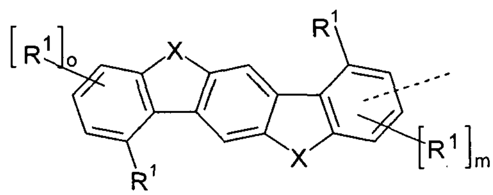

Formel (11)

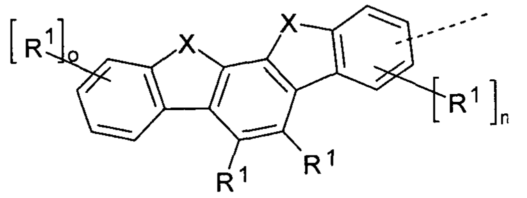
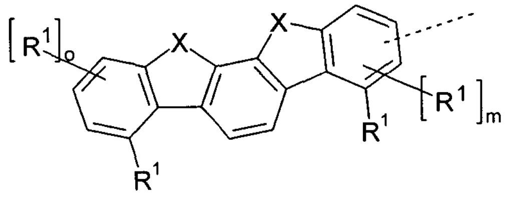

Formel (12) Formel (13)

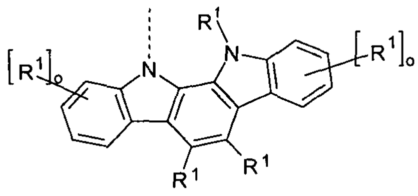

Formel (14),

wobei $R^1$ dieselbe Bedeutung hat, in Anspruch 1 beschrieben, die gestrichelte Bindung die Verknüpfungsposition darstellt und weiterhin gilt:

X ist gleich oder verschieden bei jedem Auftreten eine bivalente Brücke, ausgewählt aus $B(R^1)$, $C(R^1)_2$, $Si(R^1)_2$, C=O, $C=NR^1$, $C=C(R^1)_2$, O, S, S=O, $SO_2$, $N(R^1)$, $P(R^1)$ und $P(=O)R^1$, bevorzugt aus $C(R^1)_2$ und $N(R^1)$;
m ist bei jedem Auftreten gleich oder verschieden 0, 1, 2 oder 3;
o ist bei jedem Auftreten gleich oder verschieden 0, 1, 2, 3 oder 4.

4. Zusammensetzung gemäß einem oder mehreren der Ansprüche 1 bis 3, **dadurch gekennzeichnet, dass** $R^1$ ausgewählt ist aus einer der folgenden Formeln (34) bis (222), wobei die Verbindungen mit den angegebenen Formeln (34) bis (222) mit einem oder meherern, identischen oder verschiedenen Resten $R^2$ substituiert sein können, wobei $R^2$ wie in Anspruch 1 definiert ist

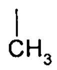
Formel (34)

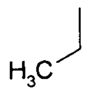
Formel (35)

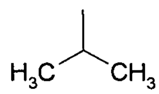
Formel (36)

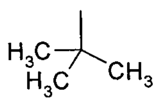
Formel (37)

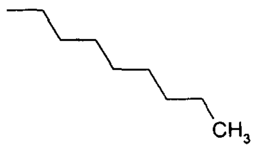
Formel (38)

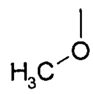
Formel (39)

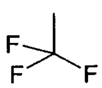
Formel (40)

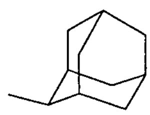
Formel (41)

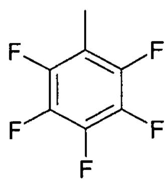
Formel (42)

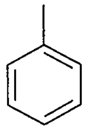
Formel (43)

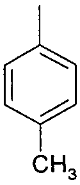
Formel (44)

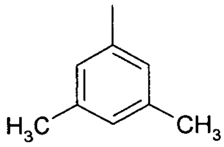
Formel (45)

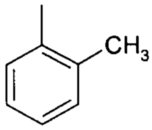
Formel (46)

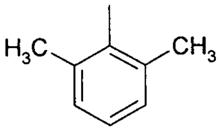
Formel (47)

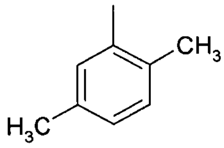
Formel (48)

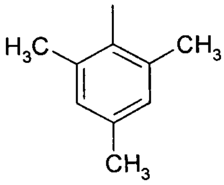
Formel (49)

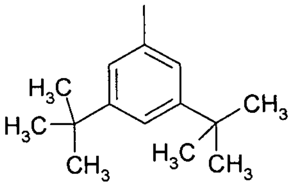
Formel (50)

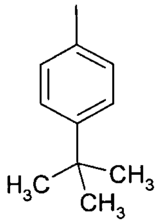
Formel (51)

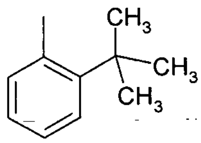
Formel (52)

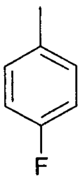
Formel (53)

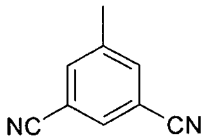
Formel (54)

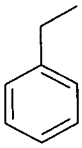
Formel (55)

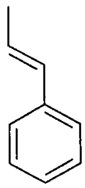
Formel (56)

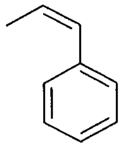
Formel (57)

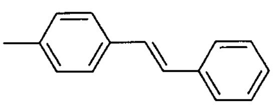
Formel (58)

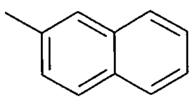
Formel (59)

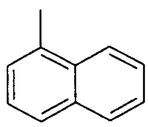
Formel (60)

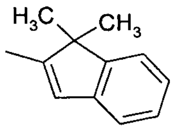
Formel (61)

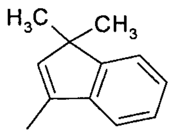
Formel (62)

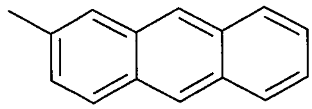
Formel (63)

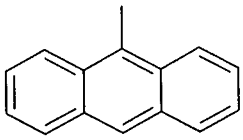
Formel (64)

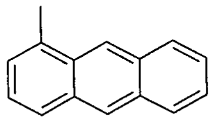
Formel (65)

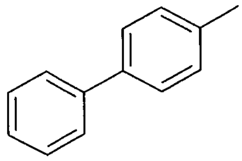
Formel (66)

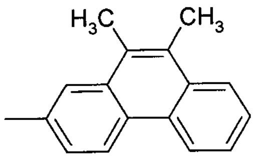
Formula (67)

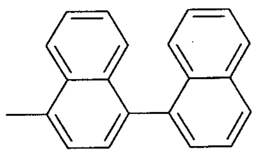
Formula (68)

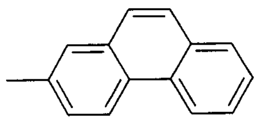
Formel (69)

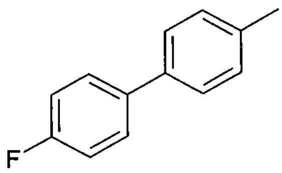
Formel (70)

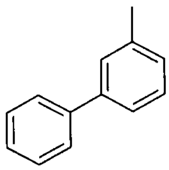
Formel (71)

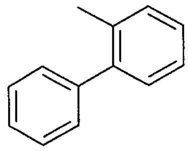
Formel (72)

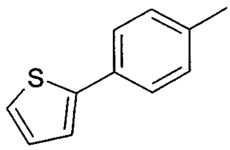
Formel (73)

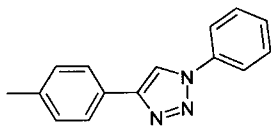
Formel (74)

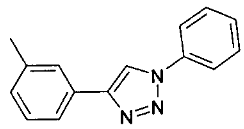
Formel (75)

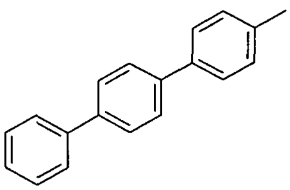
Formel (76)

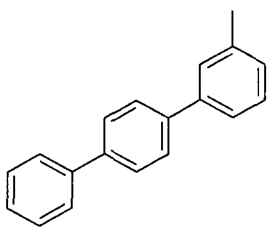
Formel (77)

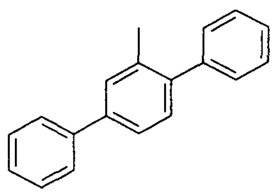
Formel (78)

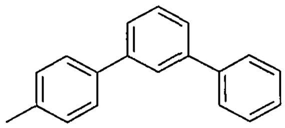

Formel (79)

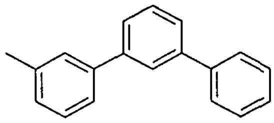

Formel (80)

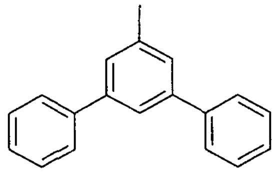

Formel (81)

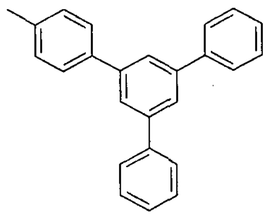

Formel (82)

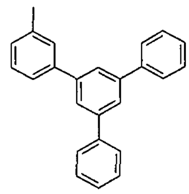

Formel (83)

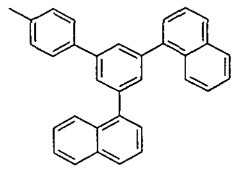

Formel (84)

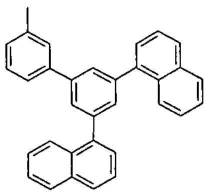

Formel (85)

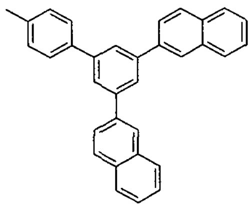

Formel (86)

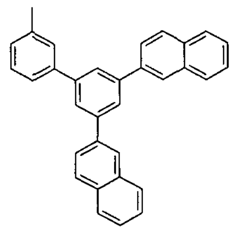

Formel (87)

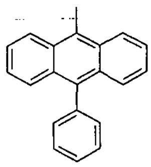

Formel (88)

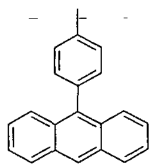

Formel (89)

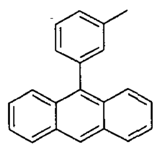

Formel (90)

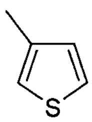

Formel (91)

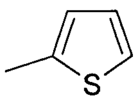

Formel (92)

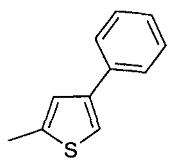

Formel (93)

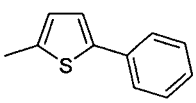

Formel (94)

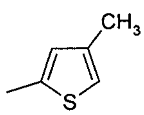

Formel (95)

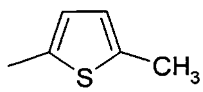

Formel (96)

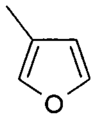

Formel (97)

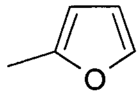

Formel (98)

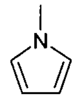

Formel (99)

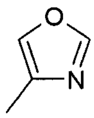

Formel (100)

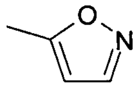

Formel (101)

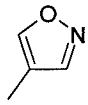

Formel (102)

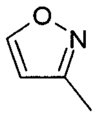

Formel (103)

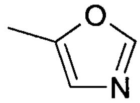

Formel (104)

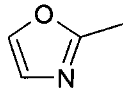

Formel (105)

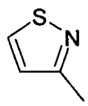

Formel (106)

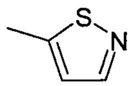

Formel (107)

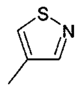

Formel (108)

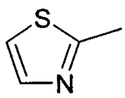

Formel (109)

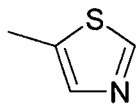

Formel (110)

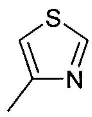

Formel (111)

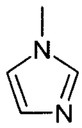

Formel (112)

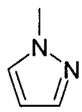

Formel (113)

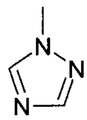

Formel (114)

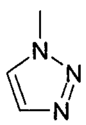

Formel (115)

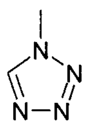

Formel (116)

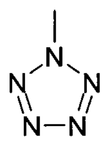

Formel (117)

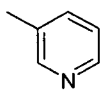

Formel (118)

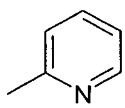

Formula (119)

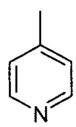

Formel (120)

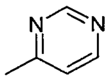
Formel (121)

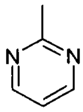
Formula (122)

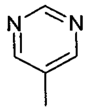
Formel (123)

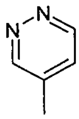
Formel (124)

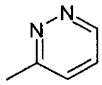
Formel (125)

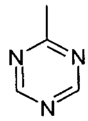
Formel (126)

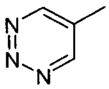
Formel (127)

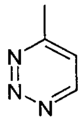
Formel (128)

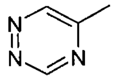
Formel (129)

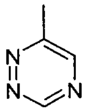
Formel (130)

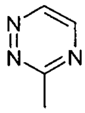
Formel (131)

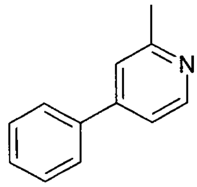
Formel (132)

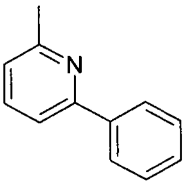
Formel (133)

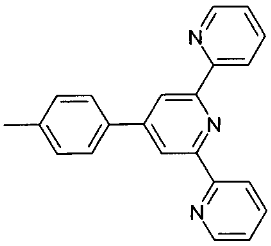
Formel (134)

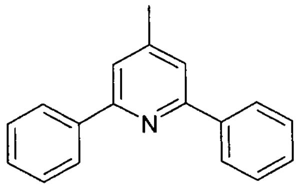
Formel (135)

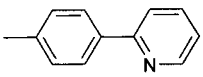
Formel (136)

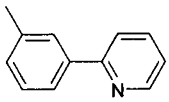
Formel (137)

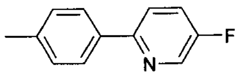
Formel (138)

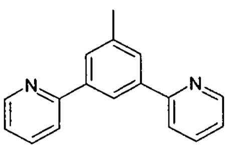
Formel (139)

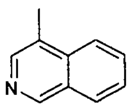
Formel (140)

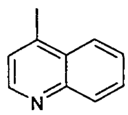
Formel (141)

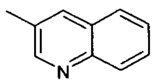

Formel (142)

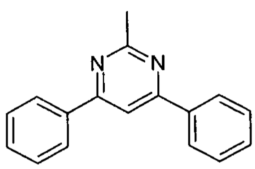

Formel (143)

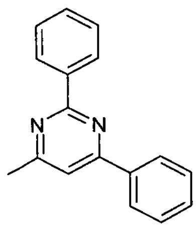

Formel (144)

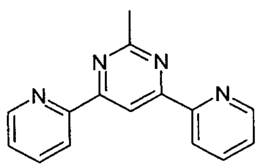

Formel (145)

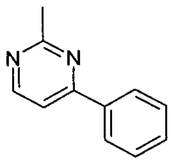

Formel (146)

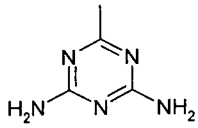

Formel (147)

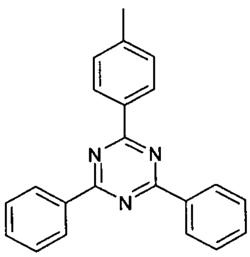

Formel (148)

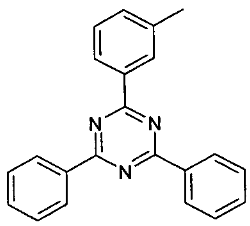

Formel (149)

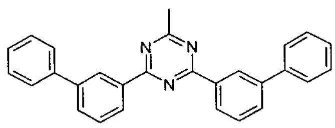

Formel (150)

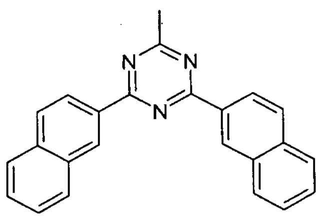

Formel (151)

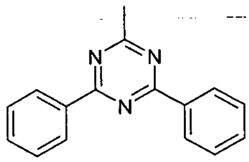

Formel (152)

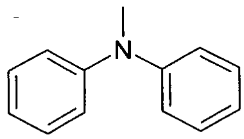

Formel (153)

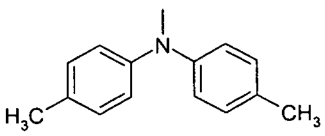

Formel (154)

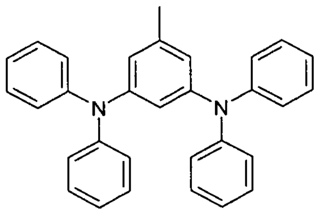

Formel (155)

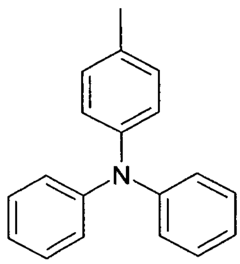

Formel (156)

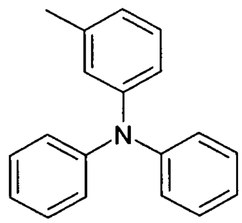

Formel (157)

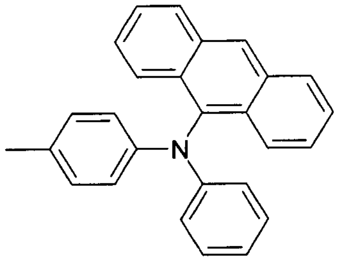

Formel (158)

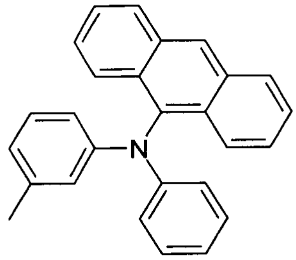

Formel (159)

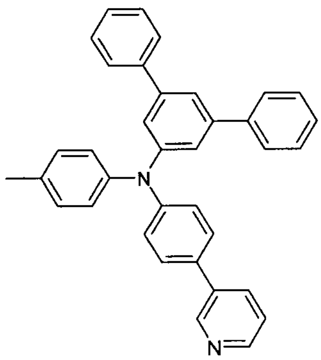

Formel (160)

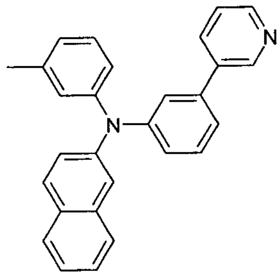

Formel (161)

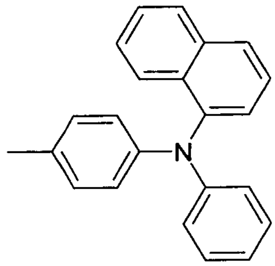

Formel (162)

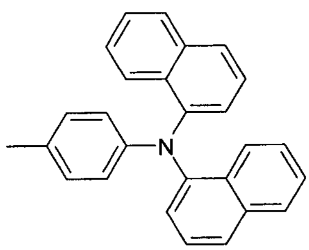

Formel (163)

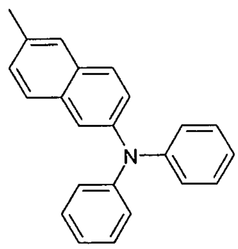

Formel (164)

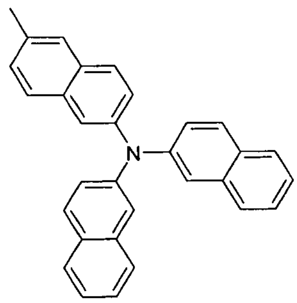

Formel (165)

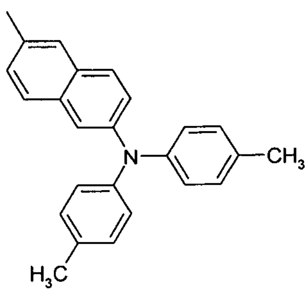

Formel (166)

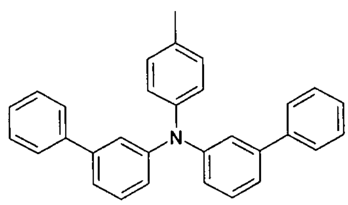

Formel (167)

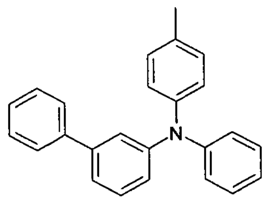

Formel (168)

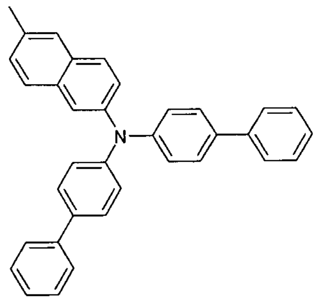
Formel (169)

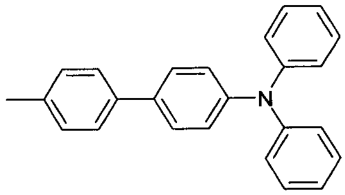
Formel (170)

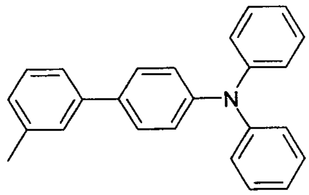
Formel (171)

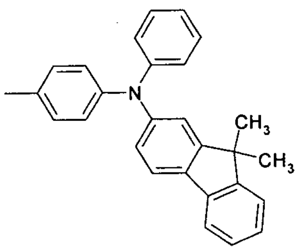
Formel (172)

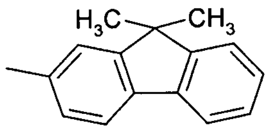
Formel (173)

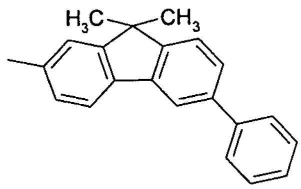
Formel (174)

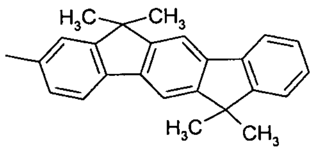
Formel (175)

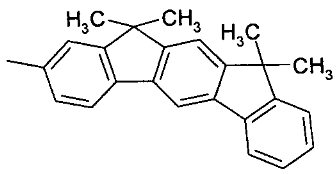
Formel (176)

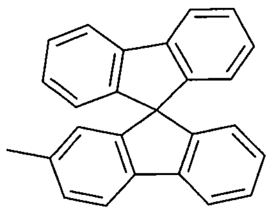
Formel (177)

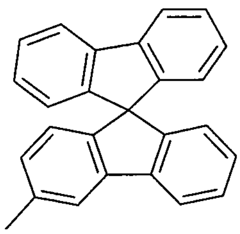
Formel (178)

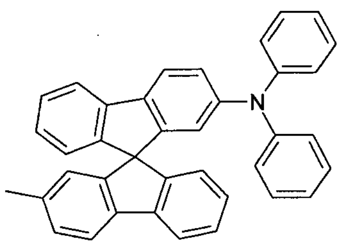
Formel (179)

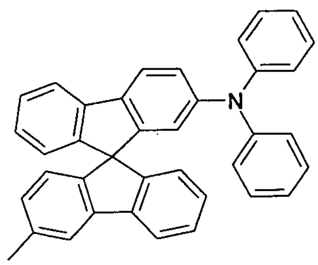
Formel (180)

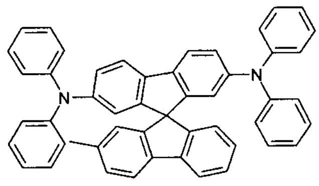

Formel (181)

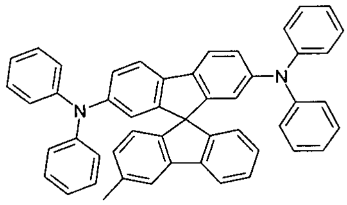

Formel (182)

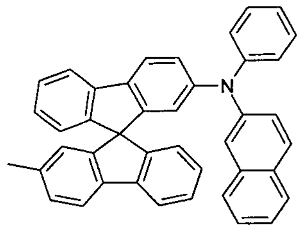

Formel (183)

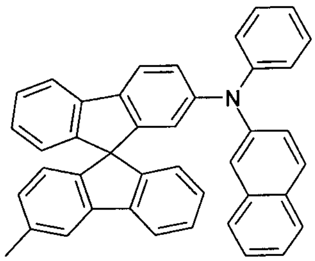

Formel (184)

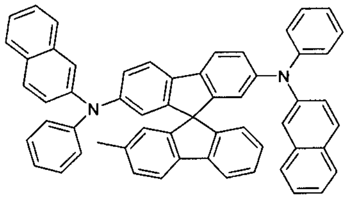

Formel (185)

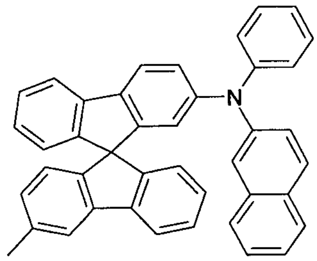

Formel (186)

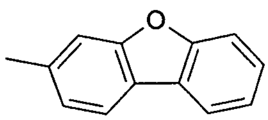

Formel (187)

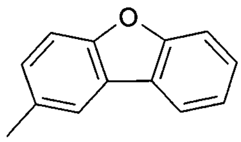

Formel (188)

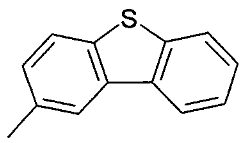

Formel (189)

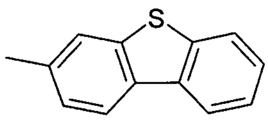

Formel (190)

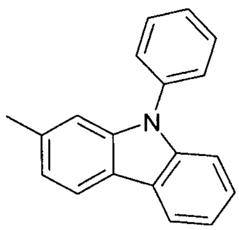

Formel (191)

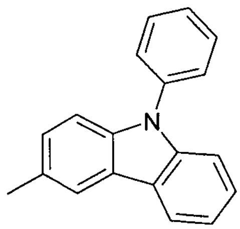

Formel (192)

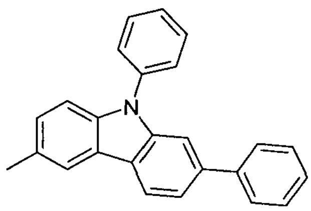

Formel (193)

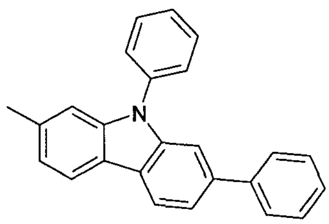

Formel (194)

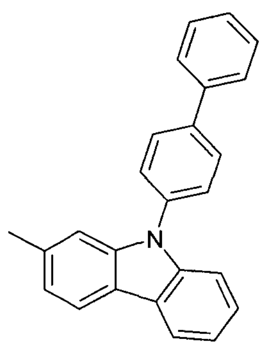

Formel (195)

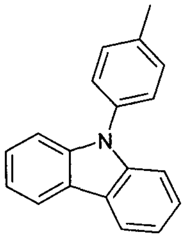

Formel (196)

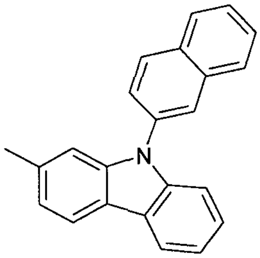

Formel (197)

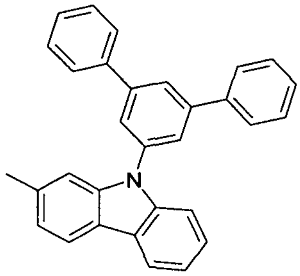

Formel (198)

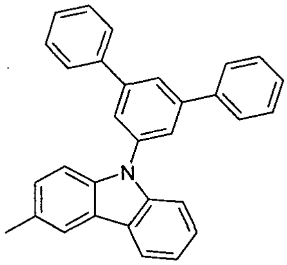

Formel (199)

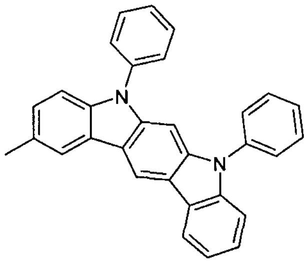

Formel (200)

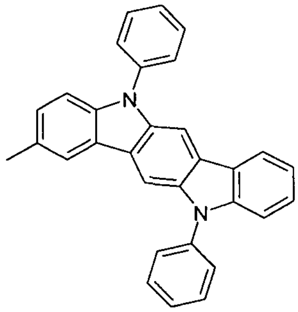

Formel (201)

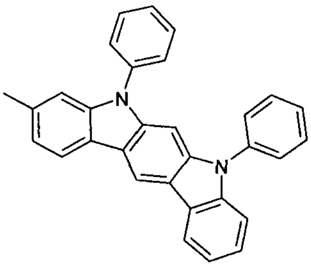

Formel (202)

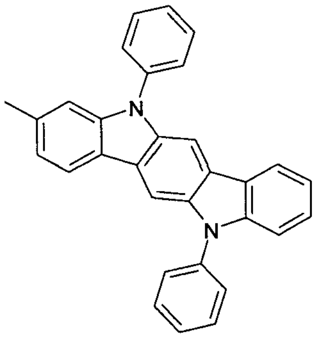

Formel (203)

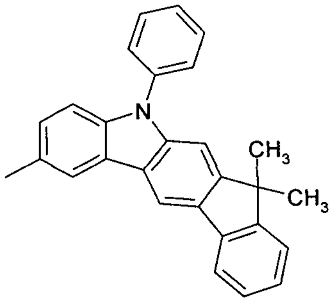

Formel (204)

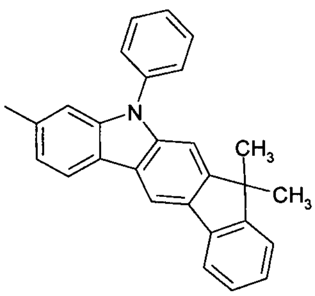

Formel (205)

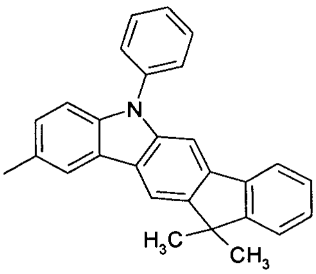

Formel (206)

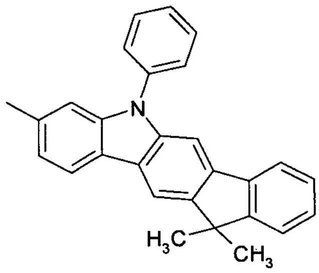

Formel (207)

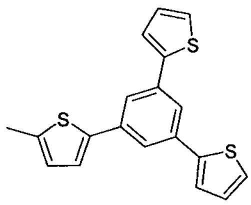
Formel (208)

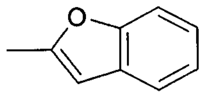
Formel (209)

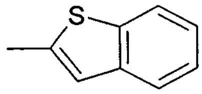
Formel (210)

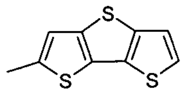
Formel (211)

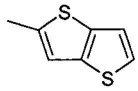
Formel (212)

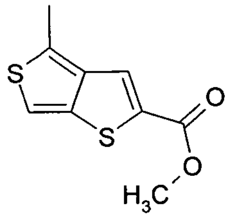
Formel (213)

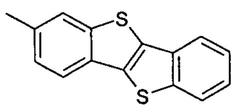
Formel (214)

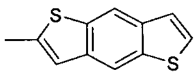
Formel (215)

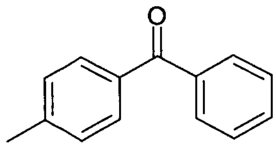
Formel (216)

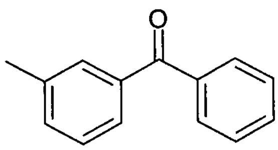
Formel (217)

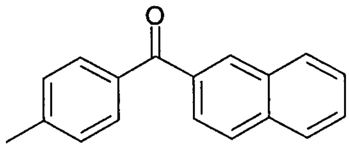
Formel (218)

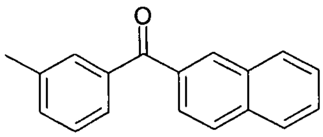
Formel (219)

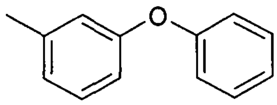
Formel (220)

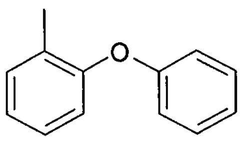
Formel (221)

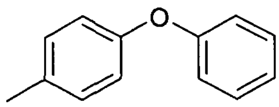
Formel (222).

**5.** Zusammensetzung gemäß einem oder mehreren der Ansprüche 1 bis 4, **dadurch gekennzeichnet, dass** das erste angeregte Singulett Niveau des Emitters $S_1(E)$ niedriger ist als das des Sensibilisators $S_1(S)$.

**6.** Verwendung der Zusammensetzung gemäß einem oder meheren der Ansprüche 1 bis 5 für Up-Conversion.

**7.** Verwendung gemäß Anspruch 6, **dadurch gekennzeichnet, dass** blaues Licht oder Strahlung in UV Bereich erzeugt wird.

**8.** Verwendung gemäß Anspruch 6 oder 7 für Up-Conversion in elektrolumineszierenden Vorrichtungen.

**9.** Optische und/oder elektronische Vorrichtung enthaltend mindestens eine Zusammensetzung gemäß einem oder

mehreren der Ansprüche 1 bis 5.

**10.** Vorrichtung gemäß Anspruch 9, **dadurch gekennzeichnet, dass** es sich um organischen Elektrolumineszenzvorrichtungen, bevorzugt um organische lichtemittierende Dioden (OLED), organische lichtemittierende Transistoren, organische lichtemittierende elektrochemische Zellen (OLEC, LEC oder LEEC), organischen lichtemittierende elektrochemische Transistoren oder einen organischen Laser handelt.

**11.** Vorrichtung gemäß Anspruch 9 oder 10 zur Verwendung in der Medizin zur Phototherapie.

**12.** Vorrichtung gemäß Anspruch 11 zur Verwendung zur Behandlung der Haut mittels Phototherapie.

**13.** Vorrichtung gemäß Anspruch 11 oder 12, **dadurch gekennzeichnet, dass** Psoriasis, Vitiligo, Neugeborenenikterus, Dermatitis und atopische Dermatitis, Hautkrebs, Veränderungen des Bindegewebes, bevorzugt Psoriasis behandelt wird.

**14.** Vorrichtung gemäß einem oder mehreren der Ansprüche 11 bis 13, **dadurch gekennzeichnet, dass** mit einer Wellenlänge kleiner als 400 nm behandelt wird.

**15.** Verwendung der Vorrichtung gemäß Anspruch 9 oder 10 in der Kosmetik zur Bestrahlung der Haut.

**16.** Verwendung gemäß Anspruch 15, **dadurch gekennzeichnet, dass** es sich bei der kosmetischen Anwendung um eine Anwendung im Bereich der Akne, Cellulite, Hautrötung, Hautfaltenbildung und Hautverjüngung handelt.

## Claims

**1.** Composition for up-conversion, comprising at least one sensitiser, which is a polymer, oligomer, dendrimer or small molecule, which is defined as having a molecular weight of less than 4000 g/mol, and at least one fluorescent organic emitter which is not an organic metal complex, **characterised in that** the sensitiser contains one or more structural units selected from the following compounds having the general formula (1) and the triplet level $T_1(S)$ of the sensitiser is higher than the triplet level of the emitter $T_1(E)$,

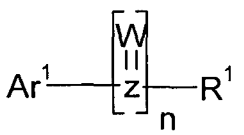

formula (1),

where the following applies to the symbols used:

n is either 1, 2 or 3, preferably 1 or 2 and very preferably 1;
W is, identically or differently on each occurrence, equal to O, S or Se, preferably O or S, very preferably O;
$Ar^1$ is an aromatic or heteroaromatic ring or an aromatic or heteroaromatic ring system, where the rings may be substituted by one or more radicals $R^1$, with the proviso that $Ar^1$ contains at least 9 ring atoms;
Z is, identically or differently on each occurrence, C or S, preferably C;
$R^1$ is, identically or differently on each occurrence, H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy, alkylalkoxy or thioalkoxy group having 3 to 40 C atoms, which may in each case be substituted by one or more radicals $R^2$, where one or more H atoms may be replaced by D, F, Cl, Br, I, CN or $NO_2$, or an aromatic or heteroaromatic ring system having 5 to 60 aromatic ring atoms, which may in each case be substituted by one or more radicals $R^2$, or an aryloxy, arylalkoxy or heteroaryloxy group having 5 to 60 aromatic ring atoms, which may be substituted by one or more radicals $R^2$, or a diarylamino group, diheteroarylamino group or arylheteroarylamino group having 10 to 40 aromatic ring atoms, which may be substituted by one or more radicals $R^2$, or a combination of two or more of these groups or a cross-linkable group Q;
$R^2$ is, identically or differently on each occurrence, H, D, F, Cl, Br, I, $N(R^3)_2$, CN, $NO_2$, $Si(R^3)_3$, $B(OR^3)_2$, $C(=O)R^3$,

$P(=O)(R^3)_2$, $S(=O)R^3$, $S(=O)_2R^3$, $OSO_2R^3$, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy, alkylalkoxy or thioalkoxy group having 3 to 40 C atoms, which may in each case be substituted by one or more radicals $R^3$, where one or more non-adjacent $CH_2$ groups may be replaced by $R^3C=CR^3$, C=C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S or $CONR^3$ and where one or more H atoms may be replaced by D, F, Cl, Br, I, CN or $NO_2$, or an aromatic or heteroaromatic ring system having 5 to 60 aromatic ring atoms, which may in each case be substituted by one or more radicals $R^3$, or an aryloxy, arylalkoxy or heteroaryloxy group having 5 to 60 aromatic ring atoms, which may be substituted by one or more radicals $R^3$, or a diarylamino group, diheteroarylamino group or arylheteroarylamino group having 10 to 40 aromatic ring atoms, which may be substituted by one or more radicals $R^3$, or a combination of two or more of these groups; two or more adjacent radicals $R^2$ here may form a mono- or polycyclic, aliphatic or aromatic ring system with one another;

$R^3$ is, identically or differently on each occurrence, H, D, F or an aliphatic, aromatic and/or heteroaromatic hydrocarbon radical having 1 to 20 C atoms, in which, in addition, one or more H atoms may be replaced by F; two or more substituents $R^3$ here may also form a mono- or polycyclic, aliphatic or aromatic ring system with one another.

2. Composition according to Claim 1, **characterised in that** the structural units of the formula (1) are selected from those of the formulae (3) to (7),

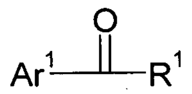

formula (3)

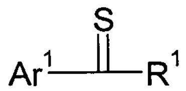

formula (4)

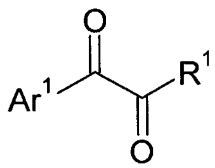

formula (5)

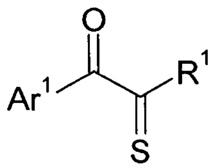

formula (6)

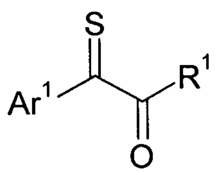

formula (7),

where the definitions of Claim 1 apply to the symbols used.

3. Composition according to Claim 1 or 2, **characterised in that** $Ar^1$ is selected from the groups of the formulae (8) to (14),

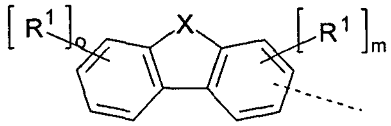

formula (8)

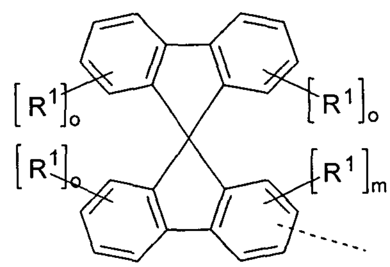

formula (9)

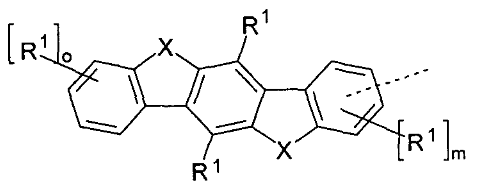

formula (10)

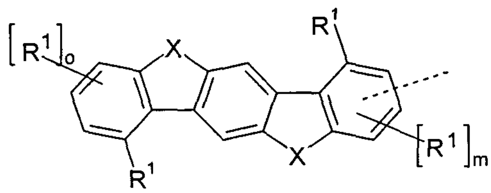

formula (11)

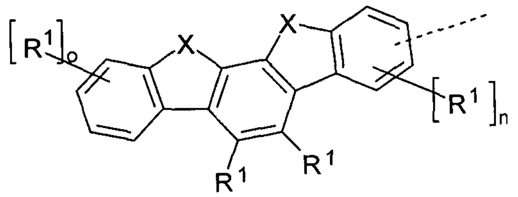

formula (12)

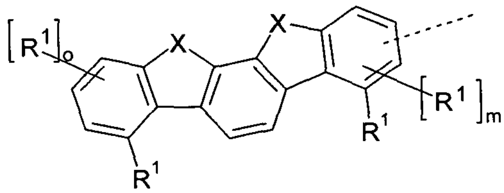

formula (13)

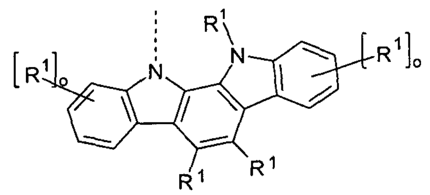

formula (14),

where $R^1$ has the same meaning as described in Claim 1, the dashed bond represents the linking position and furthermore:

X is, identically or differently on each occurrence, a divalent bridge selected from $B(R^1)$, $C(R^1)_2$, $Si(R^1)_2$, C=O, $C=NR^1$, $C=C(R^1)_2$, O, S, S=O, $SO_2$, $N(R^1)$, $P(R^1)$ and $P(=O)R^1$, preferably from $C(R^1)_2$ and $N(R^1)$;
m is on each occurrence, identically or differently, 0, 1, 2 or 3;
o is on each occurrence, identically or differently, 0, 1, 2, 3 or 4.

**4.** Composition according to one or more of Claims 1 to 3, **characterised in that** $R^1$ is selected from one of the following formulae (34) to (222), where the compounds having the formulae (34) to (222) indicated may be substituted by one or more, identical or different radicals $R^2$, where $R^2$ is defined as in Claim 1

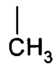

formula (34)

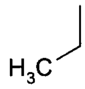

formula (35)

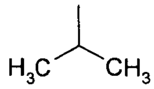

formula (36)

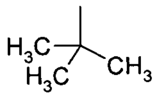

formula (37)

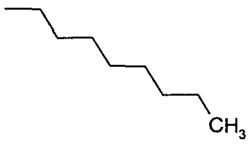

formula (38)

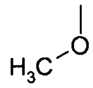

formula (39)

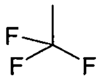

formula (40)

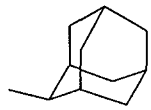

formula (41)

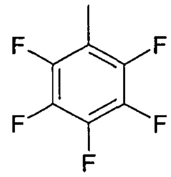

formula (42)

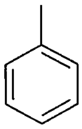

formula (43)

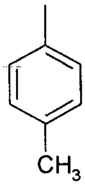

formula (44)

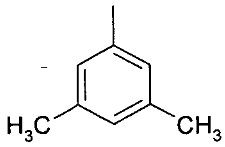

formula (45)

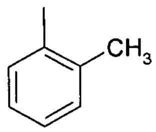

formula (46)

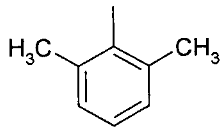

formula (47)

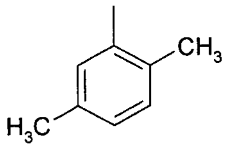

formula (48)

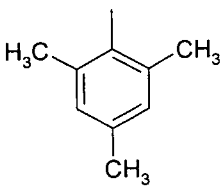

formula (49)

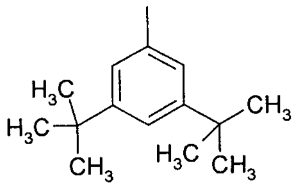

formula (50)

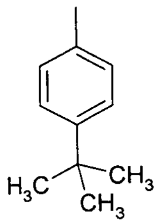

formula (51)

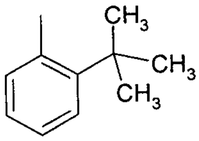

formula (52)

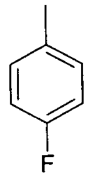

formula (53)

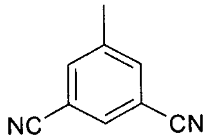

formula (54)

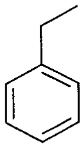

formula (55)

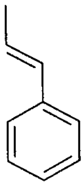

formula (56)

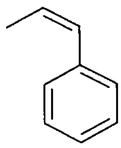

formula (57)

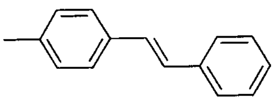

formula (58)

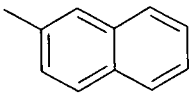

formula (59)

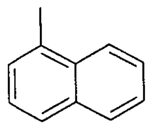

formula (60)

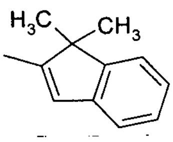

formula (61)

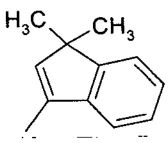

formula (62)

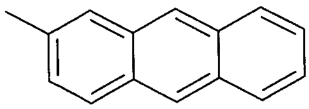

formula (63)

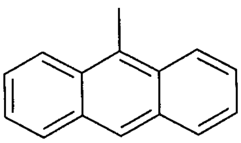

formula (64)

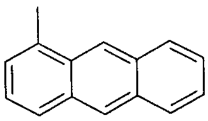

formula (65)

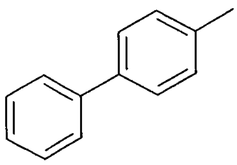

formula (66)

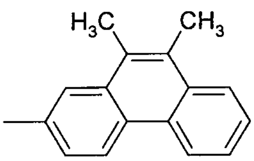

Formula (67)

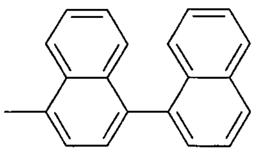

Formula (68)

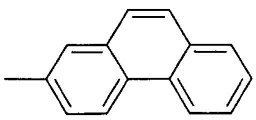

formula (69)

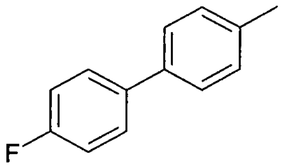
formula (70)

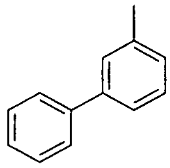
formula (71)

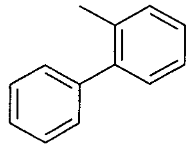
formula (72)

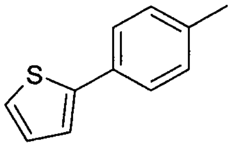
formula (73)

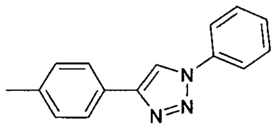
formula (74)

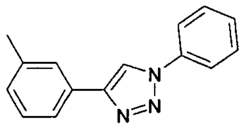
formula (75)

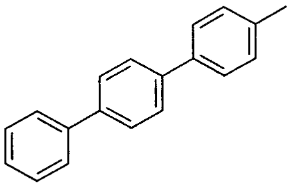
formula (76)

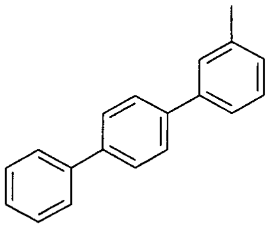
formula (77)

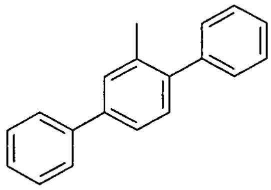
formula (78)

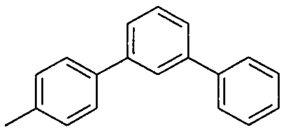
formula (79)

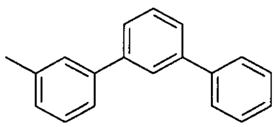
formula (80)

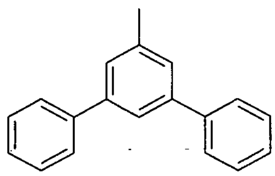
formula (81)

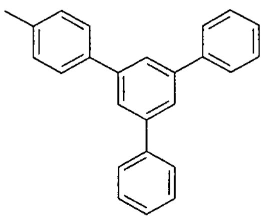
formula (82)

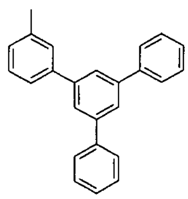
formula (83)

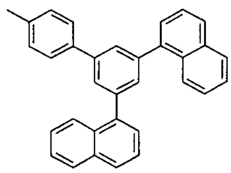
formula (84)

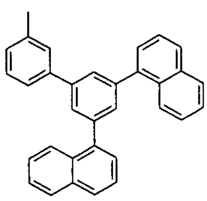
formula (85)

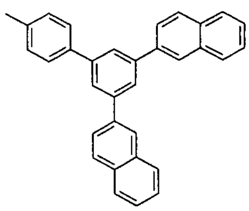
formula (86)

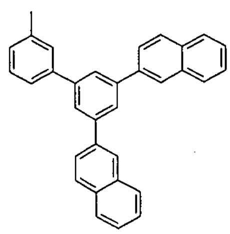
formula (87)

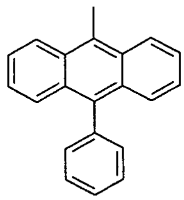
formula (88)

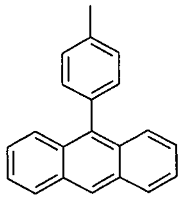
formula (89)

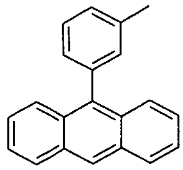
formula (90)

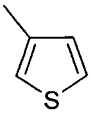
formula (91)

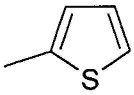
formula (92)

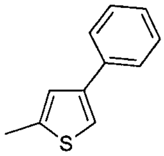
formula (93)

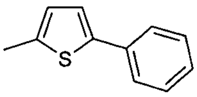
formula (94)

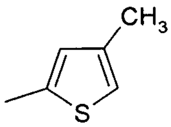
formula (95)

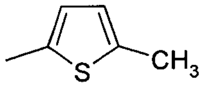
formula (96)

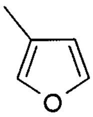
formula (97)

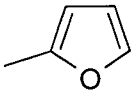
formula (98)

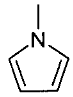
formula (99)

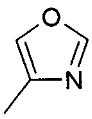
formula (100)

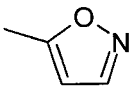
formula (101)

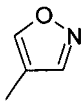
formula (102)

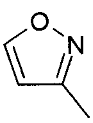
formula (103)

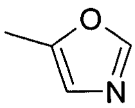
formula (104)

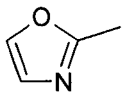
formula (105)

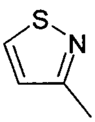
formula (106)

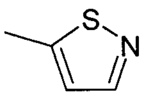
formula (107)

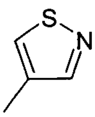
formula (108)

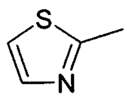

formula (109)

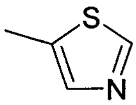

formula (110)

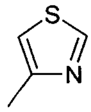

formula (111)

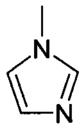

formula (112)

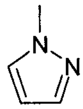

formula (113)

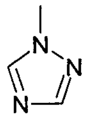

formula (114)

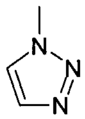

formula (115)

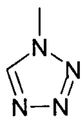

formula (116)

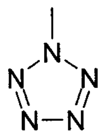

formula (117)

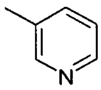

formula (118)

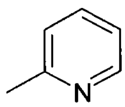

Formula (119)

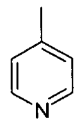

formula (120)

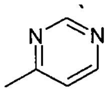

formula (121)

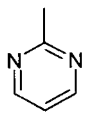

Formula (122)

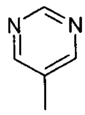

formula (123)

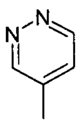

formula (124)

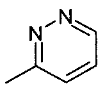

formula (125)

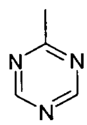

formula (126)

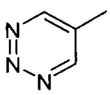

formula (127)

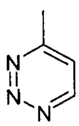

formula (128)

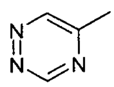

formula (129)

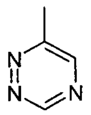

formula (130)

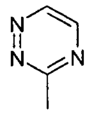

formula (131)

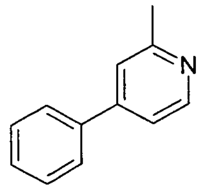

formula (132)

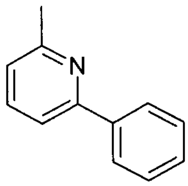

formula (133)

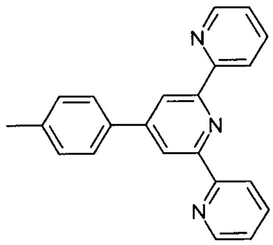

formula (134)

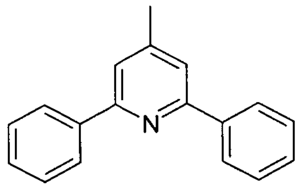

formula (135)

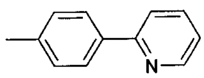

formula (136)

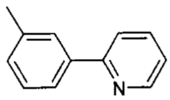

formula (137)

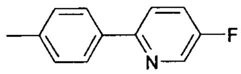

formula (138)

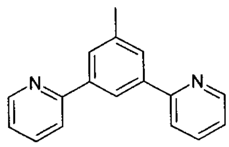

formula (139)

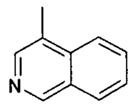

formula (140)

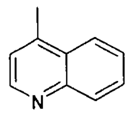

formula (141)

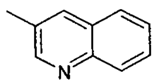

formula (142)

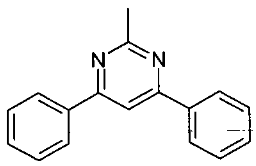

formula (143)

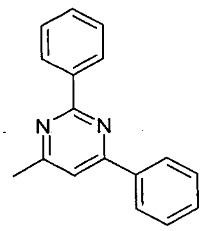

formula (144)

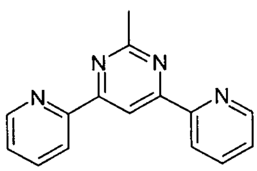

formula (145)

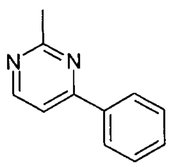

formula (146)

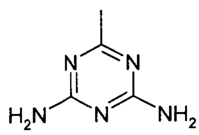

formula (147)

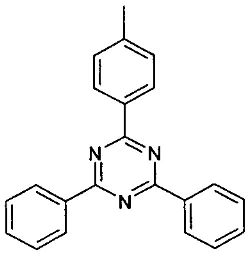

formula (148)

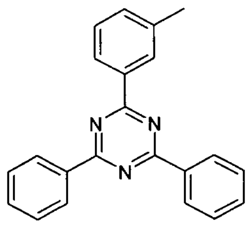

formula (149)

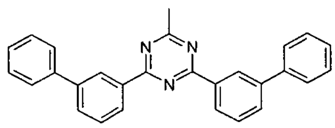

formula (150)

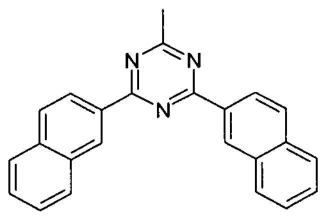

formula (151)

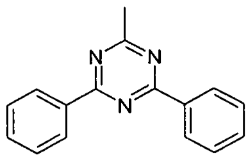

formula (152)

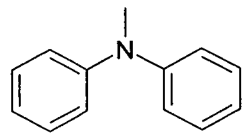

formula (153)

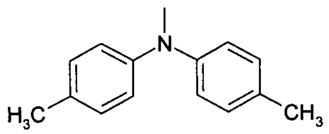

formula (154)

‼ EMBED ISISServer

formula (155)

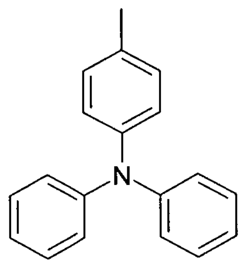

formula (156)

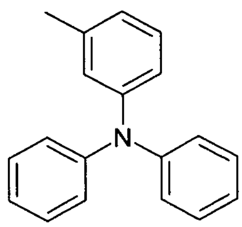

formula (157)

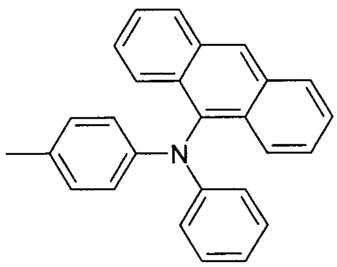

formula (158)

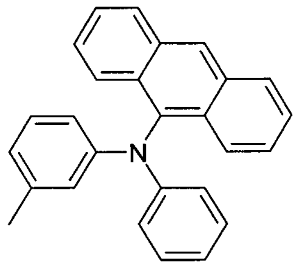

formula (159)

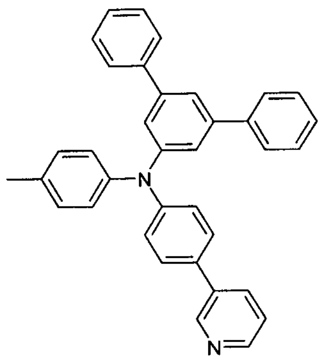

formula (160)

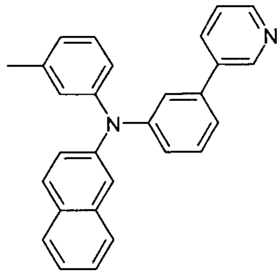

formula (161)

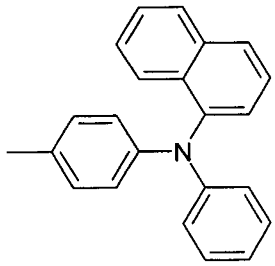

formula (162)

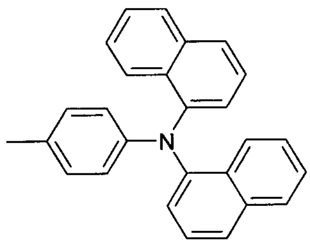

formula (163)

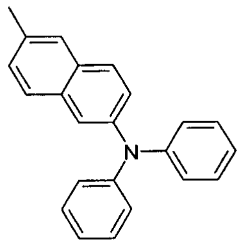

formula (164)

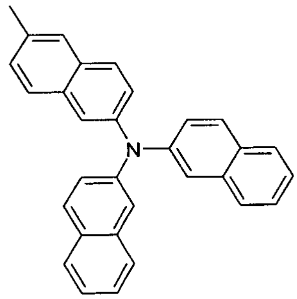

formula (165)

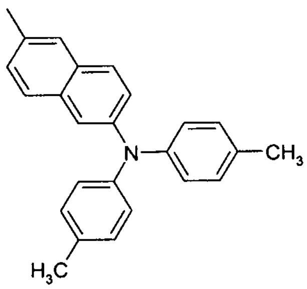

formula (166)

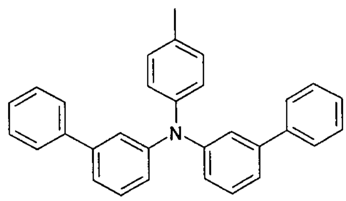

formula (167)

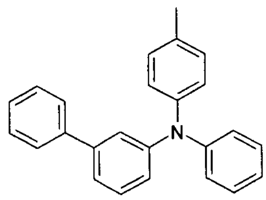

formula (168)

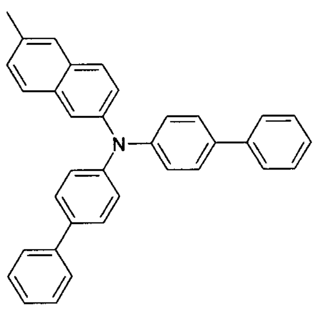

formula (169)

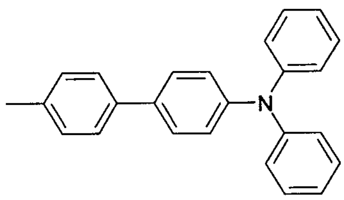

formula (170)

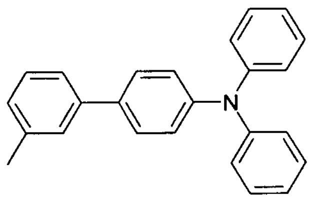

formula (171)

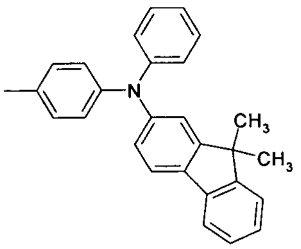
formula (172)

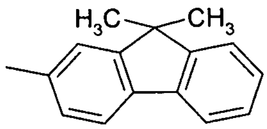
formula (173)

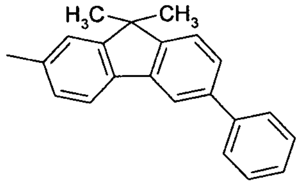
formula (174)

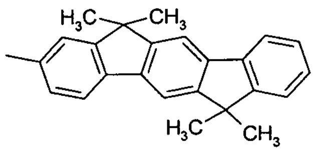
formula (175)

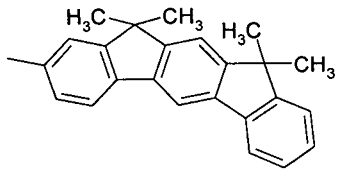
formula (176)

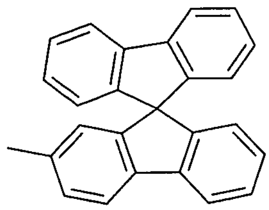
formula (177)

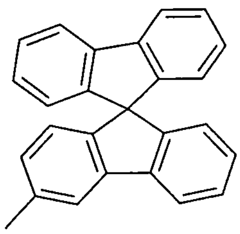
formula (178)

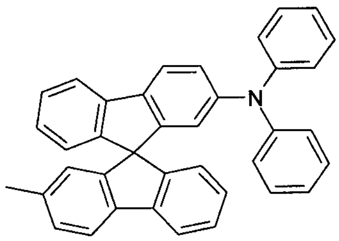
formula (179)

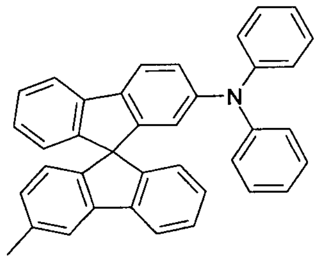
formula (180)

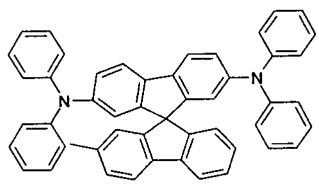
formula (181)

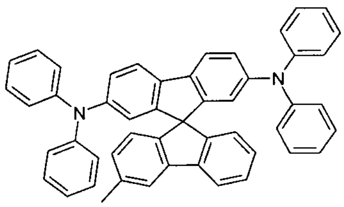
formula (182)

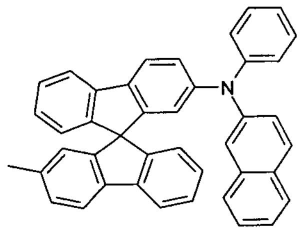
formula (183)

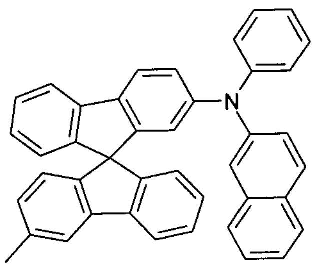
formula (184)

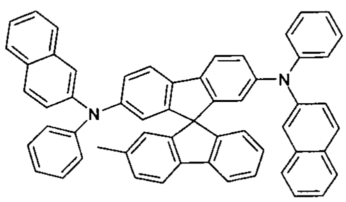
formula (185)

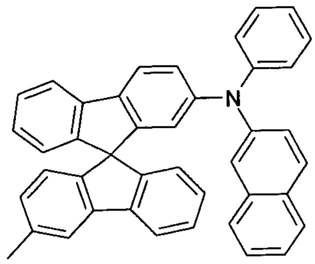
formula (186)

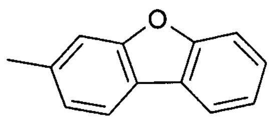
formula (187)

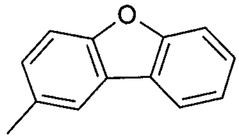
formula (188)

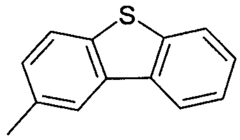
formula (189)

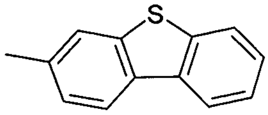
formula (190)

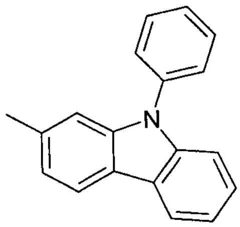
formula (191)

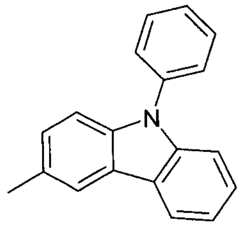
formula (192)

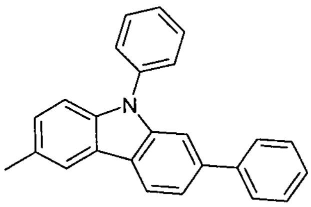
formula (193)

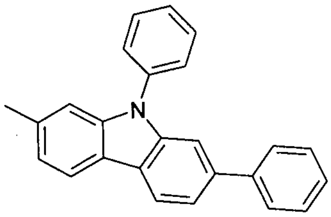
formula (194)

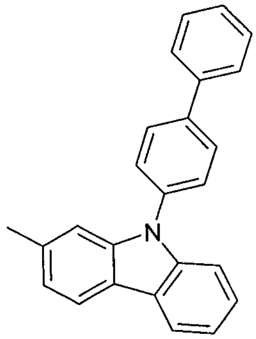
formula (195)

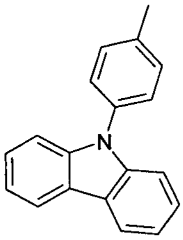
formula (196)

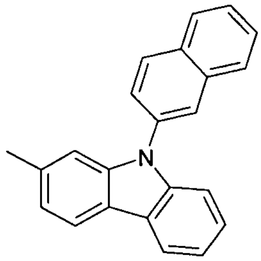
formula (197)

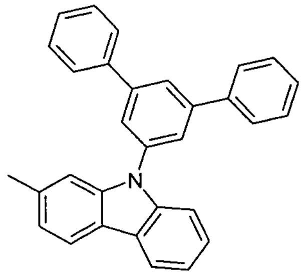
formula (198)

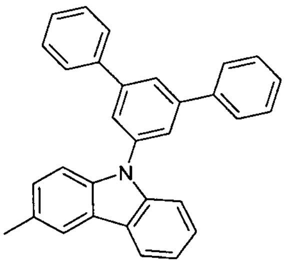
formula (199)

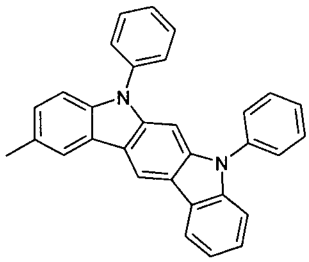
formula (200)

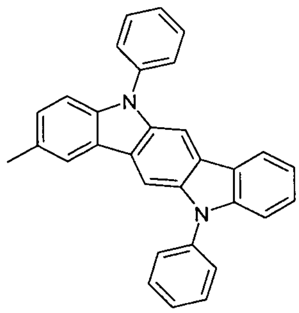
formula (201)

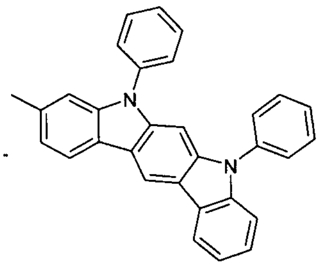

formula (202)

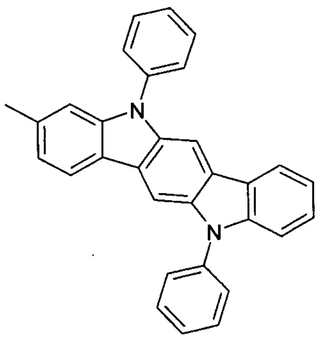

formula (203)

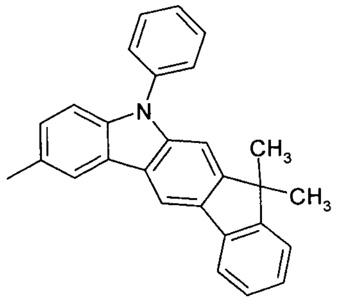

formula (204)

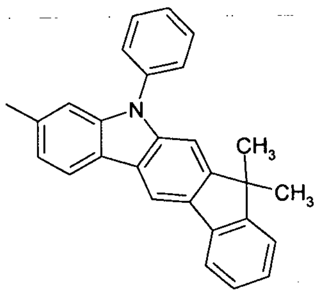

formula (205)

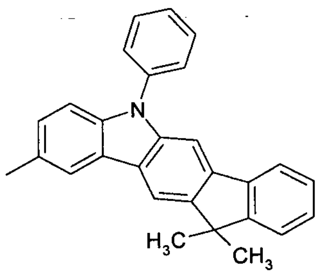

formula (206)

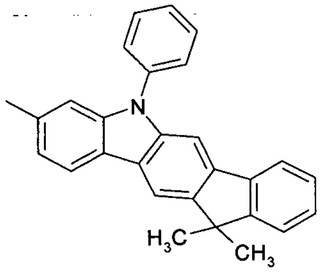

formula (207)

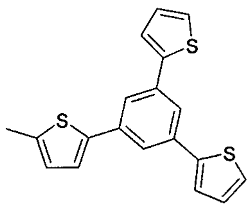

formula (208)

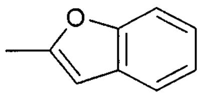

formula (209)

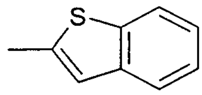

formula (210)

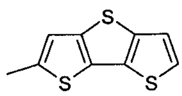

formula (211)

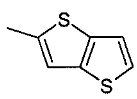

formula (212)

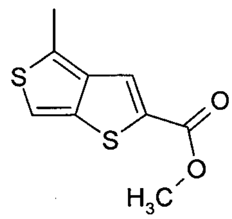

formula (213)

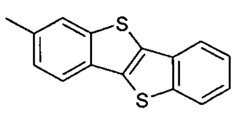

formula (214)

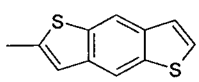

formula (215)

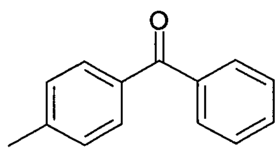

formula (216)

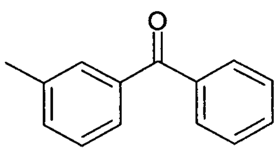

formula (217)

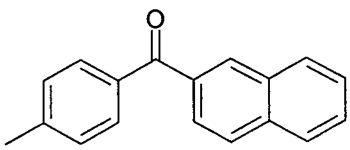

formula (218)

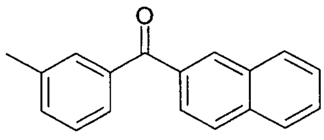

formula (219)

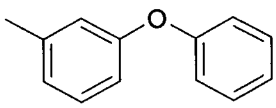

formula (220)

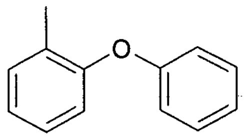

formula (221)

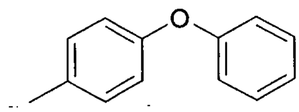

formula (222).

5. Composition according to one or more of Claims 1 to 4, **characterised in that** the first excited singlet level of the emitter $S_1$(E) is lower than that of the sensitiser $S_1$(S).

6. Use of the composition according to one or more of Claims 1 to 5 for up-conversion.

7. Use according to Claim 6, **characterised in that** blue light or radiation in UV region is generated.

8. Use according to Claim 6 or 7 for up-conversion in electroluminescent devices.

9. Optical and/or electronic device containing at least one composition according to one or more of Claims 1 to 5.

10. Device according to Claim 9, **characterised in that** it is organic electroluminescent devices, preferably organic light-emitting diodes (OLEDs), organic light-emitting transistors, organic light-emitting electrochemical cells (OLECs, LECs or LEECs), organic light-emitting electrochemical transistors or an organic laser.

11. Device according to Claim 9 or 10 for use in medicine for phototherapy.

12. Device according to Claim 11 for use for the treatment of the skin by means of phototherapy.

13. Device according to Claim 11 or 12, **characterised in that** psoriasis, vitiligo, jaundice of the newborn, dermatitis and atopic dermatitis, skin cancer, changes in the connective tissue, preferably psoriasis, is treated.

14. Device according to one or more of Claims 11 to 13, **characterised in that** the treatment is carried out with a wavelength less than 400 nm.

15. Use of the device according to Claim 9 or 10 in the cosmetics field for irradiation of the skin.

16. Use according to Claim 15, **characterised in that** the cosmetic application is an application in the area of acne, cellulite, skin reddening, skin wrinkling and skin rejuvenation.

## Revendications

1. Composition pour une up-conversion ou conversion-élévation, comprenant au moins un sensibilisateur, lequel est un polymère, un oligomère, un dendrimère ou une petite molécule, laquelle est définie comme présentant un poids moléculaire inférieur à 4000 g/mol, et au moins un émetteur organique fluorescent qui n'est pas un complexe organométallique, **caractérisée en ce que** le sensibilisateur contient une ou plusieurs unité(s) structurelle(s) choisie(s) parmi les composés qui suivent présentant la formule générale (1) et le niveau de triplet $T_1$(S) du sensibilisateur est supérieur au niveau de triplet de l'émetteur $T_1$(E),

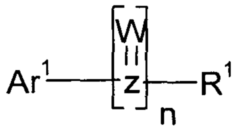

formule (1),

dans laquelle ce qui suit s'applique aux symboles utilisés :

n est soit 1, soit 2, soit 3, de façon préférable 1 ou 2 et de façon très préférable 1 ;

W est, de manière identique ou différente pour chaque occurrence, égal à O, S ou Se, de façon préférable O ou S, de façon très préférable O ;

$Ar^1$ est un cycle aromatique ou hétéroaromatique ou un système de cycle aromatique ou hétéroaromatique, où les cycles peuvent être substitués par un radical ou plusieurs radicaux $R^1$, étant entendu que $Ar^1$ contient au moins 9 atomes de cycle ;

Z est, de manière identique ou différente pour chaque occurrence, C ou S, de façon préférable C ;

$R^1$ est, de manière identique ou différente pour chaque occurrence, H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, un groupe alkyle, alcoxy ou thioalcoxy en chaîne droite qui comporte 1 à 40 atome(s) de C ou un groupe alkényle ou alkynyle en chaîne droite qui comporte 2 à 40 atomes de C ou un groupe alkyle, alkényle, alkynyle, alcoxy, alkylalcoxy ou thioalcoxy ramifié ou cyclique qui comporte 3 à 40 atomes de C, lequel peut, dans chaque cas, être substitué par un radical ou plusieurs radicaux $R^2$, où un ou plusieurs atome(s) de H peut/peuvent être remplacé(s) par D, F, Cl, Br, I, CN ou $NO_2$, ou un système de cycle aromatique ou hétéroaromatique qui comporte 5 à 60 atomes de cycle aromatique, lequel peut, dans chaque cas, être substitué par un radical ou plusieurs radicaux $R^2$, ou un groupe aryloxy, arylalcoxy ou hétéroaryloxy qui comporte 5 à 60 atomes de cycle aromatique, lequel peut être substitué par un radical ou plusieurs radicaux $R^2$, ou un groupe diarylamino, un groupe dihétéroarylamino ou un groupe arylhétéroarylamino qui comporte 10 à 40 atomes de cycle aromatique, lequel peut être substitué par un radical ou plusieurs radicaux $R^2$, ou une combinaison de deux de ces groupes ou plus ou un groupe réticulable Q;

$R^2$ est, de manière identique ou différente pour chaque occurrence, H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, un groupe alkyle, alcoxy ou thioalcoxy en chaîne droite qui comporte 1 à 40 atome(s) de C ou un groupe alkényle ou alkynyle en chaîne droite qui comporte 2 à 40 atomes de C ou un groupe alkyle, alkényle, alkynyle, alcoxy, alkylalcoxy ou thioalcoxy ramifié ou cyclique qui comporte 3 à 40 atomes de C, lequel peut dans chaque cas être substitué par un radical ou plusieurs radicaux $R^3$, où un ou plusieurs groupe(s) $CH_2$ non adjacents peut/ peuvent être remplacé(s) par $R^3C=CR^3$, C=C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S ou $CONR^3$ et où un ou plusieurs atome(s) de H peut/peuvent être remplacé(s) par D, F, Cl, Br, I, CN ou $NO_2$, ou un système de cycle aromatique ou hétéroaromatique qui comporte 5 à 60 atomes de cycle aromatique, lequel peut dans chaque cas être substitué par un radical ou plusieurs radicaux $R^3$, ou un groupe aryloxy, arylalcoxy ou hétéroaryloxy qui comporte 5 à 60 atomes de cycle aromatique, lequel peut être substitué par un radical ou plusieurs radicaux $R^3$, ou un groupe diarylamino, un groupe dihétéroarylamino ou un groupe arylhétéroarylamino qui comporte 10 à 40 atomes de cycle aromatique, lequel peut être substitué par un radical ou plusieurs radicaux $R^3$, ou une combinaison de deux de ces groupes ou plus ; deux radicaux $R^2$ adjacents ou plus peuvent ici former un système de cycle aliphatique ou aromatique monocyclique ou polycyclique l'un avec l'autre ou les uns avec les autres ;

$R^3$ est, de manière identique ou différente pour chaque occurrence, H, D, F ou un radical hydrocarbone aliphatique, aromatique et/ou hétéroaromatique qui comporte 1 à 20 atome(s) de C, où, en outre, un ou plusieurs atome(s) de H peut/peuvent être remplacé(s) par F ; deux substituants $R^3$ ou plus peuvent ici également former un système de cycle aliphatique ou aromatique monocyclique ou polycyclique l'un avec l'autre ou les uns avec les autres.

2. Composition selon la revendication 1, **caractérisée en ce que** les unités structurelles de la formule (1) sont choisies parmi celles des formules (3) à (7),

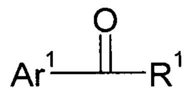

formule (3)

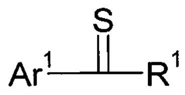

formule (4)

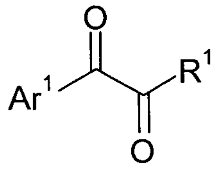

formule (5)

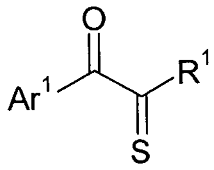

formule (6)

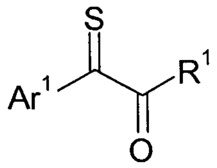

formule (7),

où les définitions de la revendication 1 s'appliquent aux symboles utilisés.

**3.** Composition selon la revendication 1 ou 2, **caractérisée en ce que** $Ar^1$ est choisi parmi les groupes des formules (8) à (14),

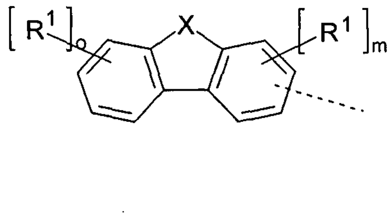

formule (8)

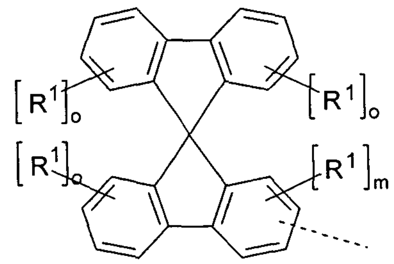

formule (9)

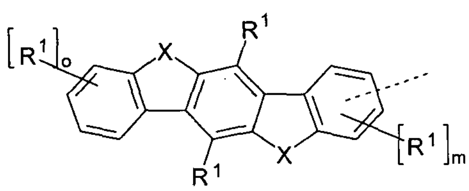

formule (10)

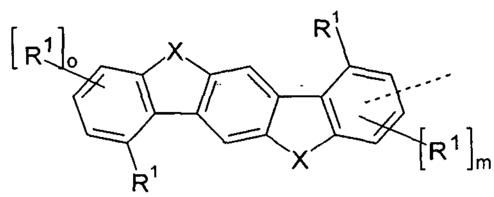

formule (11)

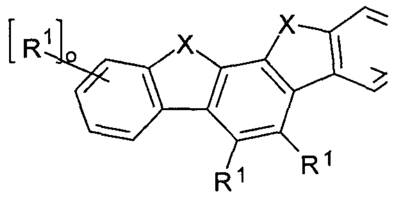

formule (12)

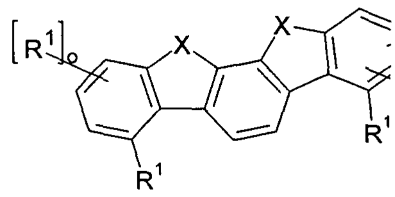

formule (13)

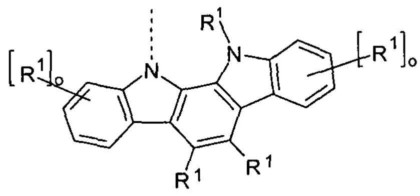

formule (14),

dans lesquelles $R^1$ présente la même signification que décrit selon la revendication 1, le lien en pointillés représente la position de liaison et en outre :

X est, de manière identique ou différente pour chaque occurrence, un pont divalent choisi parmi $B(R^1)$, $C(R^1)_2$, $Si(R^1)_2$, C=O, $C=NR^1$, $C=C(R^1)_2$, O, S, S=O, $SO_2$, $N(R^1)$, $P(R^1)$ et $P(=O)R^1$, de façon préférable parmi $C(R^1)_2$ et $N(R^1)$ ;
m est pour chaque occurrence, de manière identique ou différente, 0, 1, 2 ou 3 ;
o est pour chaque occurrence, de manière identique ou différente, 0, 1, 2, 3 ou 4.

**4.** Composition selon une ou plusieurs des revendications 1 à 3, **caractérisée en ce que** $R^1$ est choisi parmi l'une des formules qui suivent (34) à (222), où les composés présentant les formules (34) à (222) indiquées peuvent être substitués par un radical ou plusieurs radicaux $R^2$, identiques ou différents, où $R^2$ est défini comme selon la revendication 1 :

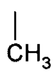

formule (34)

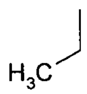

formule (35)

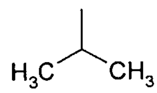

formule (36)

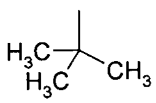

formule (37)

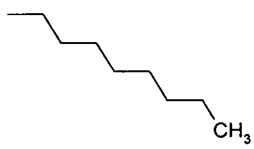

formule (38)

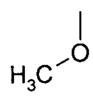

formule (39)

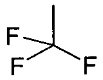

formule (40)

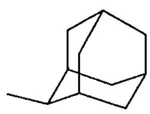

formule (41)

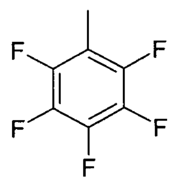

formule (42)

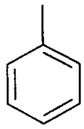

formule (43)

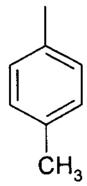

formule (44)

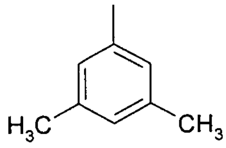

formule (45)

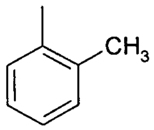

formule (46)

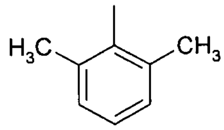

formule (47)

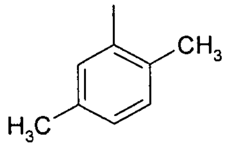

formule (48)

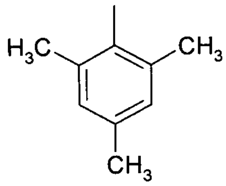

formule (49)

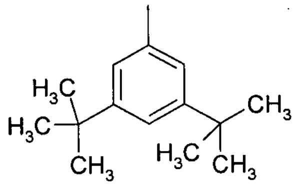

formule (50)

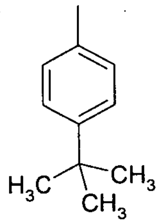

formule (51)

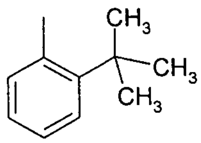

formule (52)

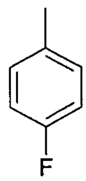

formule (53)

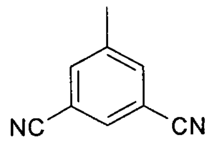

formule (54)

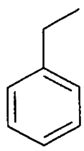

formule (55)

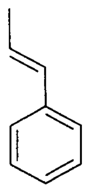

formule (56)

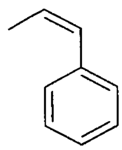

formule (57)

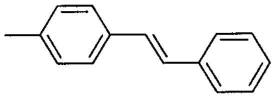

formule (58)

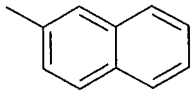

formule (59)

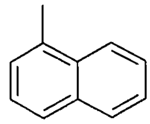

formule (60)

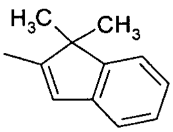
formule (61)

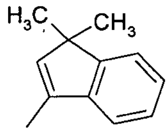
formule (62)

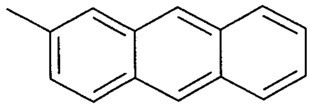
formule (63)

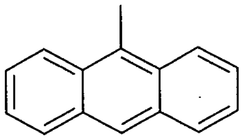
formule (64)

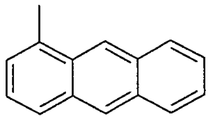
formule (65)

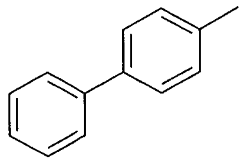
formule (66)

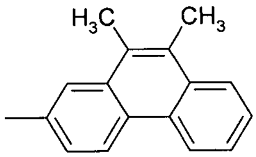
Formule (67)

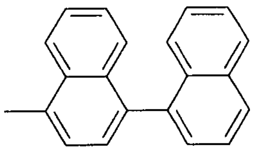
Formule (68)

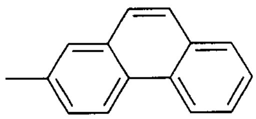
formule (69)

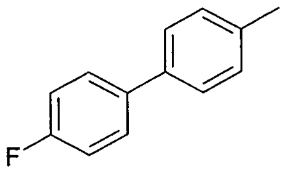
formule (70)

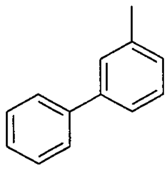
formule (71)

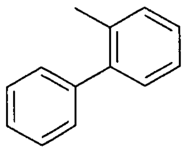
formule (72)

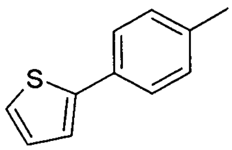
formule (73)

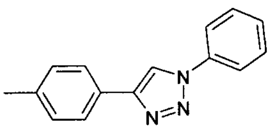
formule (74)

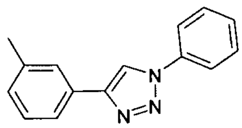
formule (75)

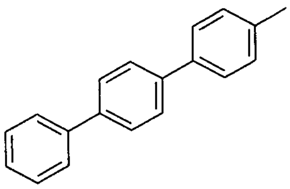
formule (76)

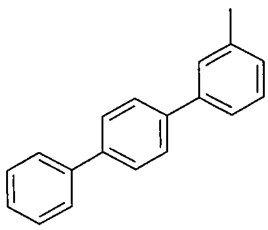
formule (77)

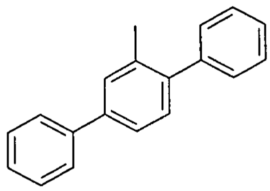
formule (78)

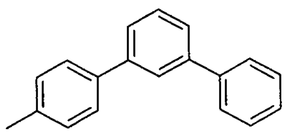

formule (79)

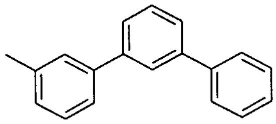

formule (80)

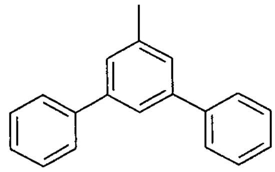

formule (81)

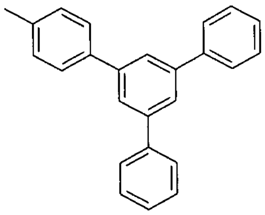

formule (82)

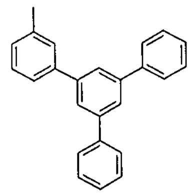

formule (83)

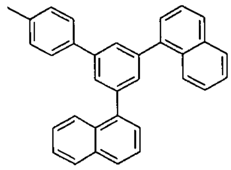

formule (84)

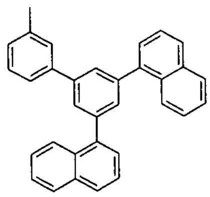

formule (85)

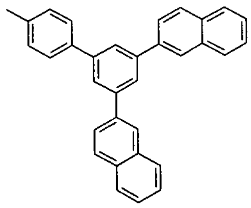

formule (86)

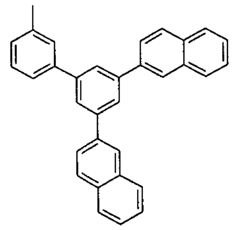

formule (87)

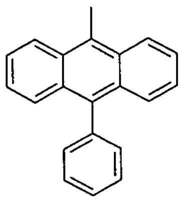

formule (88)

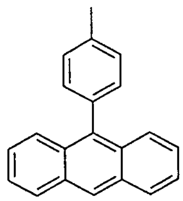

formule (89)

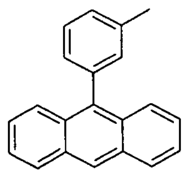

formule (90)

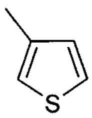

formule (91)

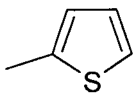

formule (92)

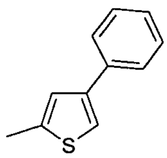

formule (93)

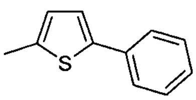

formule (94)

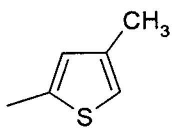

formule (95)

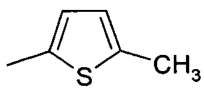

formule (96)

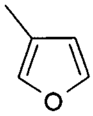
formule (97)

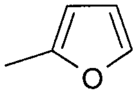
formule (98)

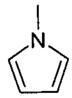
formule (99)

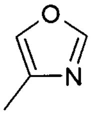
formule (100)

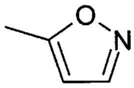
formule (101)

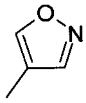
formule (102)

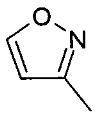
formule (103)

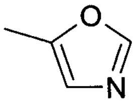
formule (104)

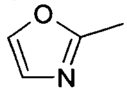
formule (105)

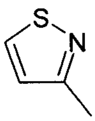
formule (106)

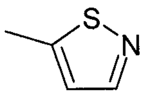
formule (107)

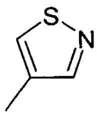
formule (108)

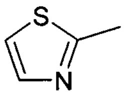
formule (109)

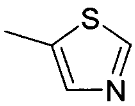
formule (110)

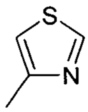
formule (111)

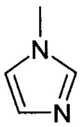
formule (112)

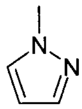
formule (113)

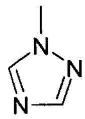
formule (114)

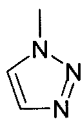
formule (115)

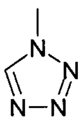
formule (116)

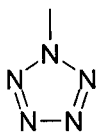
formule (117)

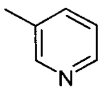
formule (118)

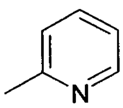
Formule (119)

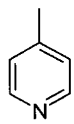
formule (120)

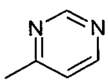
formule (121)

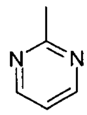
Formule (122)

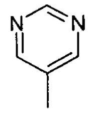
formule (123)

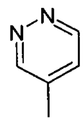
formule (124)

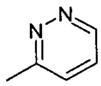
formule (125)

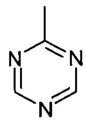
formule (126)

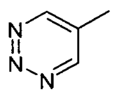
formule (127)

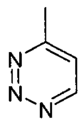
formule (128)

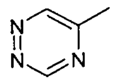
formule (129)

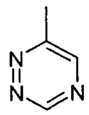
formule (130)

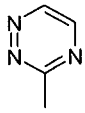
formule (131)

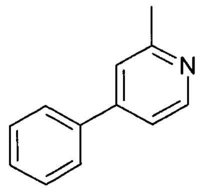
formule (132)

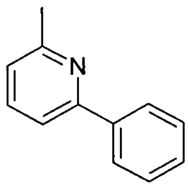
formule (133)

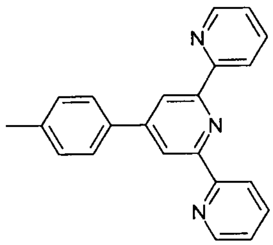
formule (134)

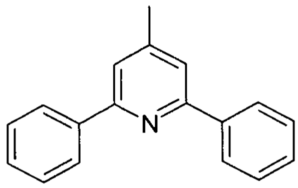
formule (135)

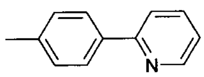
formule (136)

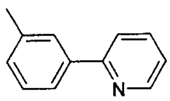
formule (137)

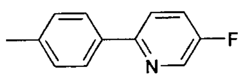
formule (138)

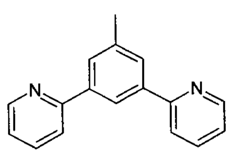
formule (139)

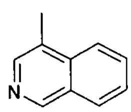
formule (140)

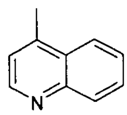
formule (141)

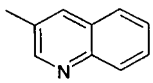

formule (142)

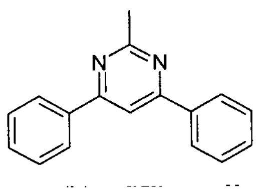

formule (143)

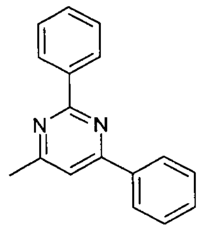

formule (144)

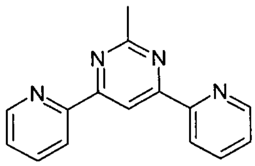

formule (145)

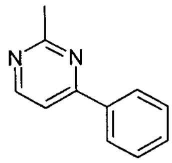

formule (146)

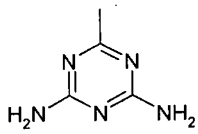

formule (147)

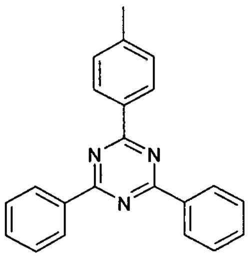

formule (148)

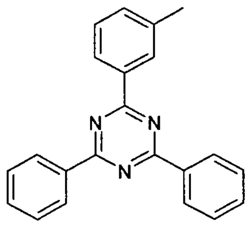

formule (149)

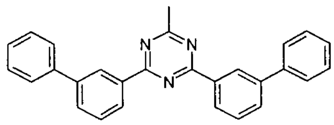

formule (150)

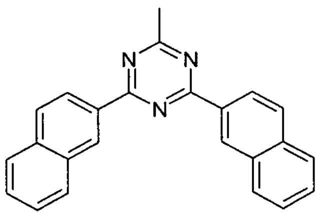

formule (151)

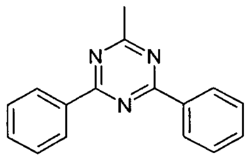

formule (152)

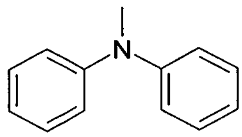

formule (153)

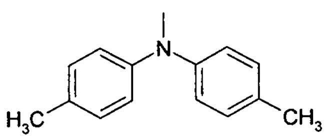

formule (154)

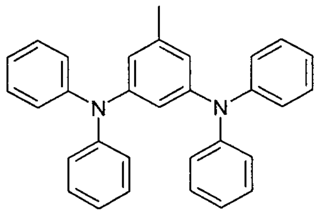

formule (155)

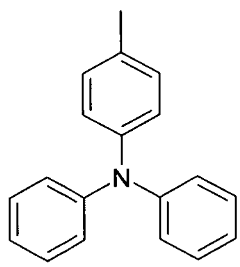

formule (156)

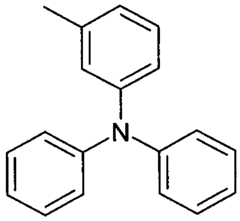

formule (157)

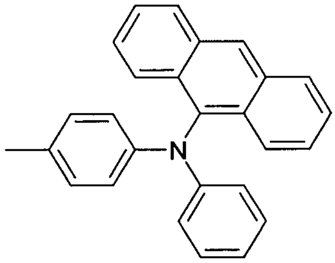

formule (158)

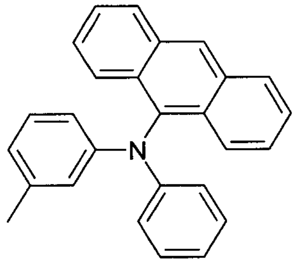

formule (159)

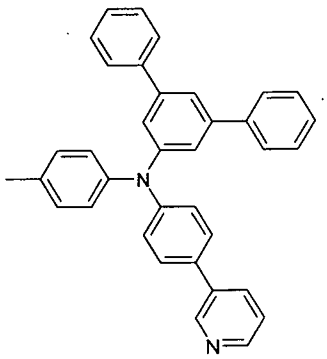

formule (160)

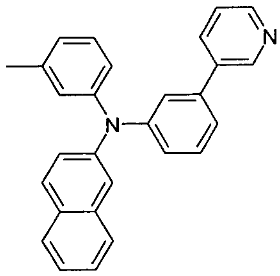

formule (161)

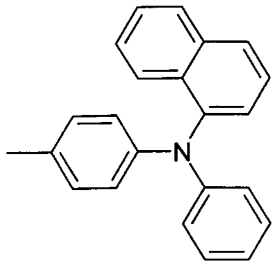

formule (162)

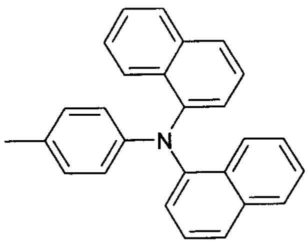

formule (163)

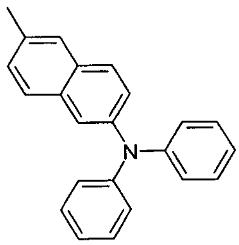

formule (164)

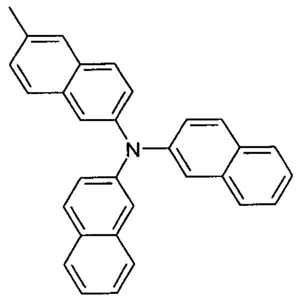

formule (165)

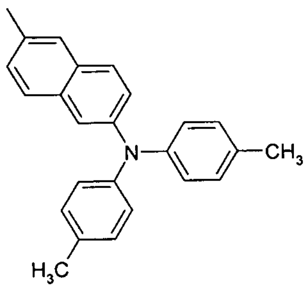

formule (166)

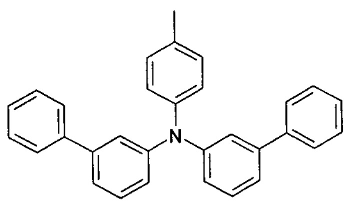

formule (167)

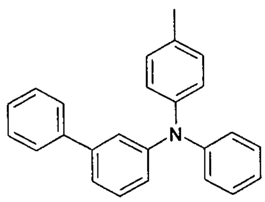

formule (168)

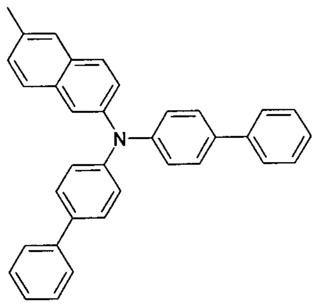

formule (169)

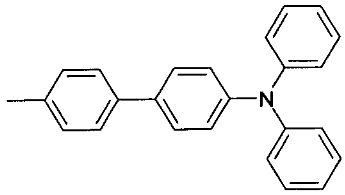

formule (170)

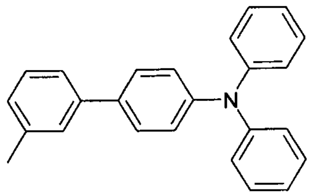

formule (171)

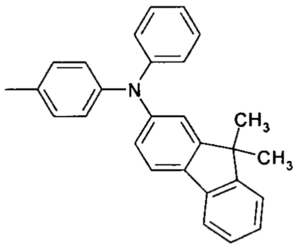

formule (172)

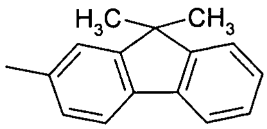

formule (173)

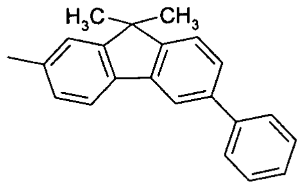

formule (174)

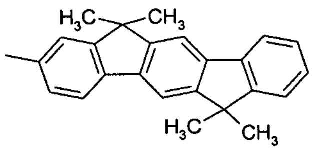

formule (175)

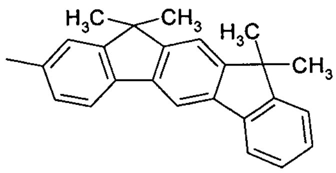

formule (176)

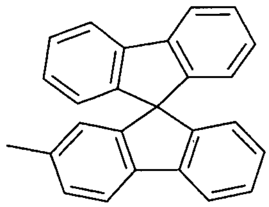

formule (177)

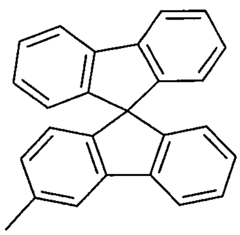

formule (178)

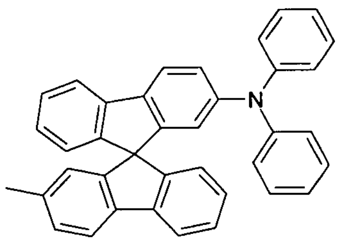

formule (179)

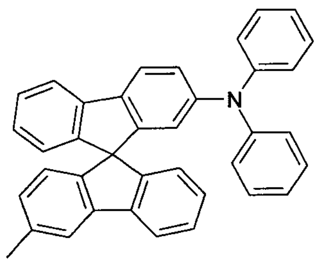

formule (180)

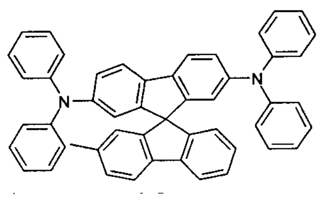

formule (181)

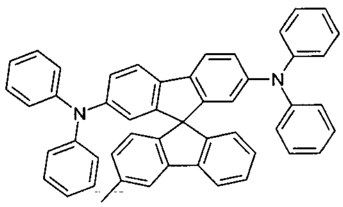

formule (182)

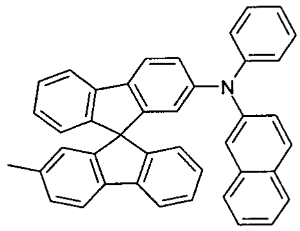

formule (183)

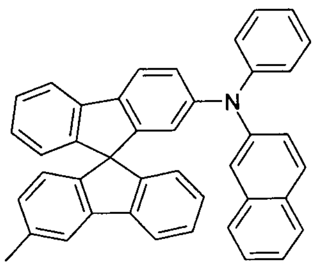

formule (184)

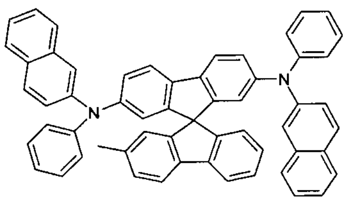

formule (185)

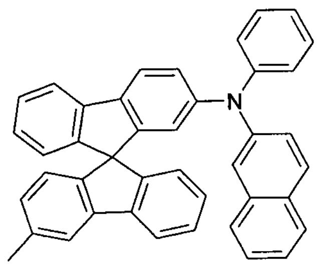

formule (186)

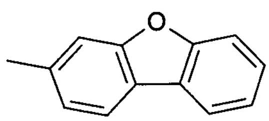

formule (187)

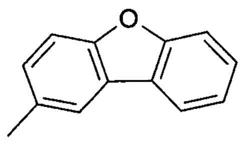

formule (188)

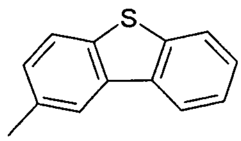

formule (189)

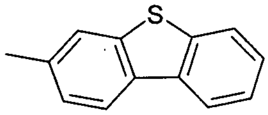

formule (190)

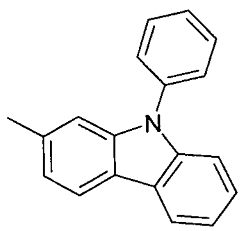

formule (191)

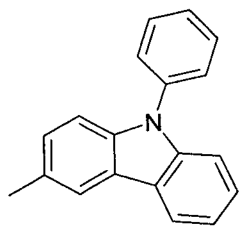

formule (192)

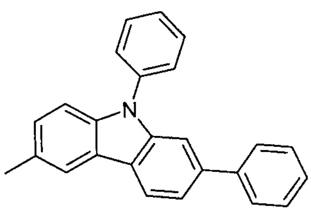

formule (193)

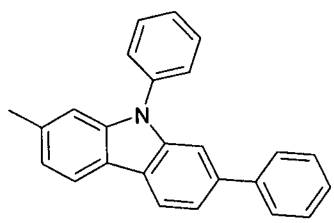

formule (194)

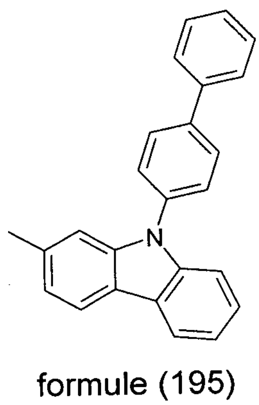

formule (195)

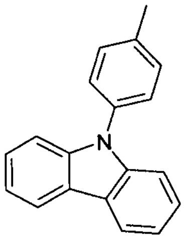

formule (196)

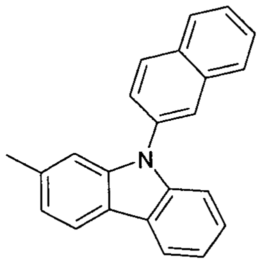

formule (197)

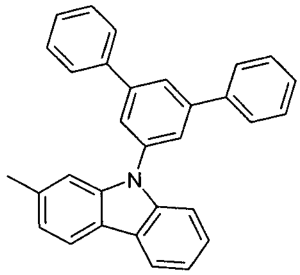

formule (198)

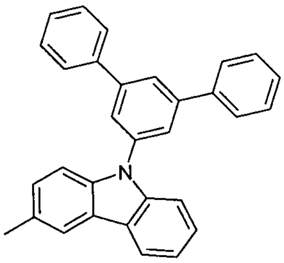

formule (199)

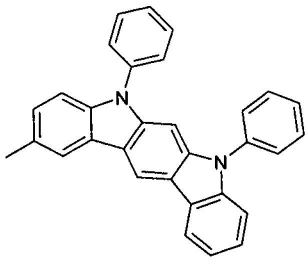

formule (200)

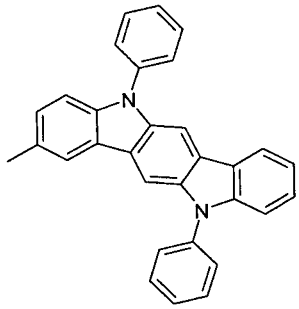

formule (201)

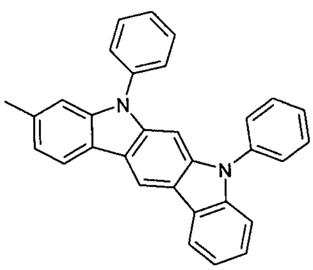

formule (202)

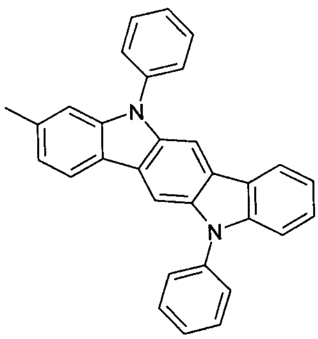

formule (203)

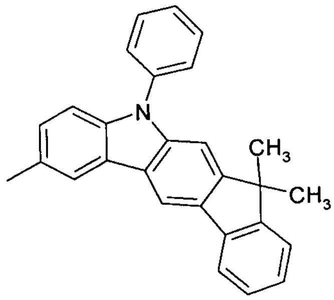

formule (204)

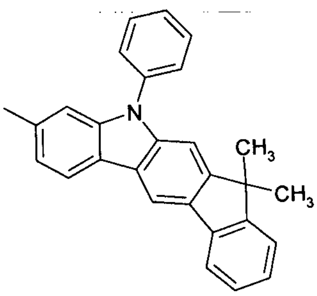

formule (205)

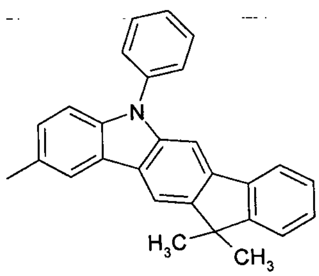

formule (206)

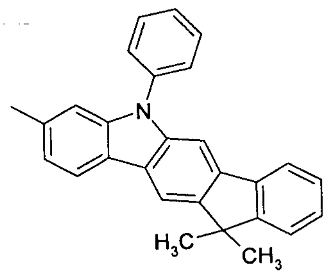

formule (207)

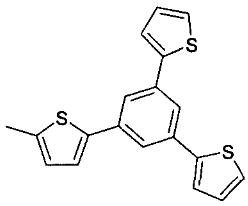

formule (208)

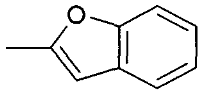

formule (209)

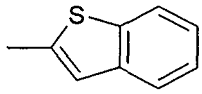

formule (210)

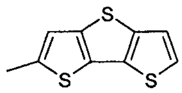

formule (211)

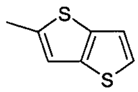

formule (212)

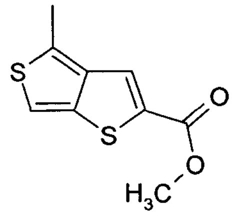

formule (213)

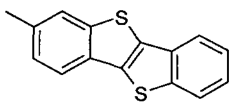

formule (214)

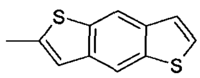

formule (215)

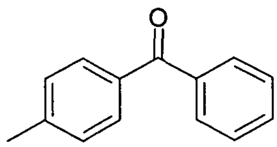

formule (216)

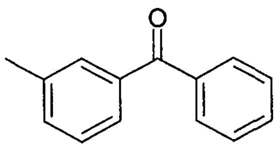

formule (217)

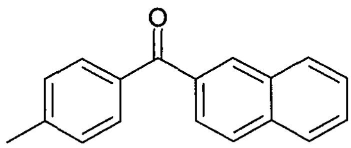

formule (218)

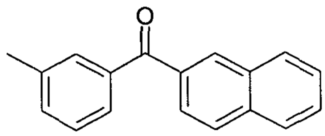

formule (219)

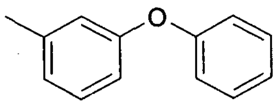

formule (220)

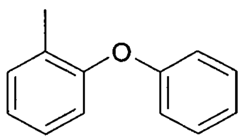

formule (221)

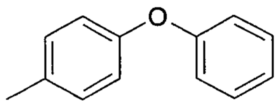

formule (222).

5. Composition selon une ou plusieurs des revendications 1 à 4, **caractérisée en ce que** le premier niveau de singlet excité de l'émetteur $S_1(E)$ est inférieur à celui du sensibilisateur $S_1(S)$.

6. Utilisation de la composition selon une ou plusieurs des revendications 1 à 5 pour une up-conversion ou conversion-élévation.

7. Utilisation selon la revendication 6, **caractérisée en ce qu'**une lumière bleue ou un rayonnement dans la région des UV est généré(e).

8. Utilisation selon la revendication 6 ou 7 pour une up-conversion ou conversion-élévation dans des dispositifs électroluminescents.

**9.** Dispositif optique et/ou électronique contenant au moins une composition selon une ou plusieurs des revendications 1 à 5.

**10.** Dispositif selon la revendication 9, **caractérisé en ce qu'**il s'agit de dispositifs électroluminescents organiques, de façon préférable des diodes émettrices de lumière organiques (OLED), des transistors à émission de lumière organiques, des cellules électrochimiques à émission de lumière organiques (OLEC, LEC ou LEEC), des transistors électrochimiques à émission de lumière organiques ou un laser organique.

**11.** Dispositif selon la revendication 9 ou 10 pour une utilisation en médecine pour la photothérapie.

**12.** Dispositif selon la revendication 11 pour une utilisation pour le traitement de la peau au moyen de la photothérapie.

**13.** Dispositif selon la revendication 11 ou 12, **caractérisé en ce que** le psoriasis, le vitiligo, la jaunisse du nouveau-né, la dermatite et la dermatite atopique, le cancer de la peau, les modifications du tissu conjonctif, de façon préférable le psoriasis, sont/est traité(s).

**14.** Dispositif selon une ou plusieurs des revendications 11 à 13, **caractérisé en ce que** le traitement est mis en oeuvre à l'aide d'une longueur d'onde inférieure à 400 nm.

**15.** Utilisation du dispositif selon la revendication 9 ou 10 dans le domaine des cosmétiques pour l'irradiation de la peau.

**16.** Utilisation selon la revendication 15, **caractérisée en ce que** l'application cosmétique est une application qui concerne l'acné, la cellulite, les rougissements de la peau, le plissement/les rides de la peau et le rajeunissement de la peau/la régénération cutanée.

Abbildung 1

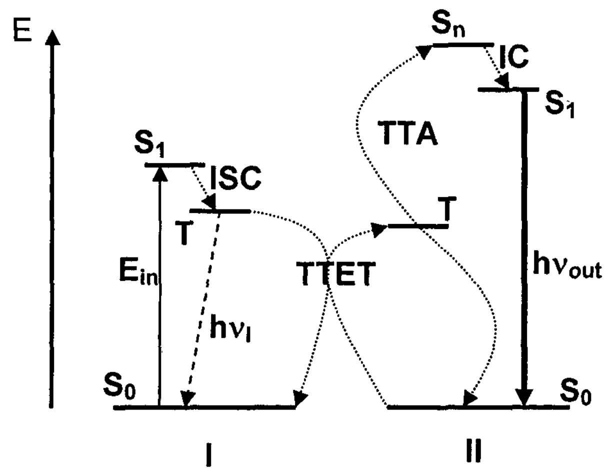


Abbildung 2

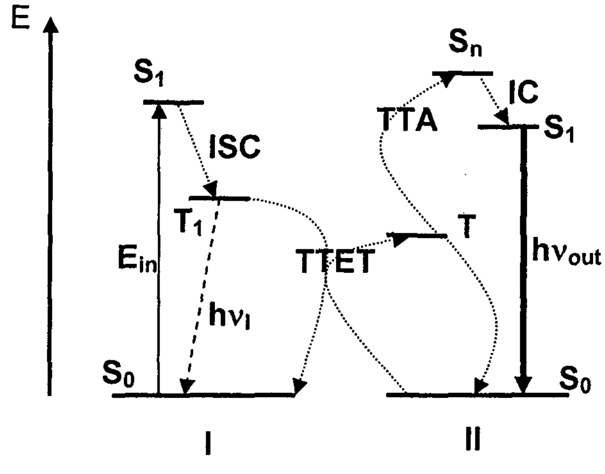

Abbildung 3

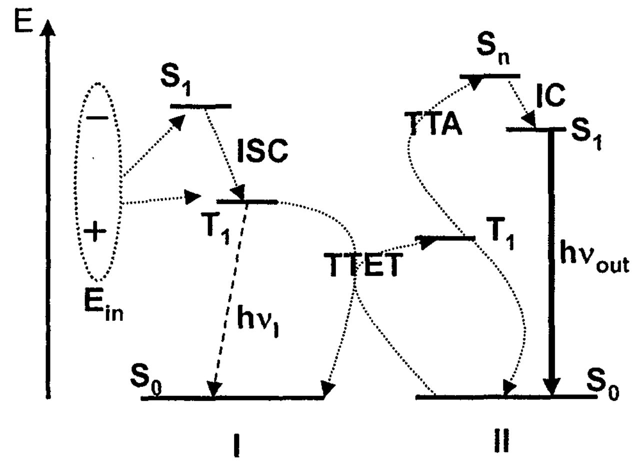


Abbildung 4

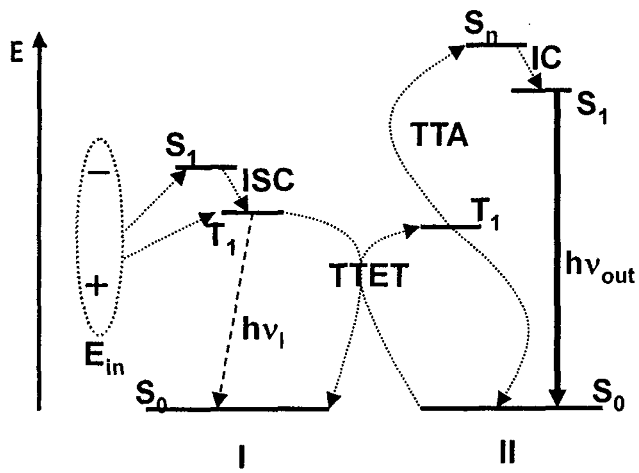

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2006008068 A1
- EP 2067839 A1
- US 5962631 A
- WO 2006052457 A2
- WO 2006118345 A1
- WO 2003020790 A1
- WO 2005104264 A1
- WO 2005014689 A2
- WO 2004041901 A1
- WO 2004113412 A2
- WO 2006063852 A1
- WO 2005056633 A1
- EP 1344788 A1
- WO 2007043495 A1
- WO 2005033174 A1
- WO 2003099901 A1
- DE 102006003710
- WO 2006122630 A
- WO 2008006449 A
- WO 2007140847 A
- WO 2006000388 A
- WO 2006058737 A
- WO 2006000389 A
- WO 2007065549 A
- WO 2007115610 A
- WO 2010012328 A
- JP 6001973 A
- WO 2004047499 A
- WO 2006098080 A
- WO 2007065678 A
- US 20050260442 A
- WO 2004092111 A
- WO 2005053051 A
- WO 2003048225 A
- WO 2004093207 A
- DE 102008035413
- WO 2008011953 A1


### In der Beschreibung aufgeführte Nicht-Patentliteratur


- **T. KOJEI et al.** *Chem. Phys. Lett.,* 1998, vol. 298, 1
- **G. S. HE et al.** *Appl. Phys. Lett.,* 1996, vol. 68, 3549
- **R. SCHROEDER et al.** *J. Chem. Phys.,* 2002, vol. 116, 3449
- *J. M. Lupton, Appl. Phys. Lett.,* 2002, vol. 80, 186
- **C. BAUER et al.** *Adv. Mater.,* 2002, vol. 14, 673
- **S. A. BAGNICH ; H. BÄSSLER.** *Chem. Phys. Lett.,* 2003, vol. 381, 464
- **P. E. KEIVANIDIS et al.** *Adv. Mater.,* 2003, vol. 15, 2095
- **CHENG et al.** *Phys. Chem. Chem. Phys.,* 2010, vol. 12, 66
- **BALUSCHEV et al.** *Appl. Phys. Lett.,* 2007, vol. 90, 181103
- **J. E. AUCKETT et al.** *J. Phys: Conference Series,* 2009, vol. 185, 012002
- **J. ZHAO et al.** *RSC Advances,* 2011, vol. 1, 937
- **M. FISCHER et al.** *Angew. Chem., Int. Ed.,* 1999, vol. 38, 885
- **ZINSLI.** *Chem. Phys. Lett.,* 1975, vol. 34, 403
- **BOLDRIDGE et al.** *J. Phys. Chem.,* 1982, vol. 86, 1976
- **KOMOROWSKI et al.** *J. Photochem.,* 1985, vol. 30, 141
- **B. M. S. ARNOLD et al.** *Appl. Phys. Lett.,* 2008, vol. 92, 053301
- *Nach J. Phys. Chem. A,* 2009, vol. 113, 5913
- *RSC Advances,* 2011, vol. 1, 937